# EUROPEAN PATENT SPECIFICATION

(11) **EP 0 274 873 B1**
(45) Date of publication and mention of the grant of the patent: **23.09.1992**
(21) Application number: 87310953.2
(22) Date of filing: 14.12.1987
(51) Int. Cl.: C07K 11/00, C12P 1/04, A61K 35/74, A23K 1/17

(54) **Antibiotic A10255 complex and factors, process, microorganisms for its production**
Antibiotikum-A10255-Komplex und Faktoren, Verfahren, Mikroorganismen für seine Herstellung
Antibiotique A10255 complexe et facteurs, procédé, micro-organismes pour sa fabrication

(30) Priority: 15.12.1986 US 941894; 15.12.1986 US 941473
(43) Date of publication of application: 20.07.1988
(73) Proprietor: ELI LILLY AND COMPANY, Indianapolis, Indiana 46285 (US)
(72) Inventor: Boeck, LaVerne Dwaine, Indianapolis Indiana 46224 (US); Hoehn, Marvin Martin, Indianapolis Indiana 46250 (US); Kirst, Herbert Andrew, Indianapolis Indiana 46227 (US); Michel, Karl Heinz, Indianapolis Indiana 46204 (US); Seno, Eugene Thomas, Indianapolis Indiana 46250 (US); Godfrey, Otis Webster, Jr., Greenwood Indiana 46142 (US)
(74) Representative: Hudson, Christopher Mark

(56) References cited:
- CHEMICAL ABSTRACTS, vol. 79, no. 25, 24th December 1973, Columbus, Ohio, USA, page 204, column 2, abstract no. 144 870n; & JP - B - 7300075 (KYOWA FERMENTATION INDUSTRY), 05-01-1973
- CHEMICAL ABSTRACTS, vol. 68, no. 25, 17th June 1968, Columbus, Ohio, USA; M.A. LANEELLE et al.: "Chemical and immunological relations in the Actinomyeetales. IV. Chemical composition of the liquids of four Streptomyces strains and a strain of Nocardia gardneri", page 10821, column 2, abstract no. 112 328y; & Ann. Inst. Pasteur vol. 114, no. 3, 1968, pages 305-312

## Description

This invention relates to newly-discovered antibiotic substances which are arbitrarily generically designated here as antibiotic A10255. Antibiotic A10255 is composed of a mixture of 6 separate compounds or factors (factor B, C, E, F, G, and H). The mixture of factors is co-produced by culturing the novel strains Streptomyces gardneri, NRRL 15537, NRRL 19260, NRRL 15922, or an A10255-producing mutant thereof, under submerged aerobic fermentation conditions until a substantial level of antibiotic A10255 is produced. The coproduced factors B, C, E, F, G and H are extracted from the fermentation broth (in minor amounts) and from the mycelia (in major amounts) with solvents. The coproduced factors are separated as a mixture by concentrating the extracts and extracting the concentrate with a water immiscible solvent, concentrating the extract, and collecting and drying the resulting complex-containing precipitate. It should be noted that the term "antibiotic complex", and "A10255 complex" as used in the fermentation art and in this specification does not refer to a covalently-bonded chemical complex, but to a mixture of co-produced individual antibiotic factors. As will be recognized by those familiar with antibiotic fermentation, the ratio of individual factors produced in an antibiotic complex may vary, depending on the fermentation conditions used. The A10255 complex is further purified and is separated into individual factors B, C, E, F, G, and H by a series of chromatographic procedures.

Antibiotic A10255 complex and the individual factors B, C, E, F, G and H inhibit the growth of microorganisms pathogenic to man and animals. The A10255 complex and the individual factors also increase feed utilization efficiency in chickens and cattle and promote the growth and increase the feed utilization efficiency of weanling pigs.

Further aspects of the instant invention include the biologically pure cultures of the microorganisms Streptomyces gardneri NRRL 15537, Streptomyces gardneri NRRL 18260, and Streptomyces gardneri NRRL 15922, or an A10255-producing mutant thereof, and the methods for producing the A10255 complex. The invention also provides feed compositions comprising the A10255 complex or the individual A10255 factors combined with the appropriate feed for chickens, weanling pigs, and cattle. Also, the invention encompasses varous pharmaceutical compositions comprising a suitable vehicle and a therapeutically effective amount of the A10255 complex, A10255 factor B, A10255 factor C, A10255 factor E, A10255 factor F, A10255 factor G, or A10255 factor H. The A10255 complex of this invention can be used to select Streptomyces cells which have the thiostrepton-resistance gene as yet a further aspect of the invention.

### Description of the Drawings

The infrared absorption spectra of individual A-10255 factors B, C, E, F, G and H run in KBr discs are persented in the drawings as follows:
FIG. 1 -- A10255 Factor B
FIG. 2 -- A10255 Factor C
FIG. 3 -- A10255 Factor E
FIG. 4 -- A10255 Factor F
FIG. 5 -- A10255 Factor G
FIG. 6 -- A10255 Factor H

### Detailed Description of the Invention

The A10255 complex, comprised mainly of factors B, C, E, F, G, and H is produced by culturing in an aqueous culture medium containing assimilable sources of carbohydrate, nitrogen and inorganic salts the heretofore undescribed strains of Streptomyces gardneri, strain NRRL 15537 or NRRL 18260, or an A10255-producing mutant thereof, until the A10255 complex is produced.

As is the case with many antibiotic-producing cultures, fermentation of an A10255-producing strain of S. gardneri NRRL 15537, NRRL 18260, or NRRL 15922 results in the coproduction of a number of antibiotic substances. Antibiotic A10255 factor B is the major factor produced by the NRRL 15537 culture, and factors C, E, F, G and H are produced in minor yet isolable amounts. Other factors either are present in such minor quantities as to render their isolation unrewarding or are relatively unstable. Factors G and H have been isolated from fermentation of the NRRL 18260 culture, with factor G being the major factor. The amounts of the individual factors coproduced may vary from fermentation to fermentation of any of the above-described microorganisms.

The antibiotic factors B, C, E, F, G, and H coporduced during the fermentation and obtained as a mixture are termed the A10255 complex. The individual factors are separated from each other and isolated as distinct entities with the following physical and biological properties.

The term "pharmaceutically-acceptable salts" includes alkali and alkaline earth metal salts of the compounds of the present invention, for example, lithium, sodium, potassium and calcium, as well as acid addition salts such as those which are formed between the compounds of the present invention and acids such as hydrochloric, hydrobromic, sulfuric and phosphoric acids.

### Physical Characteristics of the Antibiotic Factors

### A10255 Factor B

A10255 Factor B is a non-crystalline white to light-yellow powder which is soluble in dimethylsulfoxide, dimethylformamide, pyridine, chloroform/methanol mixtures, and 4:1 (v:v) tetrahydrofuran:water.

Elemental analysis of factor B indicates the following approximate percentage composition (average): carbon, 49.25%; hydrogen, 3.94%; nitrogen, 15.65%; oxygen, 21.36%; and sulfur, 6.73%.

The apparent molecular weight of A10255 factor B was determined by fast atom bombardment mass spectrometry to be approximately 1244 daltons.

Electrometric titration of A10255 factor B in 66% aqueous dimethylformamide indicates the presence of three titratable groups with pKa values of 4.9, 11.2, and 12.8.

Amino acid analysis of factor B (after hydrolysis with 6N hydrochloric acid) indicates the presence of ammonia (5,268 mmoles/mg) and threonine (629 mmoles/mg). The analysis also evinced a large, unidentified peak coming before the position for the histidine peak.

The ultraviolet absorption spectrum for factor B obtained in neutral, acidic, and basic methanol demonstrated λₘₐₓ of 245 nm (ε=66,000).

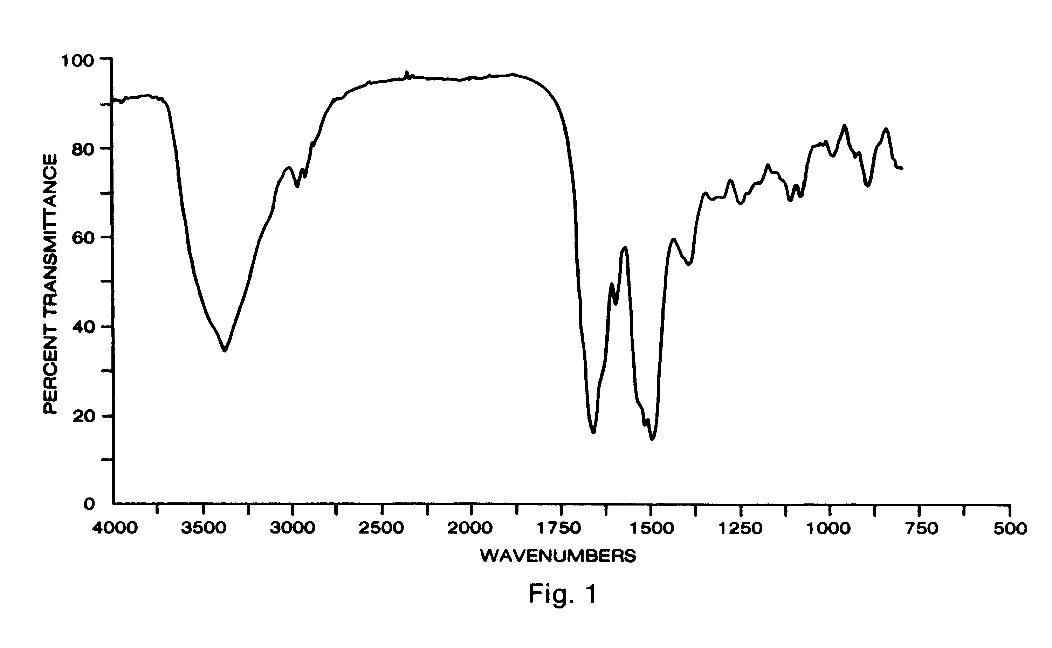

The infrared absorption spectrum (KBr disc) is set forth in FIG. 1 of the accompanying drawings. The more significant absorption maxima observed in the spectrum are those at 3373, 2969, 2932, 2875, 1661, 1598, 1520, 1494, 1395, 1250, 1114, 1084, 996, 932, and 900 cm⁻¹.

The proton nuclear magnetic resonance spectrum of factor B was obtained in perdeuterated dimethylsulfoxide at 500 MHz and had the following signals:

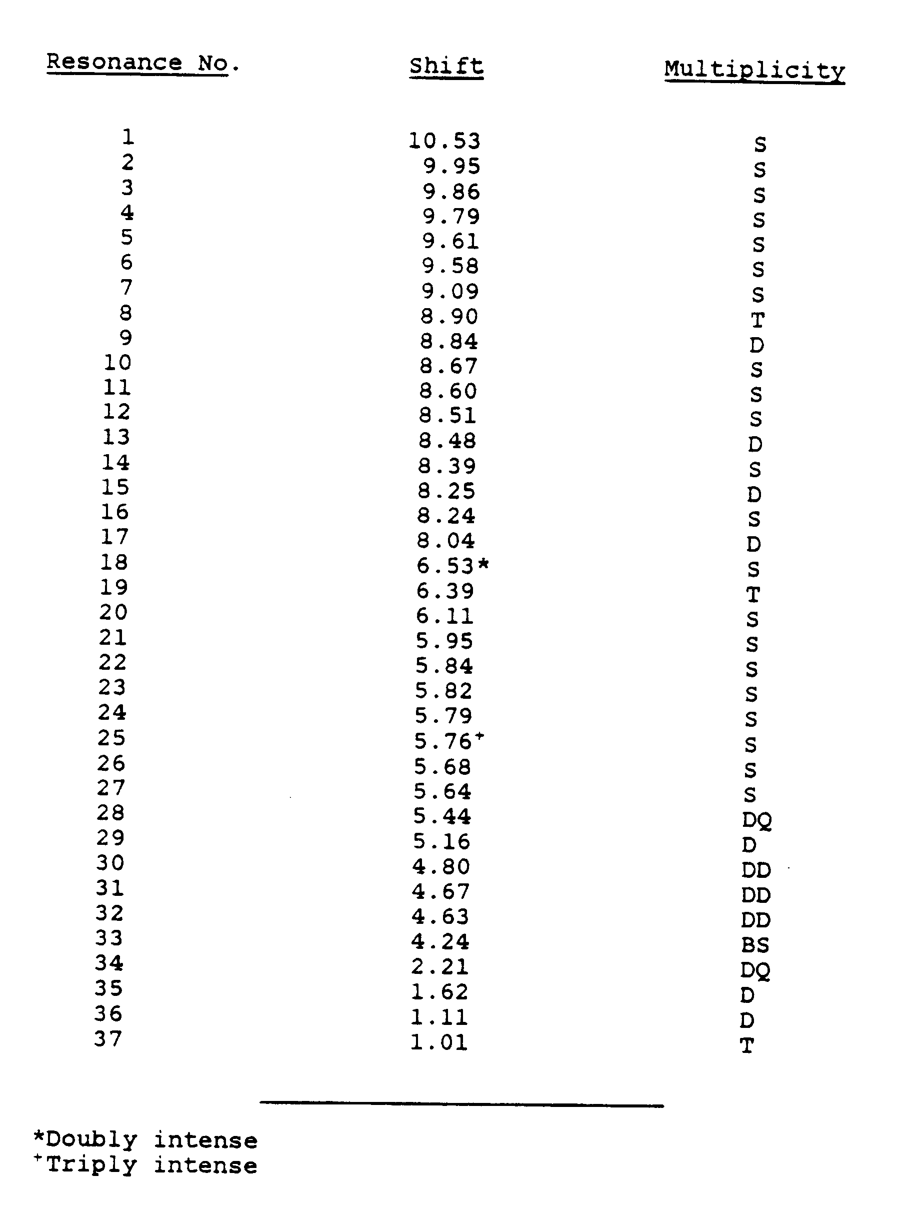

The ¹³C nuclear magnetic resonance spectrum of factor B was obtained in perdeuterated dimethylsulfoxide at 125 MHz and had the following signals:

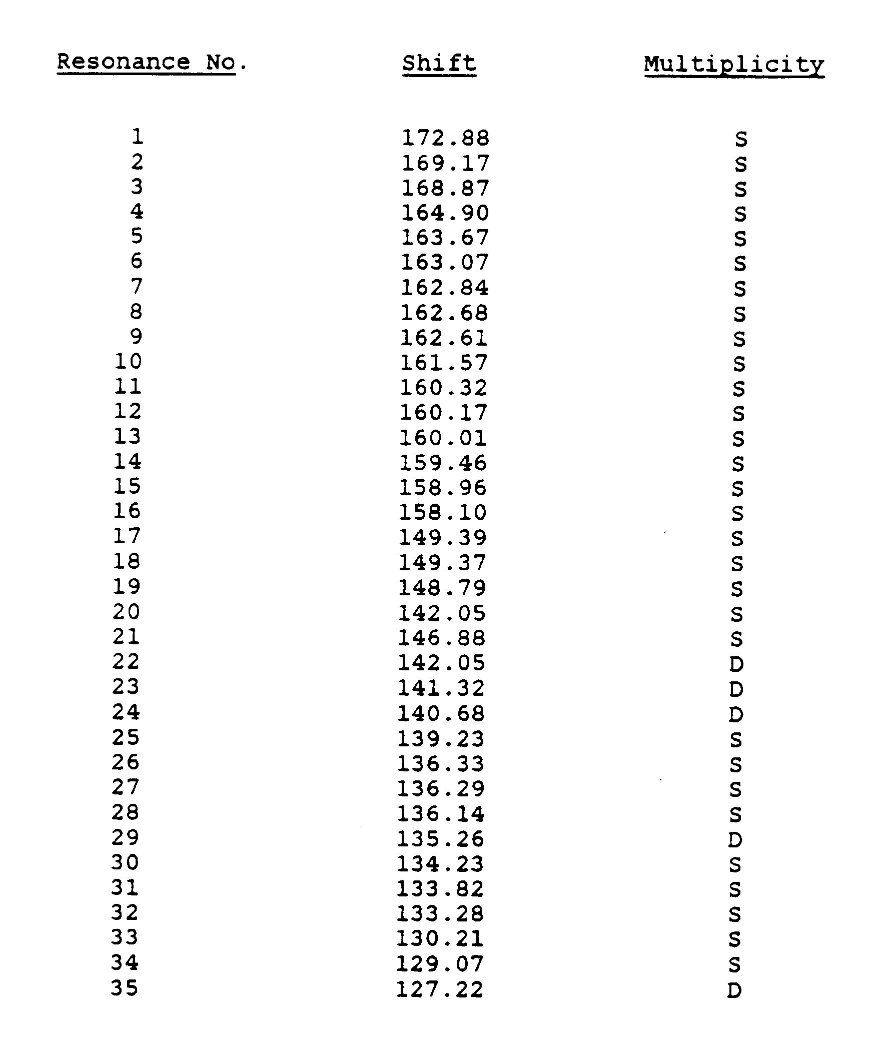

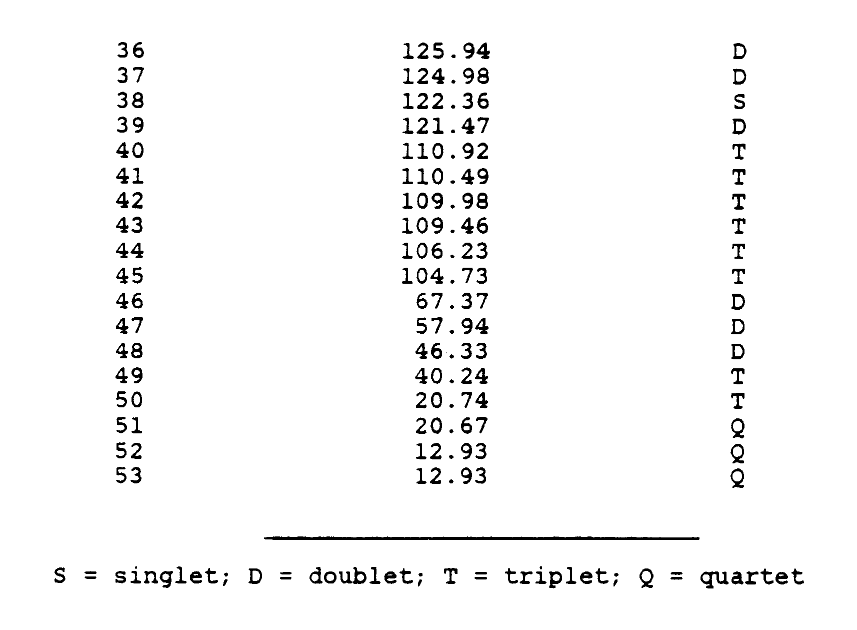

### A10255 Factor C

A10255 factor C is a non-crystalline white to light-yellow powder which is soluble in dimethylsulfoxide, dimethylformamide, pyridine, 1:1 (v:v) methylene chloride/methanol, and 4:1 (v:v) tetrahydrofuran:water.

Elemental analysis of factor C indicates the following approximate percentage composition (average): carbon, 49.18%; hydrogen, 3.86%; nitrogen 17.89%; oxygen, 18.28%; and sulfur, 6.46%.

The apparent molecular weight of A10255 factor C was determined by fast atom bombardment mass spectrometry to be approximately 1174 daltons. Using CsI as reference standard, exact mass was determined to be 1175.35 (M + H).

Electrometric titration of A10255 factor C in 66% aqueous dimethylformamide (initial pH, 7.29) indicated the presence of two titratable groups with pKa values of 2.9 (uncertain) and 12.0. Amino acid analysis of the factor C (after hydrolysis with 6N hydrochloric acid) indicated the presence of ammonia (7,429 mmoles/mg) and threonine (758 mmoles/mg). The analysis also evinced a large, unidentified peak coming before the position for the histidine peak.

The ultraviolet absorption spectrum for factor C obtained in neutral, acidic, and basic methanol demonstrated λₘₐₓ of 245 nm (ε=63,000).

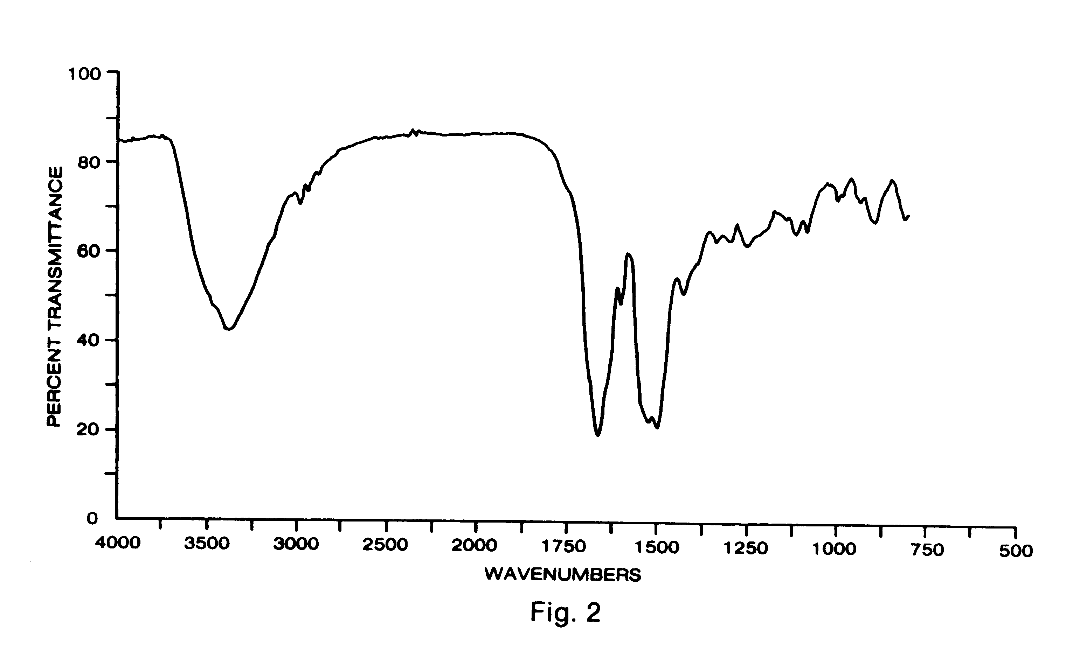

The infrared absorption spectrum (Kbr disc) is reproduced as FIG. 2 of the accompanying drawings. The more significant absorption maxima observed in the spectrum occur at 3375, 2973, 2932, 2876, 1661, 1597, 1494, 1427, 1345, 1305, 1249, 1111, 1083, 984, 933, and 894 cm⁻¹.

The proton nuclear magnetic resonance spectrum of factor C was obtained in perdeuterated dimethylsulfoxide at 360 MHz and had the following signals: δ 10,51, 10.08, 9.84, 9.57, 9.10, 8.88, 8.03, 7.94, 7.53, 5.15 (all of the foregoing are exchangeable with D₂O), 8.67, 8.59, 8.51, 8.49, 8.38, 8.25, 8.24, 6.57, 6.52, 6.38, 6.11, 5.91, 5.78, 5.74, 5.70, 5.65, 5.63, 5.44, 5.15, 4.79, 4.67, 4.63, 4.23, 2.21, 1.62, 1.11, and 1.01.

### A10255 Factor E

A10255 factor E is a non-crystalline white to light-yellow powder which is soluble in dimethylsulfoxide, dimethylformamide, pyridine, chloroform/methanol mixtures, and 4:1 (v:v) tetrahydrofuran:water.

Elemental analysis of factor E indicates the following approximate percentage composition (average): carbon, 48.03%; hydrogen, 3.91%; nitrogen, 15.76%; oxygen, 17.09%; and sulfur, 5.63%.

The apparent molecular weight of A10255 factor E was determined by fast atom bombardment mass spectrometry to be approximately 1258 daltons. The exact mass of factor E was determined to be 1259.55 (M + H) using CsI as reference standard.

Electrometric titration of A10255 factor E in 66% aqueous dimethylformamide indicates the presence of three titratable groups with pKa values of 4.85, 11.1, and 13.2. Amino acid analysis of the factor E (after hydrolysis with 6N hydrochloric acid) indicates the presence of ammonia (8,580 mmoles/mg) and threonine (716 mmoles/mg). The analysis also evinced a large, unidentified peak coming before the position of the histidine peak.

The ultraviolet absorption spectrum for factor E obtained in neutral, acidic, and basic methanol demonstrated a λₘₐₓ of 245 nm (ε=77,000).

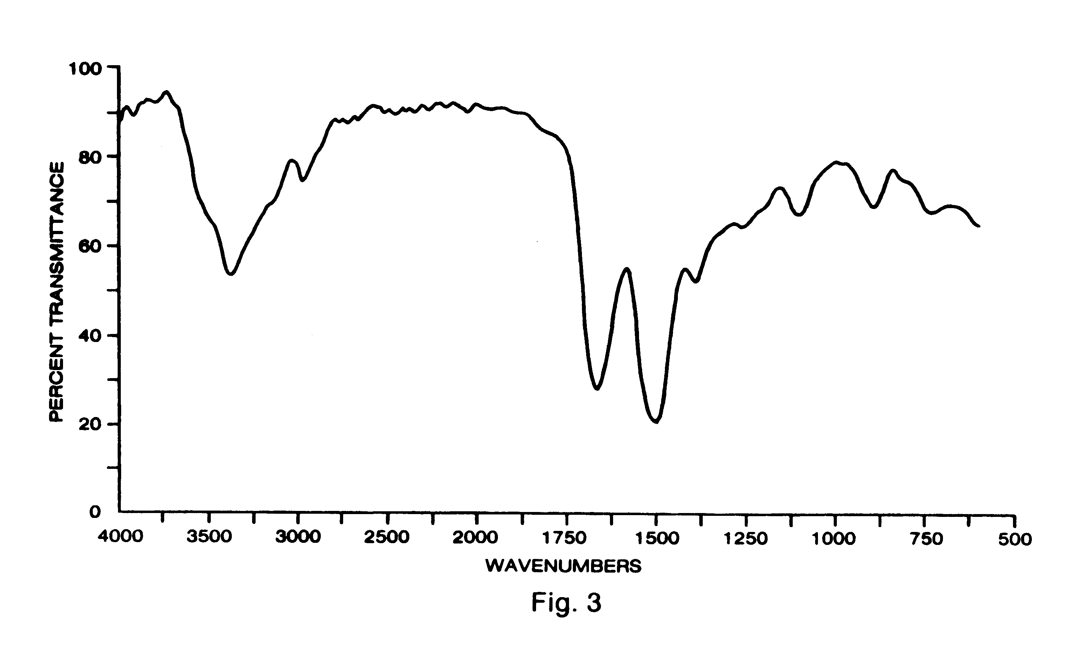

The infrared absorption spectrum (KBr disc) is reproduced in FIG. 3 of the accompanying drawings. The more significant absorption maxima observed in the spectrum occur at 3367, 3361, 2966, 1664, 1501, 1389, 1254, 1102, and 889 cm⁻¹.

The proton nuclear magnetic resonance spectrum of factor E was obtained in perdeuterated dimethylsulfoxide at 270 MHz and had the following signals : δ 10.54, 10.00, 9.94, 9.81, 9.60, 9.56, 9.45, 8.89, 8.84, 8.66, 8.59, 8.50, 8.47, 8.39, 8.25, 8.22, 8.10, 6.53, 6.50, 6.24, 5.95, 5.86, 5.84, 5.77, 5.64, 5.55, 5.52, 5.44, 5.10, 4.80, 4.66, 4.64, 4.22, 2.78, 1.60, 1.11, and 1.00.

### A10255 Factor F

A10255 factor F is a white to light-yellow non-crystalline powder which is soluble in dimethylsulfoxide, dimethylformamide, pyridine, 1:1 (v:v) methyl chloride/methanol, and 4:1 (v:v) tetrahydrofuran:water.

Elemental analysis of factor F indicates the following approximate percentage composition (average): carbon, 49.65%; hydrogen, 4.23%; nitrogen, 17.11%; oxygen, 22.08%; and sulfur, 7.78%.

The apparent molecular weight of A10255 factor F was determined by field desorption mass spectrometry to be approximately 1188 daltons.

Electrometric titration of A10255 factor F in 66% aqueous dimethylformamide (starting pH of 7.08) indicates the presence of a titratable group with a pKa value of 12.5.

Amino acid analysis of the factor F (after hydrolysis with 6N hydrochloric acid) indicates the presence of ammonia (7,226 mmoles/mg) and threonine (735 mmoles/mg). The analysis also evinced a large, unidentified peak coming before the position of the histidine peak.

The ultraviolet absorption spectrum for factor F obtained in neutral, acidic, and basic methanol demonstrated a λₘₐₓ of 245 nm (ε=71,500).

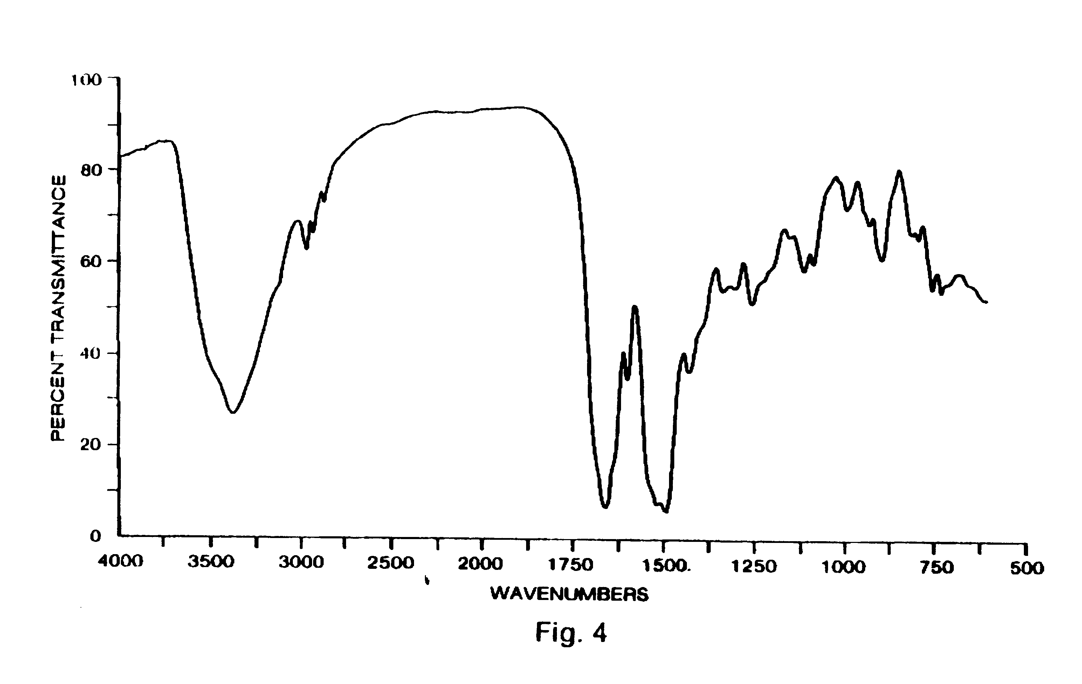

The infrared absorption spectrum (KBr disc) is reproduced as FIG. 4 of the accompanying drawings. The more significant absorption maxima observed in the spectrum occur at 3369, 2943, 2907, 2846, 1663, 1588, 1519, 1493, 1425, 1337, 1288, 1251, 1151, 1110, 1083, 995, 927, 890, 807, 776, and 751 cm⁻¹.

The proton nuclear magnetic resonance spectrum of factor F was obtained in perdeuterated dimethylsulfoxide at 360 MHz and had the following signals: δ 10.51, 10.17, 9.88, 9.77, 9.54, 9.10, 8.90, 8.88, 8.66, 8.59, 8.51, 8.49, 8.38, 8.25, 8.24, 8.06, 7.94, 7.53, 6.56, 6.51, 6.27, 6.23, 6.12, 6.12, 5.96, 5.77, 5.76, 5.71, 5.71, 5.64, 5.62, 5.47, 5.14, 4.77, 4.65, 4.62, 4.20, 2.77, 2.48, 2.48, 1.58, 1.08, 0.98, and 0.98.

### A10255 Factor G

A10255 factor G is a non-crystalline white to light-yellow powder which is soluble in dimethylsulfoxide, dimethylformamide, pyridine, chloroform/methanol mixtures and 4:1 (v:v) tetrahydrofuran:water.

Elemental analysis of factor G indicates the following approximate percentage composition:

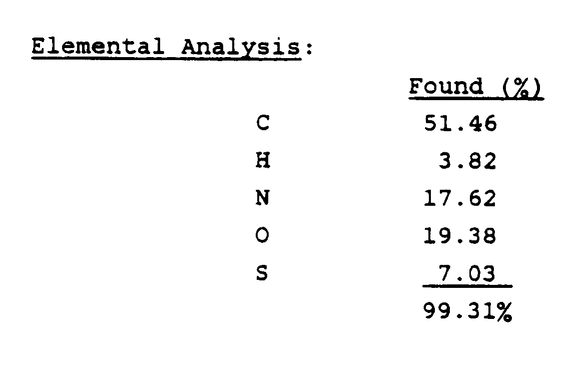

The ultraviolet absorption spectrum for factor G in neutral ethanol demonstrated a λₘₐₓ = 247 nm (ε = 72,200); acidic solution, λₘₐₓ = 247 nm (ε = 73,400); and basic solution, λₘₐₓ = 211 nm (ε = 27,200).

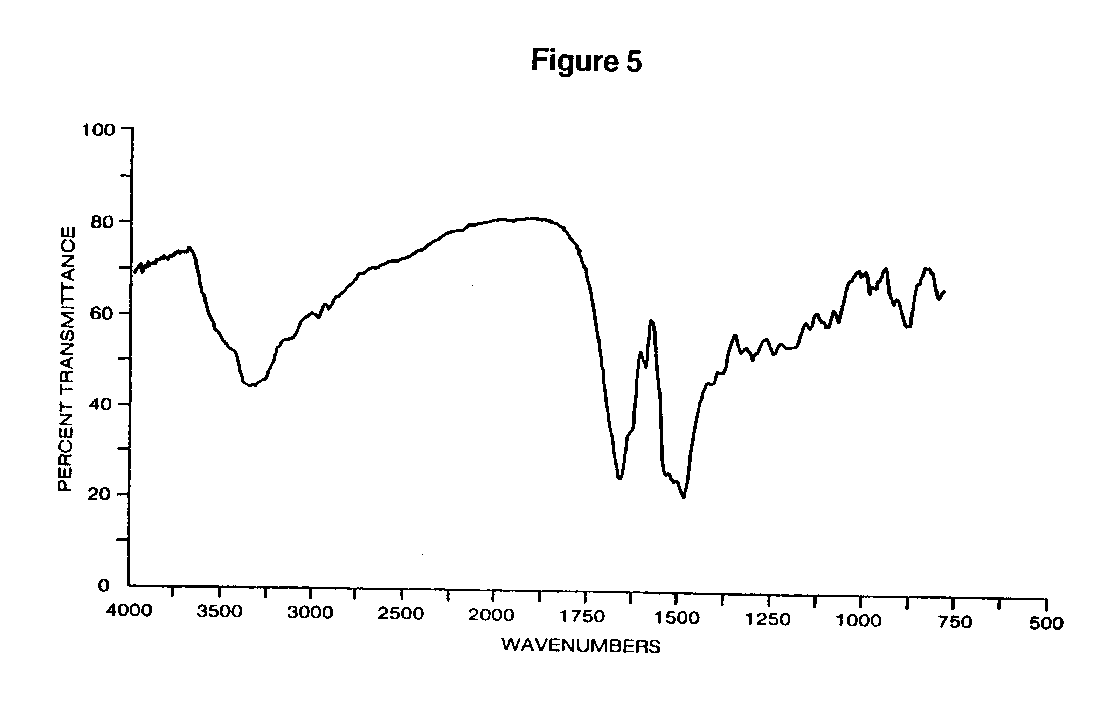

The infrared spectrum (KBr disc) for A10255 factor G is set forth in Fig. 5 of the accompanying drawings.

The ¹H nuclear magnetic resonance spectrum of factor G was obtained in perdeuterated dimethylsulfoxide at 500 MHz and had the following signals:

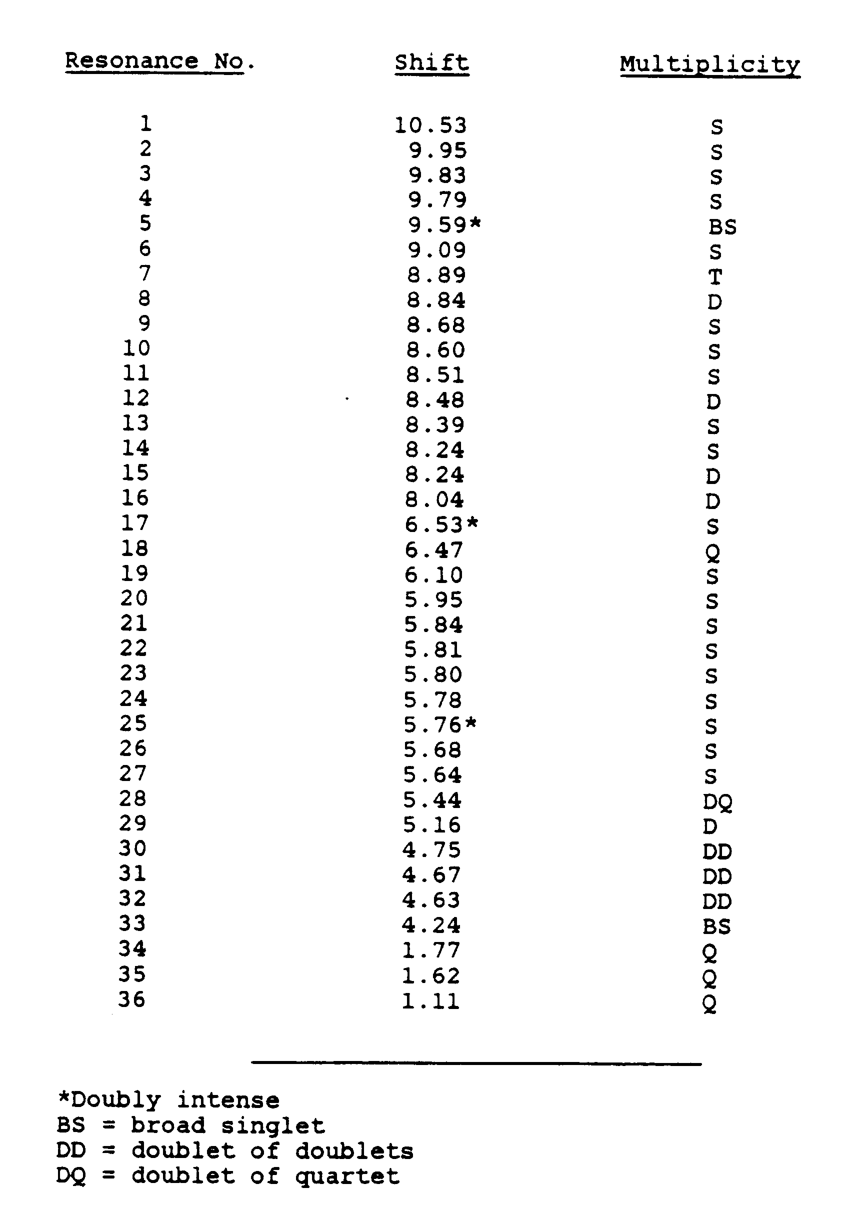

The ¹³C Nuclear Magnetic Resonance Spectrum of factor G was obtained in perdeuterated dimethylsulfoxide at 125 MHz:

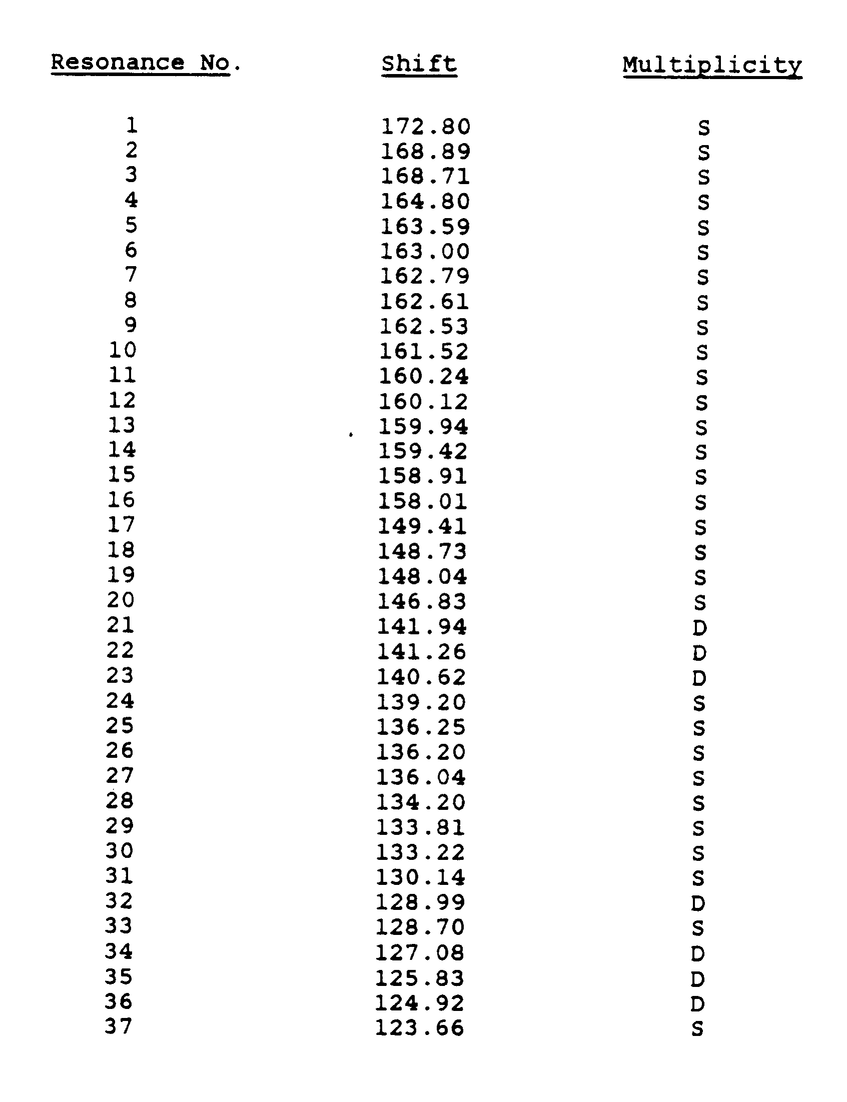

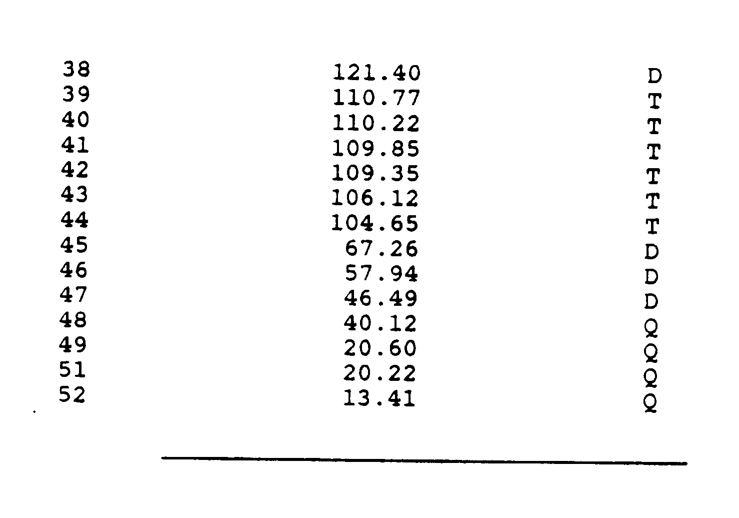

### A10255 Factor H

A10255 factor H is a non-crystalline white to light-yellow powder which is soluble in dimethylsulfoxide, dimethylformamide, pyridine, 1:1 (v:v) methylene chloride/methanol, and 4:1 (v:v) tetrahydrofuran:water.

Elemental analysis of factor H indicates the following approximate percentage composition:

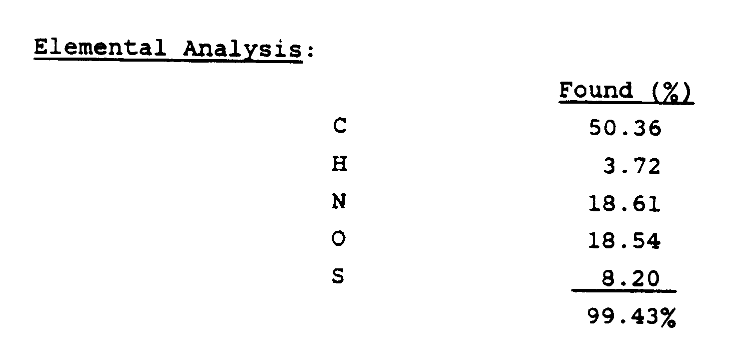

The ultraviolet absorption spectrum for factor H in neutral ethanol demonstrated a λₘₐₓ of 244 nm (ε = 74,000); acidic solution, λₘₐₓ = 245 nm (ε = 74,500); and basic solution, λₘₐₓ = 211 nm (ε = 23,800).

The ¹³C nuclear magnetic resonance spectrum of A10255 factor H at 125 MHz in perdeuterated dimethylsulfoxide had the following signals:

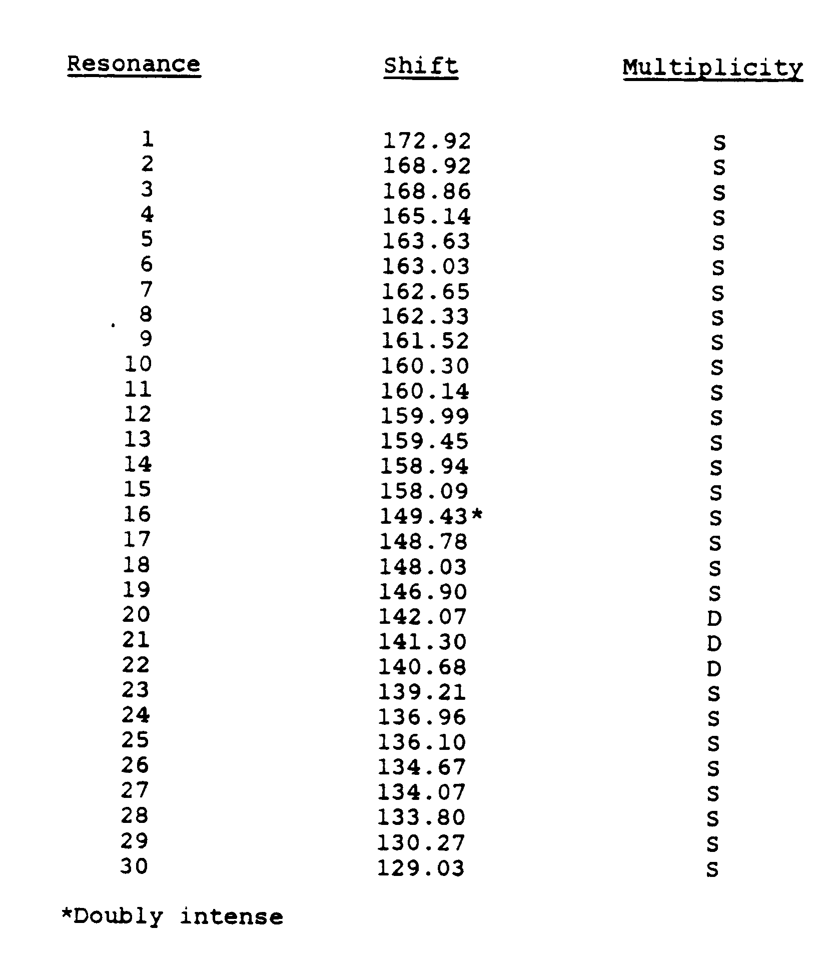

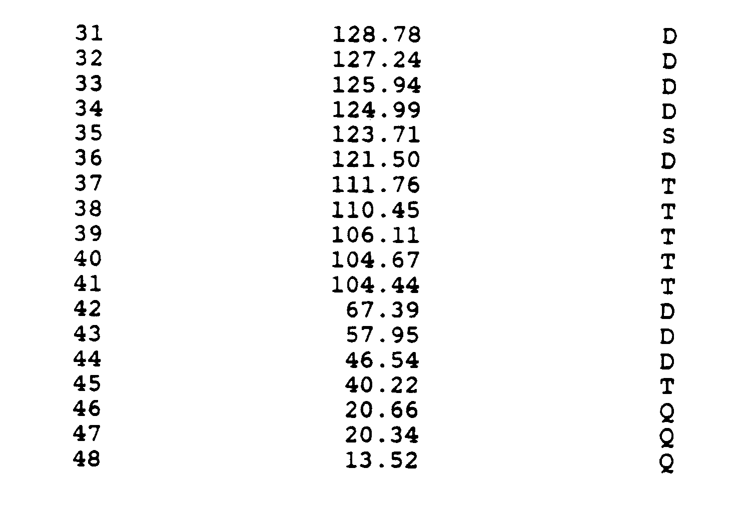

The ¹H nuclear magnetic resonance spectrum at 500 Mhz in perdeuterated dimethylsulfoxide had the following signals:

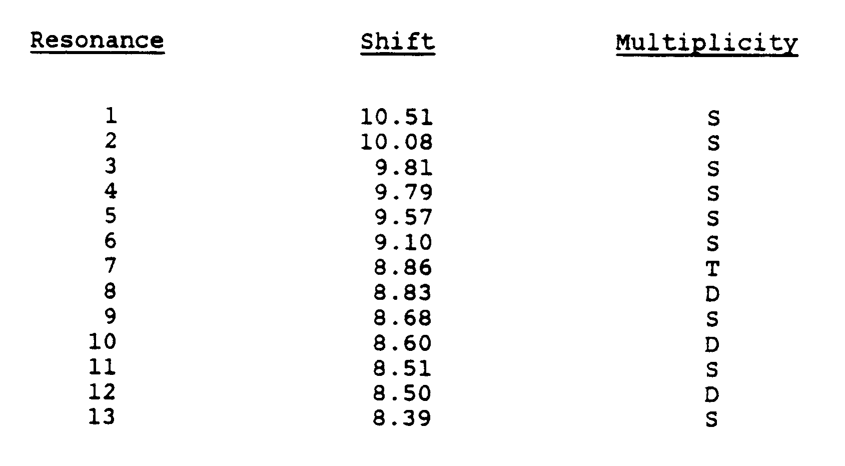

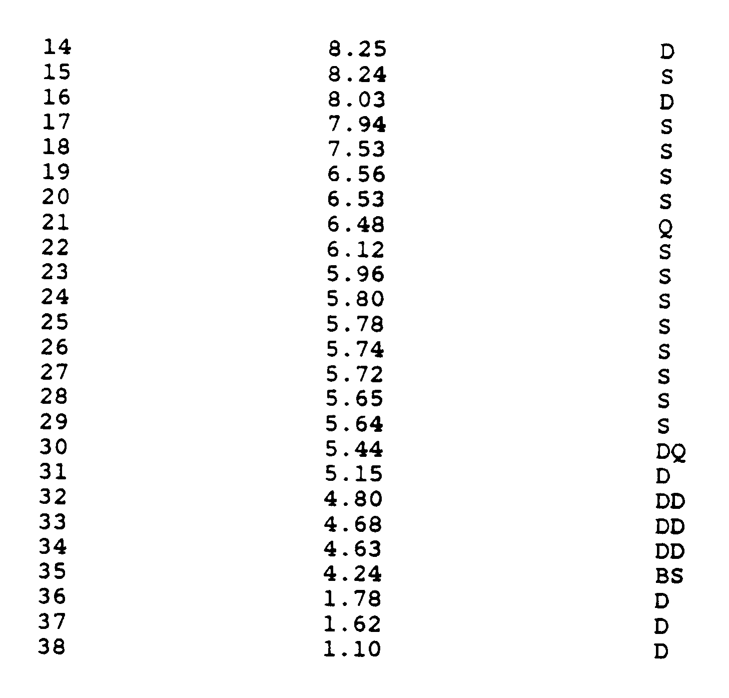

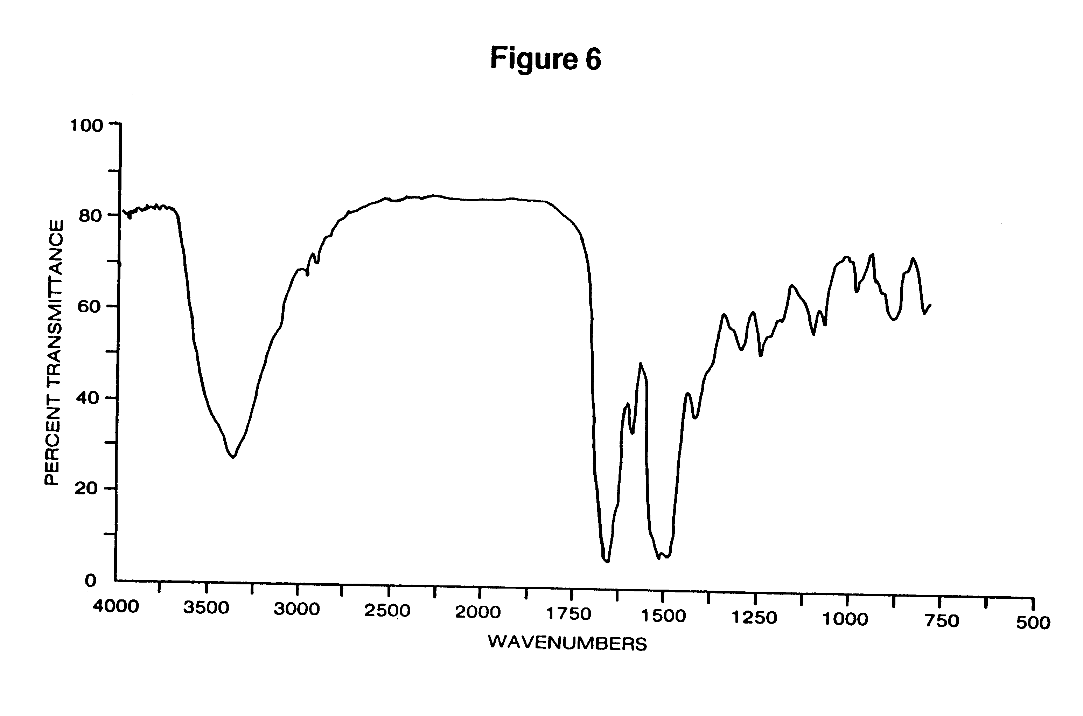

The infrared spectrum (KBr disc) for A10255 factor H is set forth in Fig. 6 of the accompanying drawings.

The molecular weight of A10255 factor H was determined by fast atom bombardment mass spectrometry to be approximately 1160; the exact mass was determined to be 1161.32 (M + H) using CsI as reference standard.

The A10255-producing parent strain S. gardneri NRRL 15537, also identified originally as A10255.1, has been classified as such by taxonomic studies carried out as described by the following paragraphs.

### Taxonomy of the A10255.1 Strain

Taxonomic studies of the A10255.1 strain were carried out by Mr. Frederick P. Mertz of the Lilly Research Laboratories. Based on these studies, the organism is classified as a new strain of Streptomyces gardneri (Waksman 1942) Waksman 1961 ATCC 23911. This classification is based on an examination of published descriptions of this species [R. E. Buchanan, and N. E. Gibbons (eds.), "Bergey's Manual of Determinative Bacteriology", 8th Edition, The Williams and Wilkins Co., Baltimore, 1974; E. B. Shirling and D. Gottlieb, "Cooperative Description of Type Cultures of Streptomyces", Int. J. Syst. Bacteriol. 18(4):279-392 (1968); and S. A. Waksman, "The Actinomycetes Vol. II", The Williams and Wilkins Co., Baltimore, 1961] and simultaneous laboratory comparsions.

The methods recommended by the International Streptomyces Project (ISP) for the characterization of Streptomyces species [E. B. Shirling and D. Gottlieb, "Methods for Characterization of Streptomyces Species", Int. J. Syst. Bacteriol. 16(3), 313-340 (1966)] were followed along with certain supplementary tests [D. J. Blazevic and G. M. Ederer, "Principles of Biochemical Tests in Diagnostic Microbiology", John Wiley and Sons, Inc., New York, 1975].

Carbon utilization was determined on ISP No. 9 basal medium to which filter-sterilized carbon sources were added to equal a final concentration of 1.0 percent. Plates were incubated at 30°C and read after 14 days.

Melanoid pigment production (chromogenicity) was determined with ISP No. 1 (tryptone-yeast extract broth), ISP No. 6 (peptone-yeast extract iron agar), ISP No. 7 (tyrosine agar), and modified ISP No. 7 which has tyrosine removed.

Starch hydrolysis was determined by testing for the presence of starch with iodine on ISP No. 4 (inorganic salts-starch agar) plates (see Blazevic and Ederer, supra).

Morphology was studied using an optical light microscope. A scanning electron microscope (SEM) was used to study the spore surface ornamentation.

Sodium chloride tolerance was measured by adding sodium chloride to ISP No. 2 agar to equal the concentration desired.

ICSS-NBS Centroid Color Charts, standard sample No. 2106 (National Bureau of Standards, 1958, U.S. Department of Commerce, Washington, D.C.) and the Color Harmony Manual (4th ed., Color Standards Department, Container Corporation of America, Chicago, Illinois, 1958) were used to assign color names.

The cell wall sugars were determined with the procedure of M. P. Lechevalier, "Identification of Aerobic Actinomycetes of Clinical Importance," J. Lab. Clin. Med., 71, 934-944 (1968). The isomers of diaminopimelic acid (DAP) were established by the chromatographic methods set forth in B. Becker, M. P. Lechevalier, R. E. Gordon, and H. A. Lechevalier, "Rapid Differentiation between Nocardia and Streptomyces by Paper Chromatography of Whole-cell Hydrolysates", Appl. Microbiol 11, 421-423 (1964).

### Cultural Characteristics

A10255.1 is characterized by limited vegetative and very poorly developed aerial mycelia. The aerial mycelia has a spore mass color in the white (W) to gray (GY) color series. The nearest matching color tab in the Tresner and Backus system [Color Harmony Manual, supra, and E. J. Backus and H. D. Tresner, "System of Color Wheels for Streptomyces Taxonomy, Appl. Microbiol., 11, 335-338 (1956)] for the white color series is b oyster white and in the gray color series is d light gray. This cultural feature is best observed on glycerol asparagine agar (ISP No. 5). Aerial mycelia is so poorly developed on most media that a color determination is very difficult.

The reverse side of this culture has no distinctive pigments. The color of the reverse side is orange-yellow to yellow-brown and the color is unaffected by pH. The only soluble pigment produced is a light brown pigment on tyrosine agar (ISP No. 7) and tomato paste oatmeal agar (TPO) and a light-orange pigment produced on Glycerol-Glycine agar. When plated for variability, this culture was stable and homogeneous.

Cultural characteristics of the A10255.1 strain and S. gardneri ATCC 23911 are set forth below in Table 1.

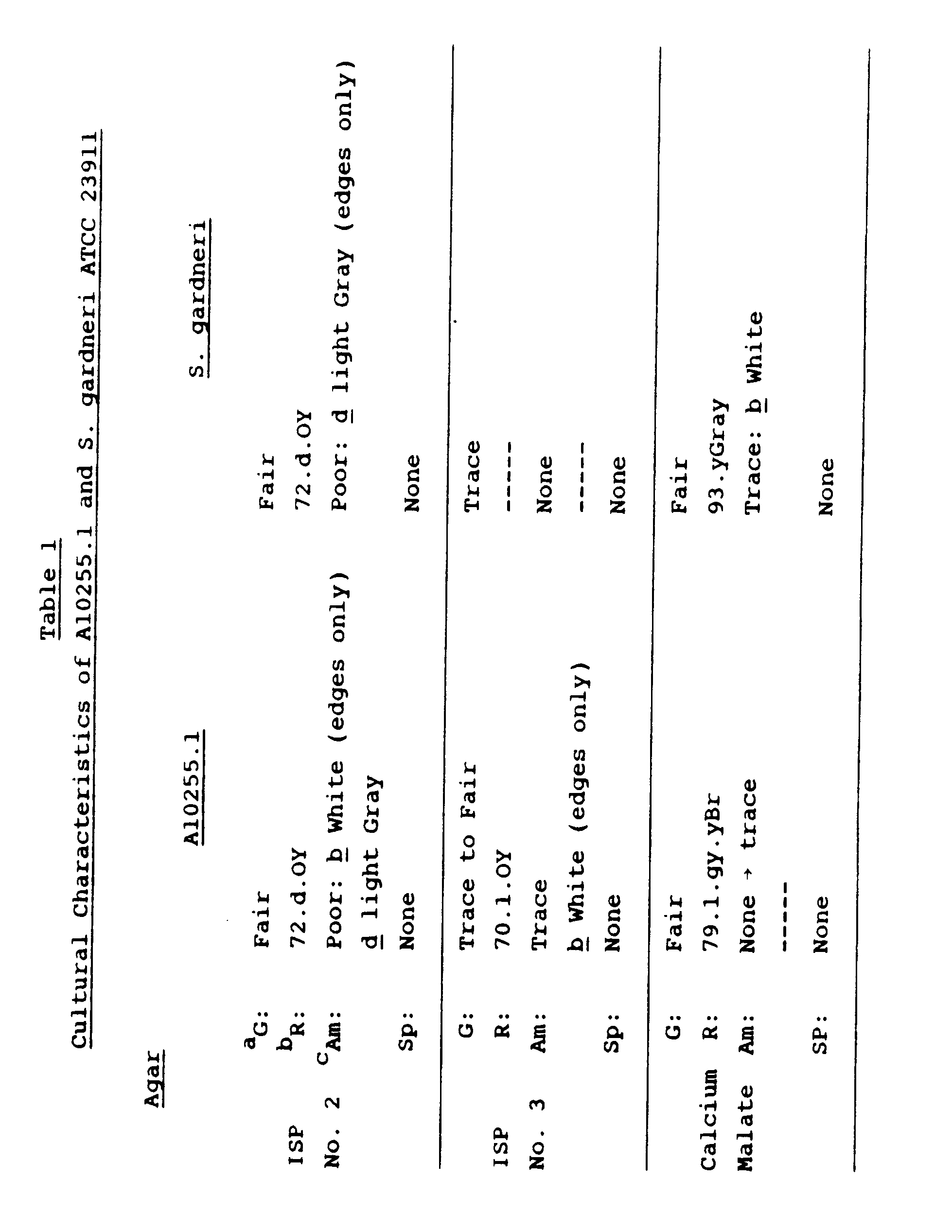

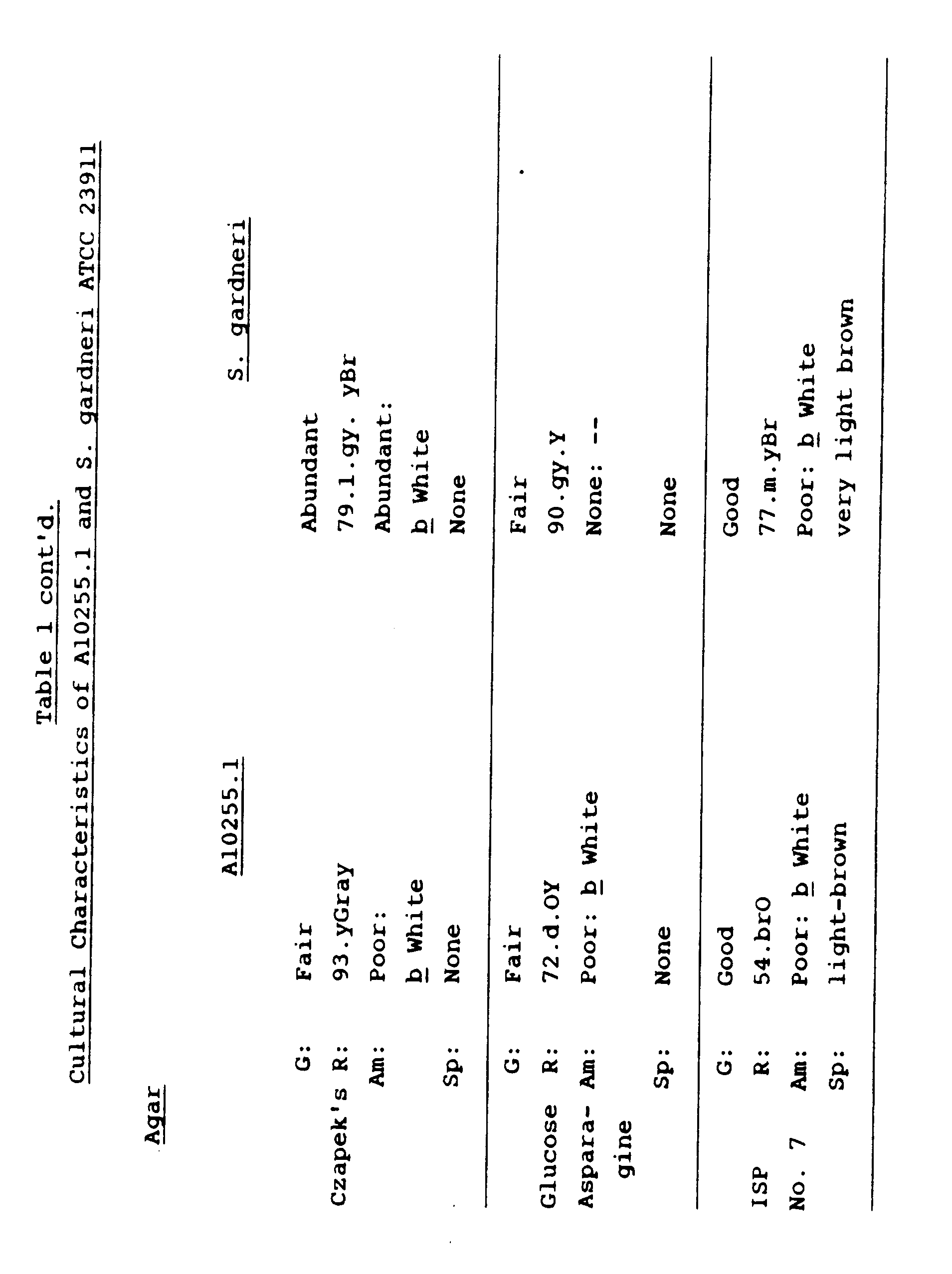

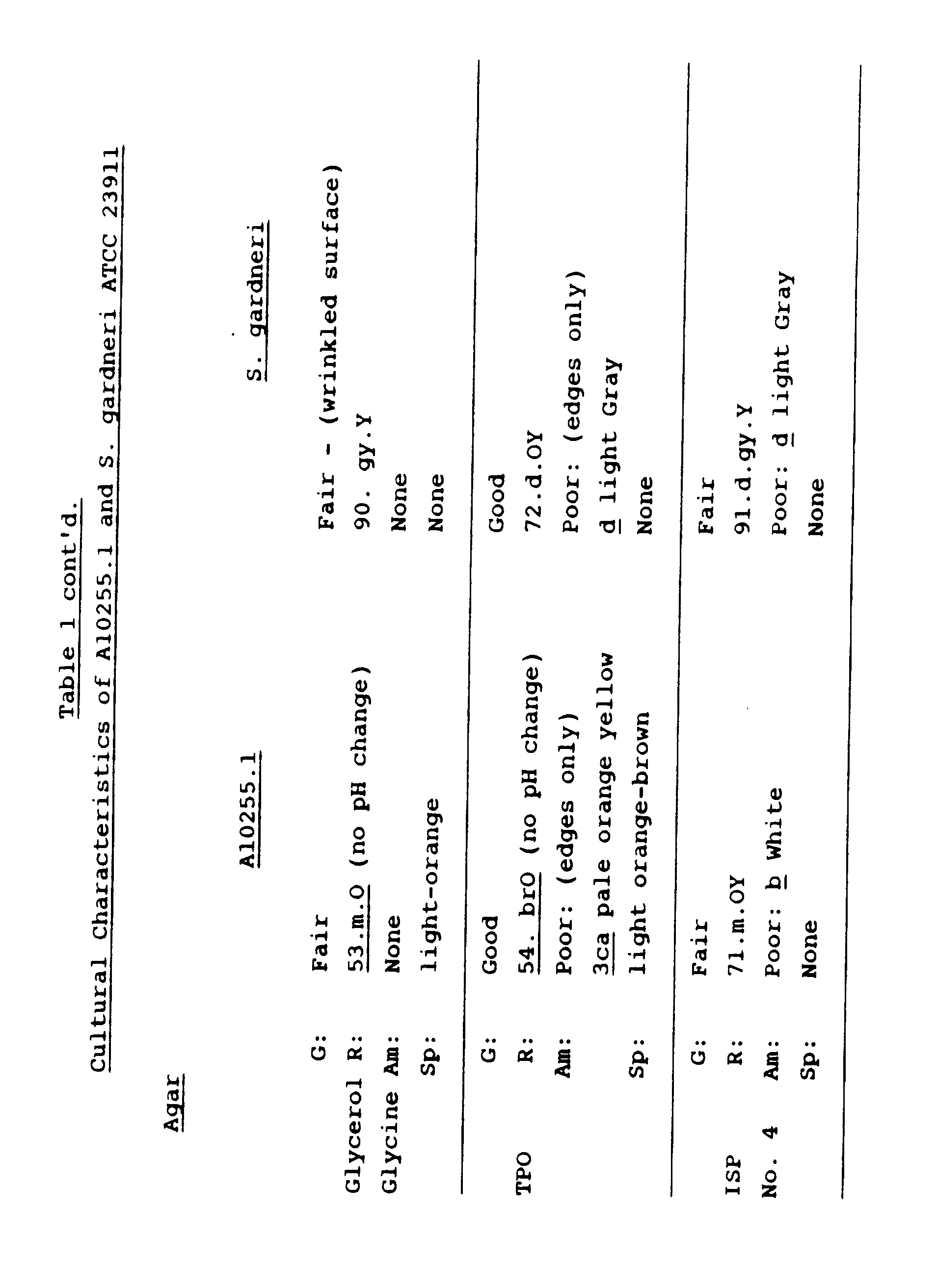

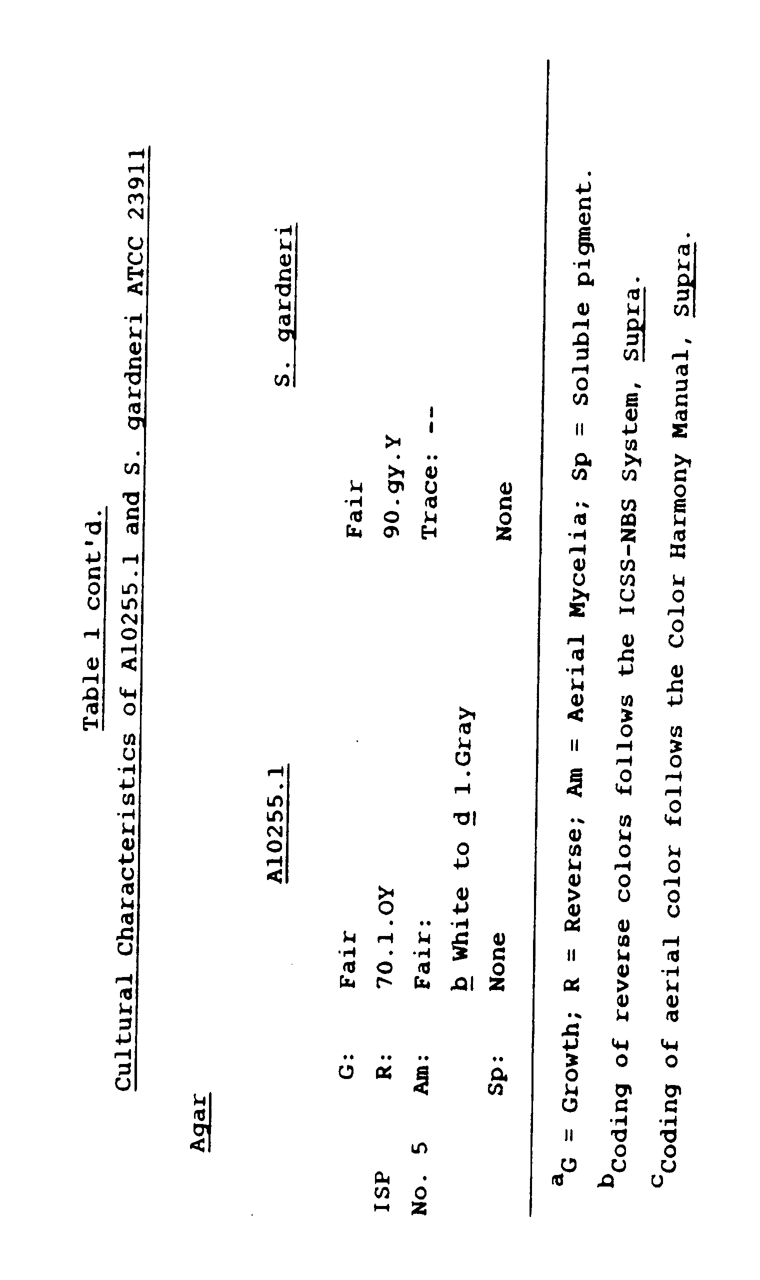

### Morphological Characteristics

Culture A10255.1 produces a poorly developed non-fragmenting aerial mycelium which is monopodially branched. Sporophores are arranged as straight and flexuous branches. No spirals, sclerotia, sporangia, or motile spores were observed. A10255.1 is placed in the Rectus-flexibilus (RF) section of Pridham et al. [T. G. Pridham et al., "A Guide for the Classification of Streptomyces According to Selected Groups", Appl. Microbiol., 6, 52-79 (1957)].

The same morphology is observed on all media where aerial mycelia could be observed. Mature spore chains generally contain from 10 to 50 spores per chain.

The spore shape is cylindrical. The spore size ranges from 0.9 - 1.0 µM in length and 0.5 - 0.6 µM in width. The average size is 1.6 X 0.6 µM. The spore surface ornamentation is smooth.

### Physiological Characteristics

Whole cell hydrolysates contain LL-diaminopimelic acid with no meso isomer present. Sugars present in whole cell hydrolysates were glucose, mannose, and ribose. These characteristics represent a Type I cell wall and a NC, or no characteristic, sugar pattern [M. P. Lechevalier, supra]. This combination of major cell wall constituents is indicative of the genus Streptomyces [M. P. Lechevalier, supra, and R. E. Buchanan and N. E. Gibbons (eds)., supra].

The carbon utilization pattern for A10255.1 is as follows: L-arabinose, D-fructose, D-galactose, D-glucose, i-Inositol, raffinose, and D-xylose are utilized for growth. D-mannitol, L-rhamnose, salicin and sucrose do not support growth. Table 2 below compares the carbon utilization patterns observed for A10255.1 and S. gardneri ATCC 23911.

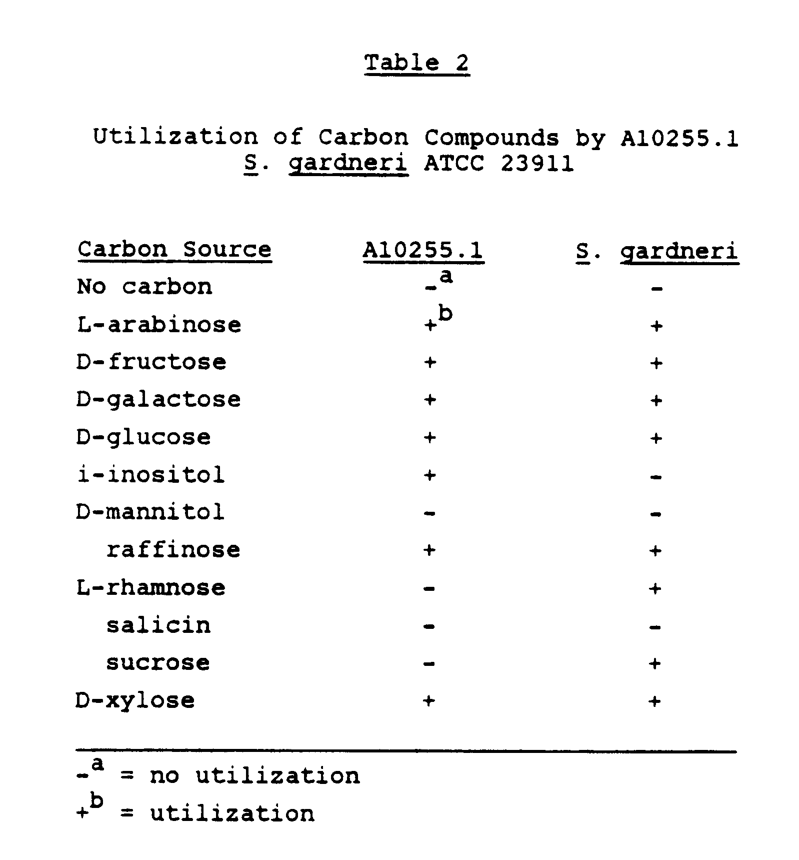

Culture A10255.1 hydrolyzed starch and partially hydrolyzed skim milk, produced catalase, liquified gelatin, and reduced nitrates to nitrites.

A10255.1 will tolerate up to 6 percent sodium chloride and will grow at temperatures ranging from 4° to 40°C.

Melanoid pigments are produced when A10255.1 is grown in tryptone-yeast extract broth (ISP No. 1) and on slants of peptone-yeast extract iron agar (ISP No. 6). No melanoid pigments were produced on slants of tyrosine agar (ISP No. 7).

### Species Determination

Using the cultural, morphological, and physiological characteristics of A10255.1, comparison was made with the published descriptions of similar species. Four species of Streptomyces were selected to examine in simultaneous laboratory comparison:
Streptomyces aureofasciculus^{a}
Streptomyces aureomonopodiales^{a}
Streptomyces flavochromogenes^{b}
Streptomyces gardneri^{c}
^{a}E. B Shirling and D. Gottlieb, "Cooperative Description of Type Cultures of Streptomyces", Int. J. Syst. Bacteriol., 19(4), 375-390 (1969).
^{b}E. B. Shirling and D. Gottlieb, "Cooperative Description of Type Cultures of Streptomyces", Int. J. Syst. Bacteriol. 22(4), 265-394 (1972).
^{c}E. B. Shirling and D. Gottlieb, "Cooperative Description of Type Cultures of Streptomyces", Int. J. Syst. Bacteriol. 18(4), 279-392 (1968).

Laboratory comparisons indicated significant differences with S. aureofasciculus and S. flavochromo genes, and good agreement with S. aureomonopodiales and S. gardneri. However, as S. aureomonopodiales is not in the Approved List of Bacterial Names, it was removed from consideration.

A10255.1 is quite similar to S. gardneri in cultural, morphological, and physiological characteristics. The predominate cultural feature that distinguishes both strains is the very poor formation of aerial hyphae on most media. S. gardneri is described in the literature as belonging in the Gray (GY) series. However, in the original description by Waksman [S. A. Waksman, supra], and in laboratory comparisons with A10255.1, it produces white (W) and gray (GY) aerial hyphae. The reverse color of both cultures is almost identical. Both cultures possess a Rectus-flexibilus (RF) morphology, smooth spore surface ornamentation, cylindrical spore shape, and chains of 10 - 50 spores.

Catalase production, liquefaction of gelatin, melanoid pigment production, reduction of nitrate, and hydrolysis of milk and starch were the same for both strains.

Differences between A10255.1 and S. gardneri are minimal. S. gardneri had less tolerance to sodium chloride and a lower temperature range than A10255.1. The utilization of L-rhamnose, sucrose, and inability to utilize i-inositol distinguish S. gardneri from A10255.1. These similarities and differences are summarized below.

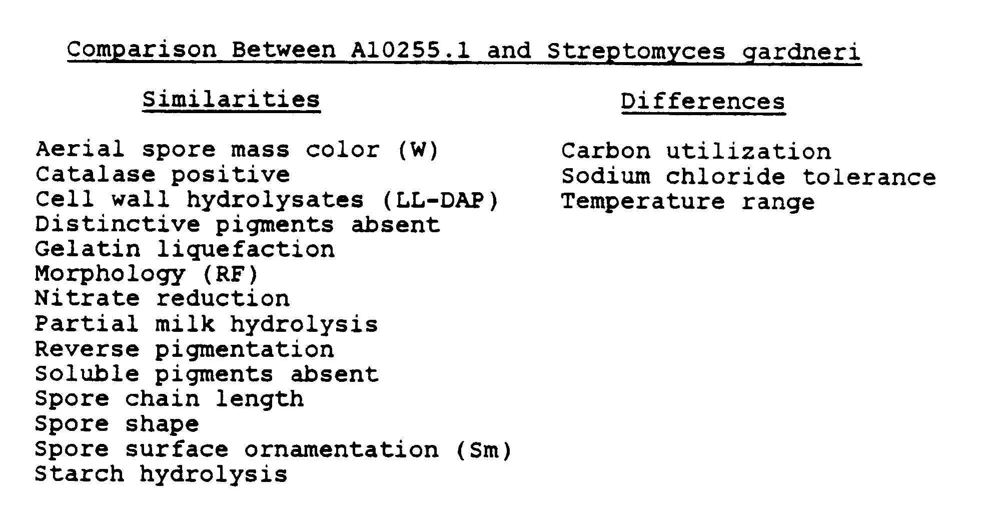

The similarities and differences between the two cultures are set forth below in detail in Table 3:

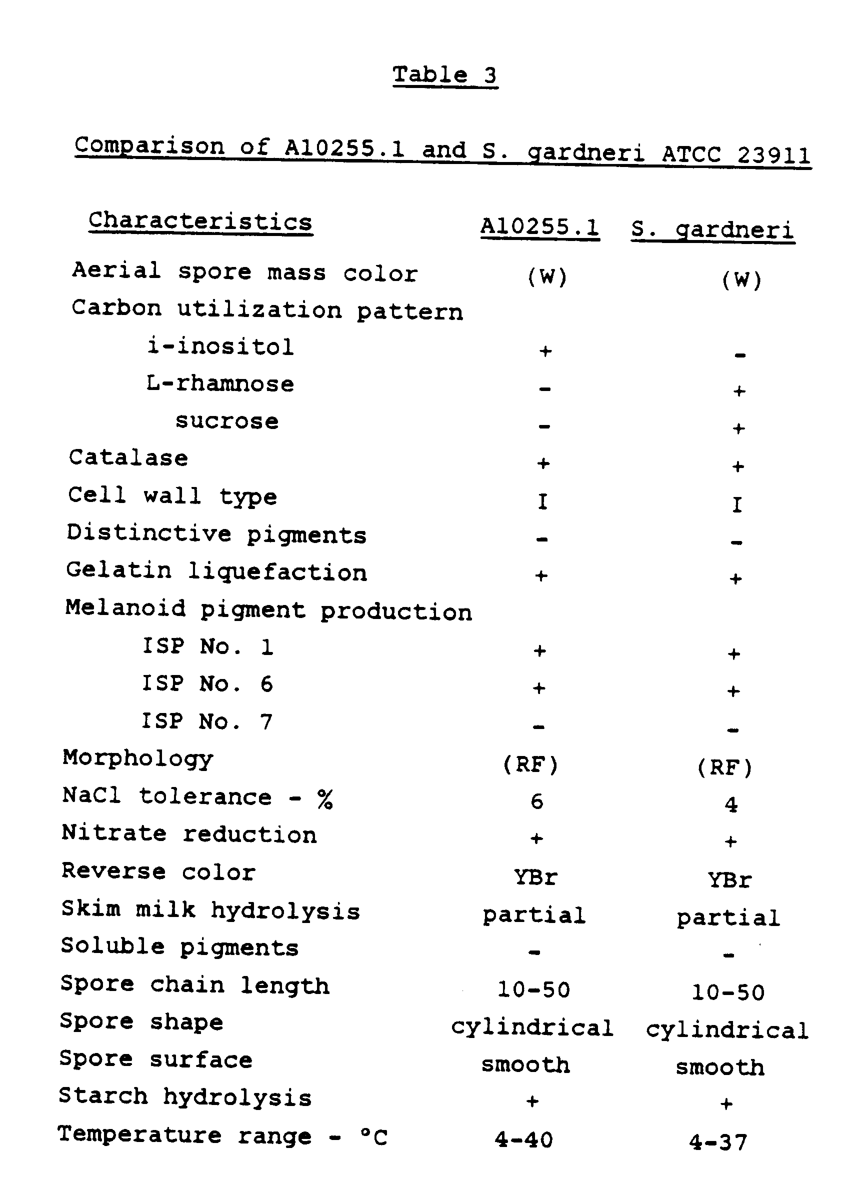

The results of the above comparisons indicate the A10255.1 is very similar to S. gardneri. Therefore culture A10255.1 is classified as a strain of Streptomyces gardneri (Waksman, 1942) Waksman 1961, ATCC 23911. S. gardneri is recognized in the Approved List of Bacterial Names [V. B. D. Skerman et al., "Approved Lists of Bacterial Names", Int. J. Syst. Bacteriol., 30 (1), 225-420 (1980)] and consequently is a validly published species.

It should be mentioned that Kurylowicz et al. [W. Kurylowicz, A. Paszkiewicz, W. Woznicka, and W. Kurzatkowski, "Numerical Taxonomy of Streptomyces", Polish Medical Publishers, 1975], when classifying Streptomyces, in both the Wroclaw dentrite of similarity and the Overall Similarity Method numerical methods place S. aureomonopodiales and S. gardneri in the same cluster. A dendrogram based on this study relates these two strains at a percentage similarity of 94. This similarity suggests that a distinction in species is not justified.

The Streptomyces gardneri culture described above has been deposited and made a part of the stock culture collection of the Northern Regional Research Division, U.S. Department of Agriculture, Agricultural Research Service, Peoria, IL, 61604. Upon issuance of the instant specification, the culture will be made available to the public from this branch of the Department of Agriculture under the number NRRL 15537 and otherwise as required by Rule 28 EPC.

### Description of the A10255.10 Strain (NRRL 18260)

The A10255.10 strain NRRL 18260 is derived from the parent strain A10255.1, NRRL 15537, by a series of sequential treatments with NTG (N-nitrosoguanidine). NRRL 18260 has been deposited and made part of the stock culture collection of the Northern Regional Research Division, U.S. Department of Agriculture, Agricultural Research Service, Peoria, IL, 61604. Upon issuance of the instant specification, the culture will be made available to the public from this brach of the Department of Agriculture and otherwise as required by Rule 28 EPC.

### Taxonomy of the A10255.2 Strain

The A10255.2 strain (referred to above as the Streptomyces gardneri strain NRRL 15922) is an NTG (N-nitrosoguanidine) mutant of the A10255.1 strain. The latter strain is Streptomyces gardneri NRRL 15537.

There are many similarities in the taxonomies of the A10255.1 and A10255.2 strains. The taxonomic comparison between the A10255.1 and A10255.2 strains are set below in tabular form.

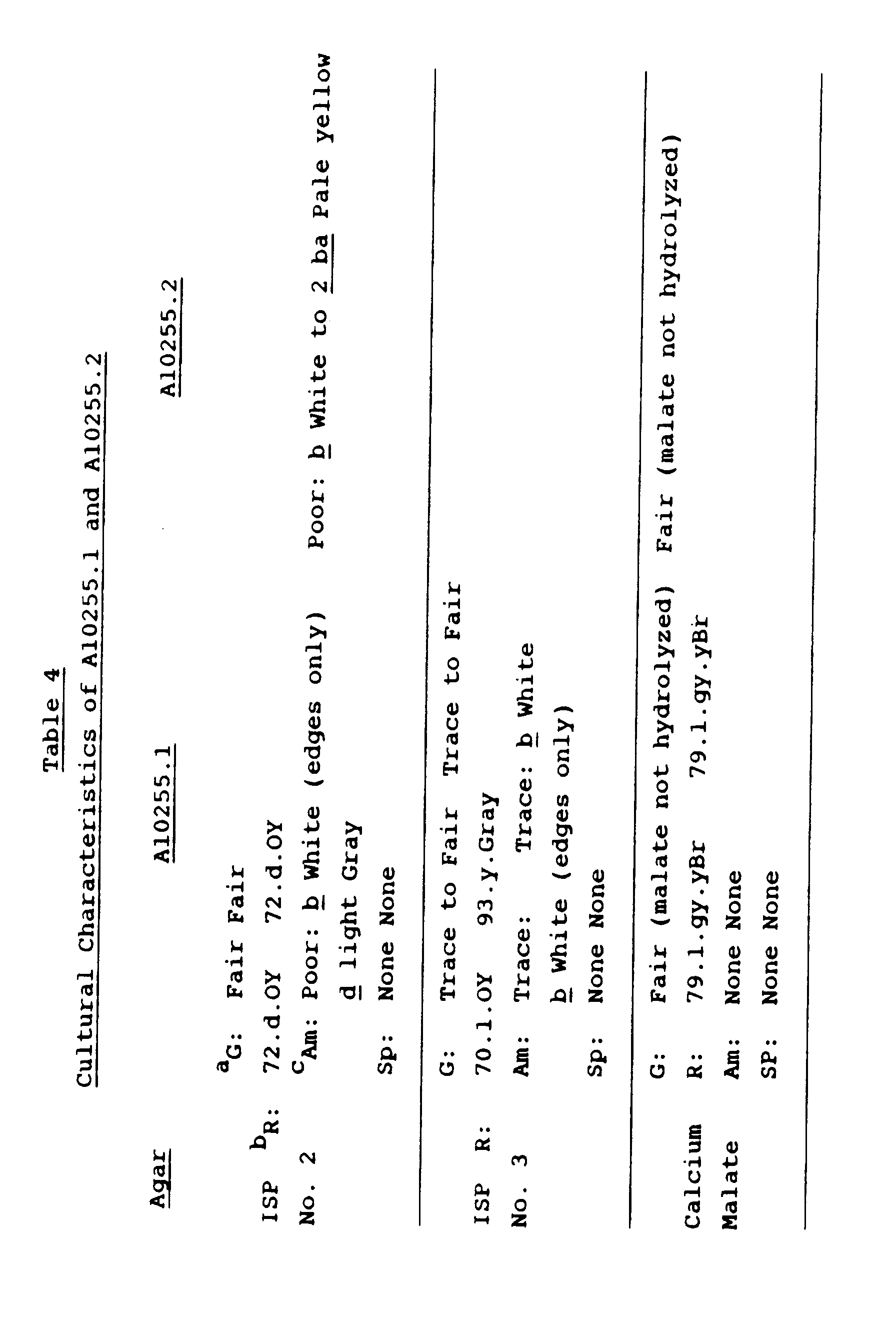

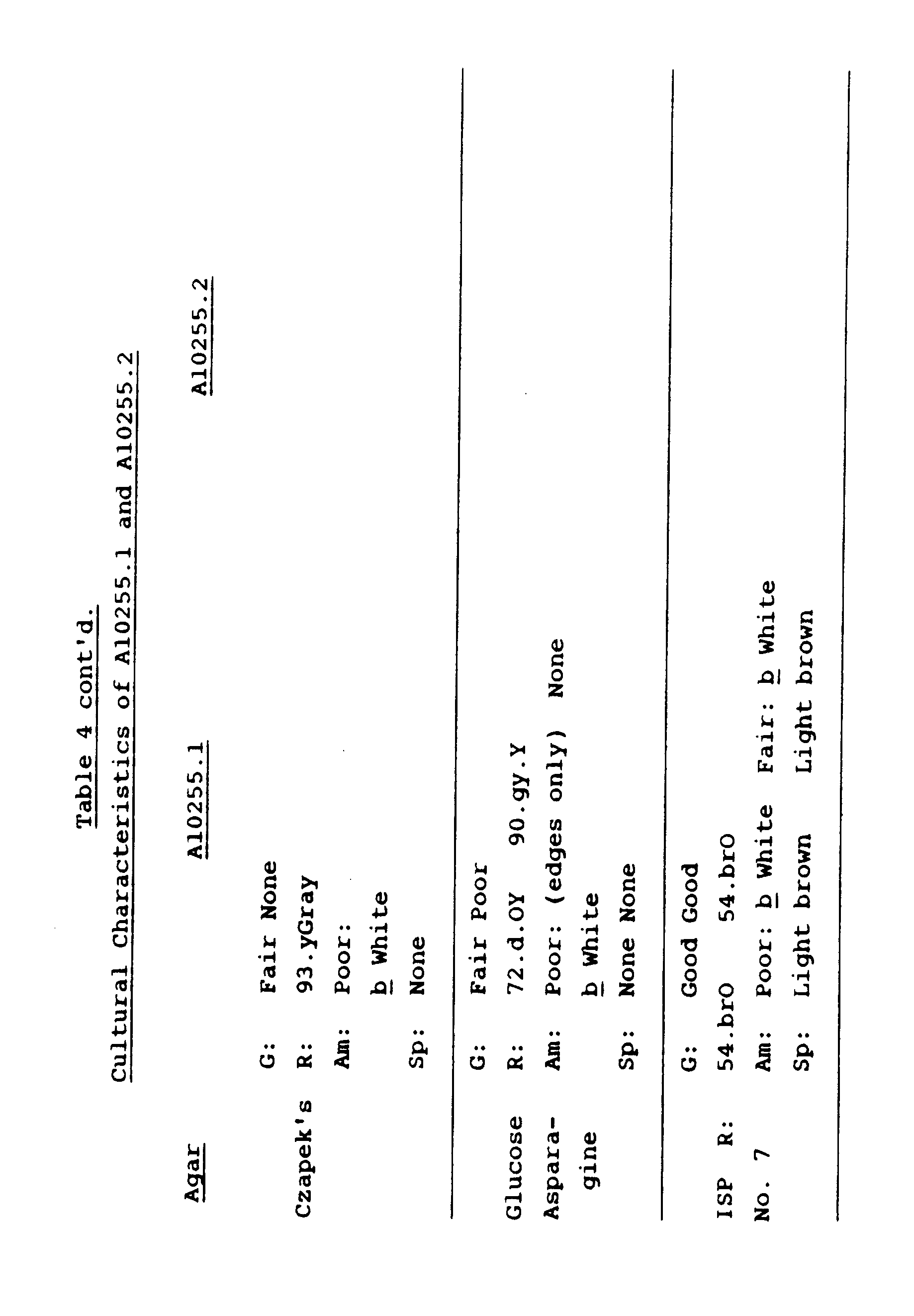

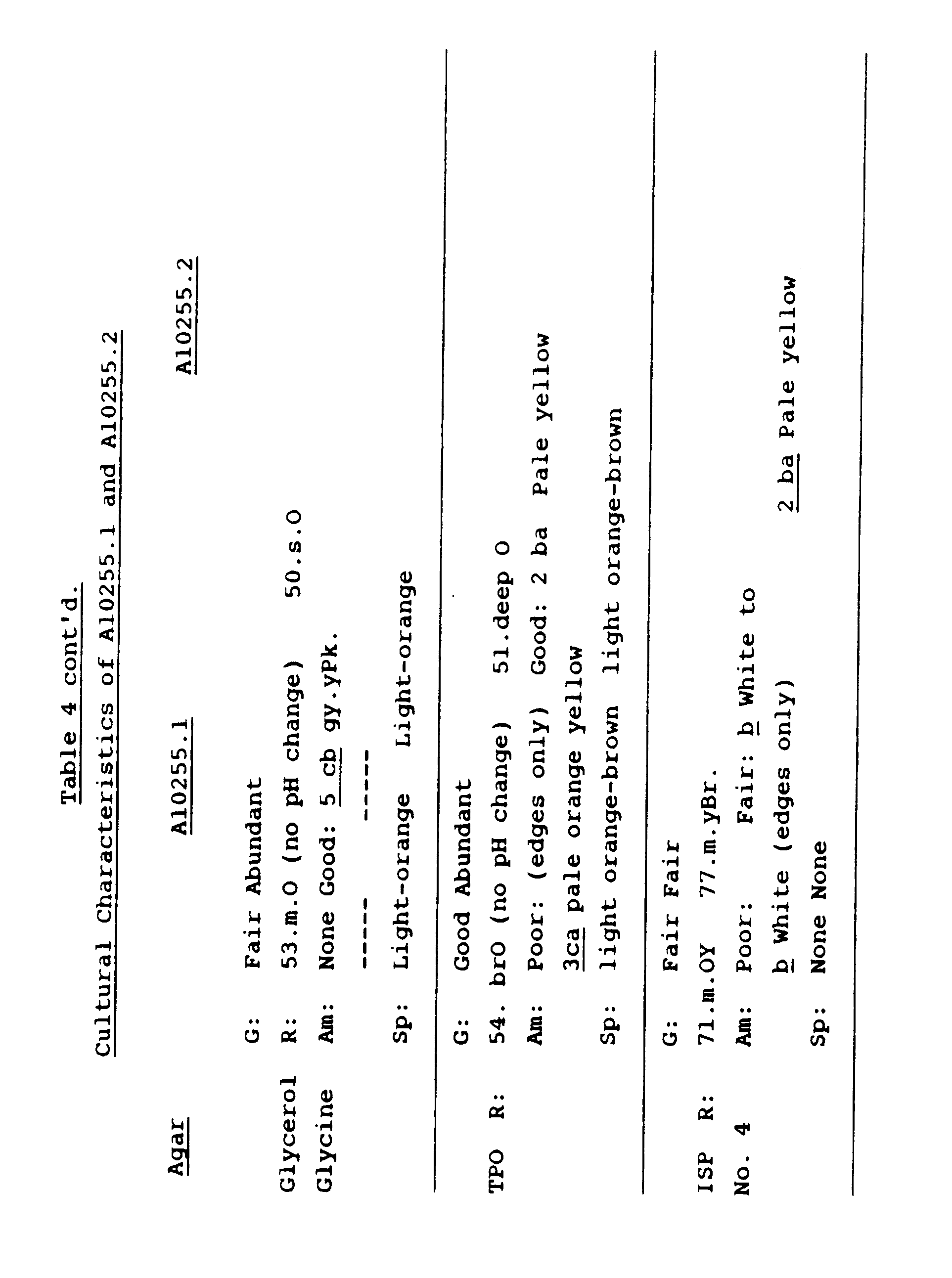

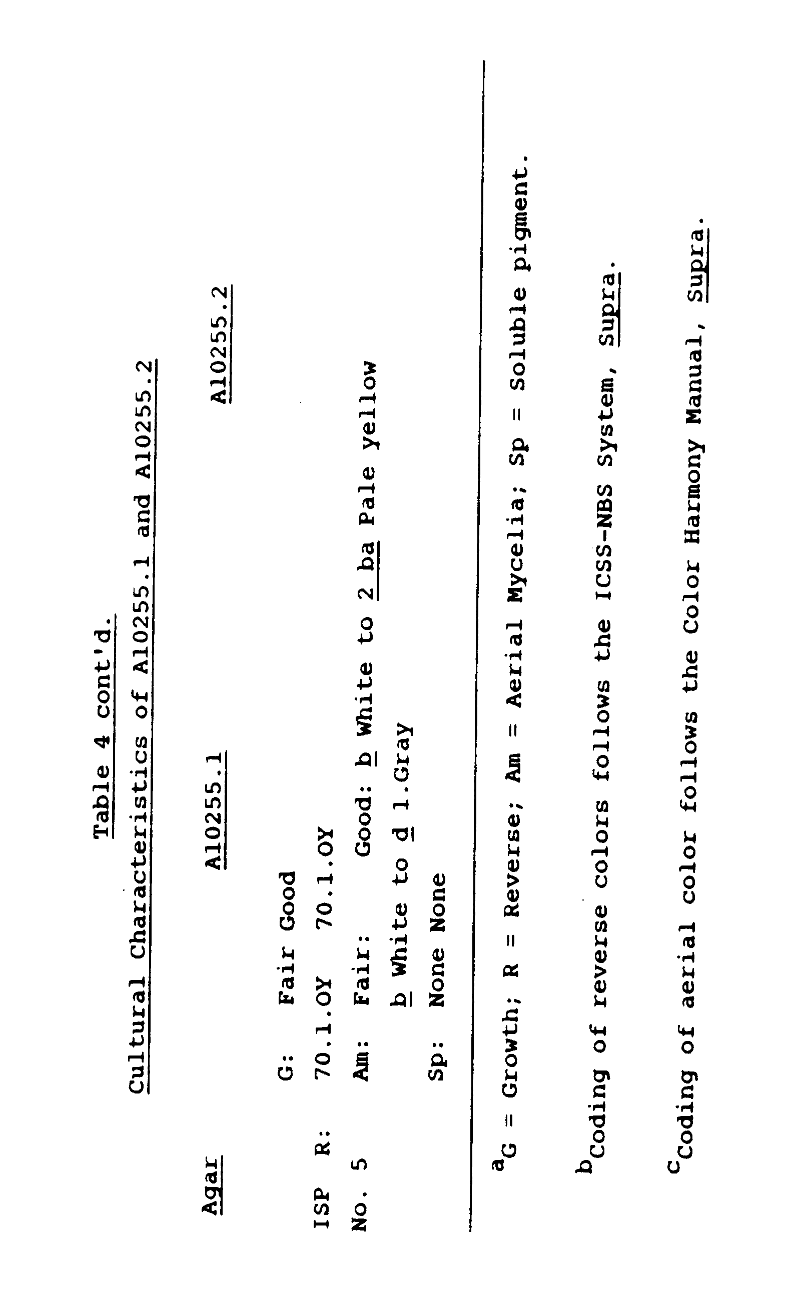

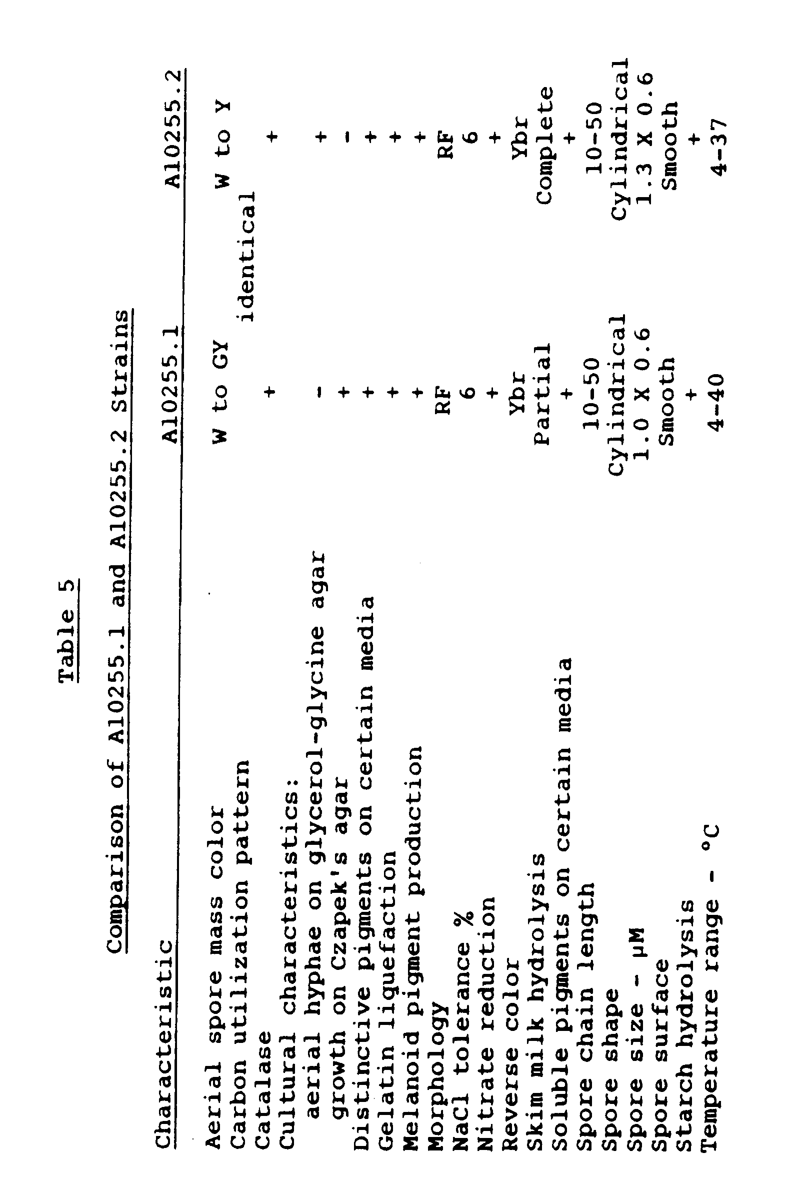

The Streptomyces gardneri culture A10255.2 described above has been deposited and made a part of the stock culture collection of the Northern Regional Research Division, U.S. Department of Agriculture, Agricultural Research Service, Peoria, IL, 61604. Upon issuance of the instant specification, the culture will be made available to the public from this branch of the Department of Agriculture under the number NRRL 15922 and otherwise as required by Rule 28 EPC.

### Production of the Antibiotic Factors and Their Biological Activity

The A10255 antibiotic complex can be produced by culturing the prevoiusly undescribed microorganism Streptomyces gardneri, NRRL 15537, strain NRRL 18260, or strain NRRL 15922, or an A10255-producing mutant thereof, in a culture medium containing assimilable sources of carbon, nitrogen, and inorganic salts, under submerged aerobic fermentation conditions until the A10255 antibiotic complex is produced, and preferably until a substantial level of antibiotic activity is produced. Most of the antibiotic activity is generally found associated with the mycelia, while minor amounts of antibiotic activity are found in the broth. The A10255 complex is most readily separated from the fermentation mixture by removal of the mycelia (the biomass) by filtration. The broth is generally discarded. The antibiotic complex is then isolated from the mycelia.

The parent strain NRRL 15537 produces factor B as the most abundant factor. The mutant strain NRRL 18260 produces B and G as the most abundant factors. The remaining factors C, E, F and H have thus far been produced in only minor amounts with either strain.

The mycelia are extracted with polar solvents (such as 4:1 acetone:water), concentrated, acidified, again extracted with an organic solvent (ethyl acetate, for example), and the resultant solutions are concentrated to precipitate the A10255 complex.

The A10255 complex can be used without further purification and mixed directly into animal feed or animal feed premix. Alternatively, the A10255 antibiotic complex can be further purified and separated into its individual factors by well-known chromatographic techniques such as thin layer chromatography, column chromatography, and especially various high performance liquid chromatography procedures. Some specific procedures for isolating the individual factors are discussed in the Experimental Section.

A number of different media may be used with NRRL 15537, NRRL 18260, or NRRL 15922 to produce the A10255 complex. For economy in production, optimal yield, and ease of product isolation, however, certain culture media are preferred. These media should contain assimilable sources of carbon, nitrogen, and inorganic salts. Suitable carbon sources include glucose, starch, maltose, fructose, and glycerol. Optimum levels of carbon sources are from about 2 to about 5 percent by weight.

Preferred nitrogen sources include acid digest of soybeans, acid or enzymatic digests of casein, ammonium salts, nitrate salts, glycine, alanine, serine, asparagine, and glutamine.

Essential trace elements necessary for the growth and development of the organism may occur as impurities in other constituents of the media in amounts sufficient to meet the growth and biosynthetic requirements of the organism. However, it may be beneficial to incorporate in the culture media additional soluble nutrient inorganic salts capable of yielding sodium, potassium, magnesium, calcium, ammonium, ferrous, chloride, carbonate, phosphate, sulfate, nitrate, and like ions.

Although small quantities of the A10255 antibiotic may be obtained by shake-flask culture, submerged aerobic fermentation in tanks is a preferred method for producing substantial quantities of the A10255 antibiotic. For tank fermentation, it is preferable to use a vegetative inoculum. The vegetative inoculum is prepared by inoculating a small volume of culture medium with the spore form, or mycelial fragments, to obtain a fresh, actively growing culture of the organism. The vegetative inoculum is then transferred to a larger tank where, after a suitable incubation time, the A10255 antibiotic is produced in optimal yield.

The A10255-producing organisms produce the A10255 complex over a temperature range of from about 23° to about 37°C. Optimum production of A10255 antibiotic complex appears to occur at a temperature of about 30° to about 32°C.

As is customary in aerobic submerged culture processes, sterile air is dispersed through the culture medium. For efficient growth of organism, the volume of air used in tank production is in the range of from about 0.1 to about 0.5 volumes of air per volume of culture medium per minute (v.v.m), with from about 100 to about 300 RPM agitation. An optimum rate in a 165-liter vessel containing 100 liters of fermentation medium is about 0.125 v/v/m, with agitation provided by an impeller rotating at about 200 RPM.

The fermentation generally produces antibiotic activity after about 40 hours. Peak antibiotic production occurs at from about 110 hours to about 140 hours fermentation time.

Production of the A10255 antibiotic can be monitored during the fermentation by either agar diffusion using Bacillus subtilis ATCC 6633, or by a turbidimetric method using Staphylococcus aureus ATCC 9114.

The A10255 complex and individual factors are antimicrobial agents and are especially active against gram-positive microorganisms, as illustrated by the following in vitro and in vivo test data. In the following Table 6 is presented the minimum inhibitory concentration, (MIC, in micrograms/milliliters), for the factors against a sampling of pathogenic gram-positive and gram-negative bacteria. The MIC values were obtained by the standard agar dilution test method.

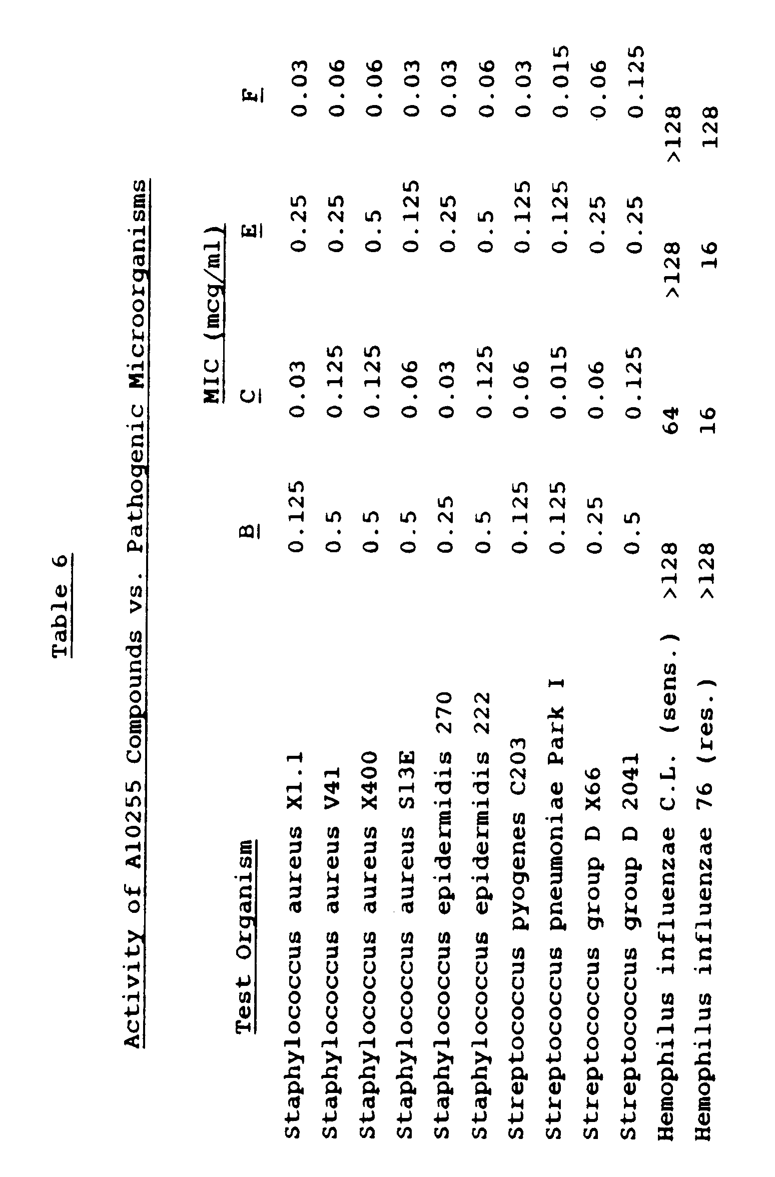

The A10255 compounds also demonstrate excellent antimicrobial activity against a number of Clostridium difficile strains. In particular, in standard agar dilution tests the A10255 complex and factors B, C, E, and F exhibited MIC's of less than or equal to 0.03 microgram/milliliter. By comparison, in the same tests for the complex and factors B and C, the antibiotic vancomycin exhibited an MIC of 2 or 4 microgram/milliliter.

The A10255 complex and factor C were tested against several Bacteroides species and demonstrated excellent antimicrobial activities. The results of this agar-dilution test is set forth below in Table 7.

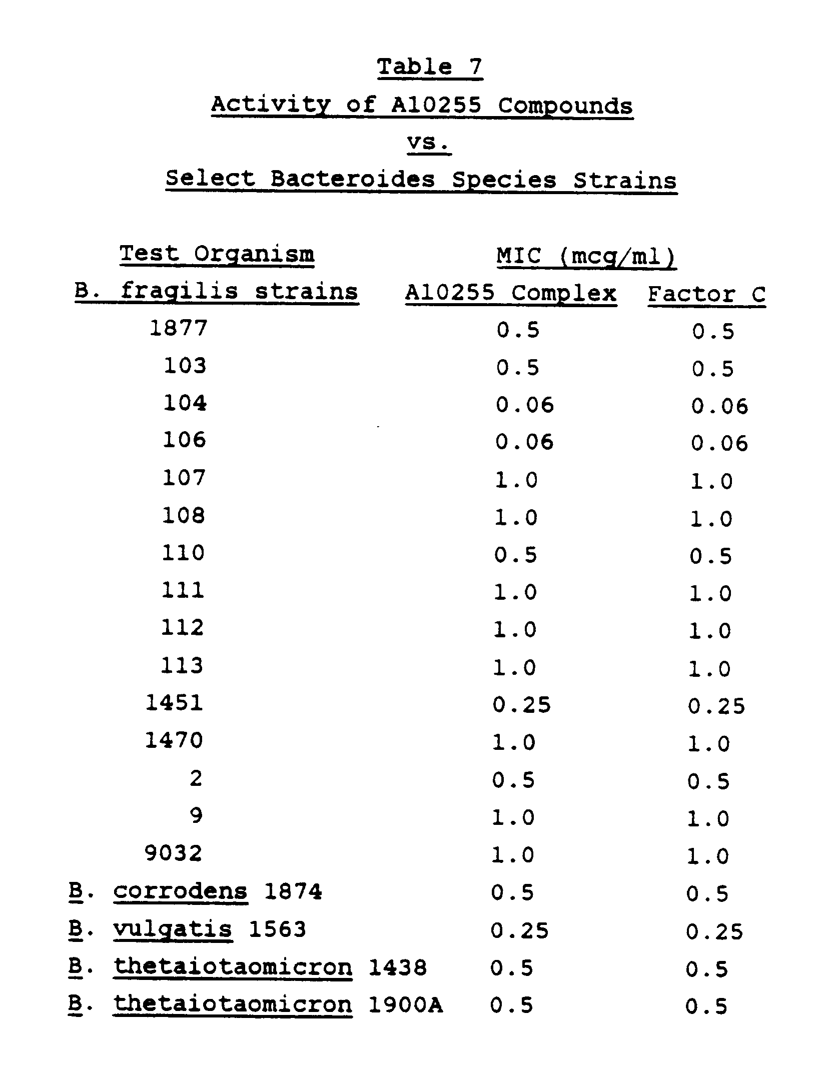

The A10255 complex and the individual factors have demonstrated antimicrobial activity against a wide variety of anaerobic microorganisms. The antimicrobial activity is set forth below in Table 8. The results set forth in the Table are MIC values for a standard agar dilution test.

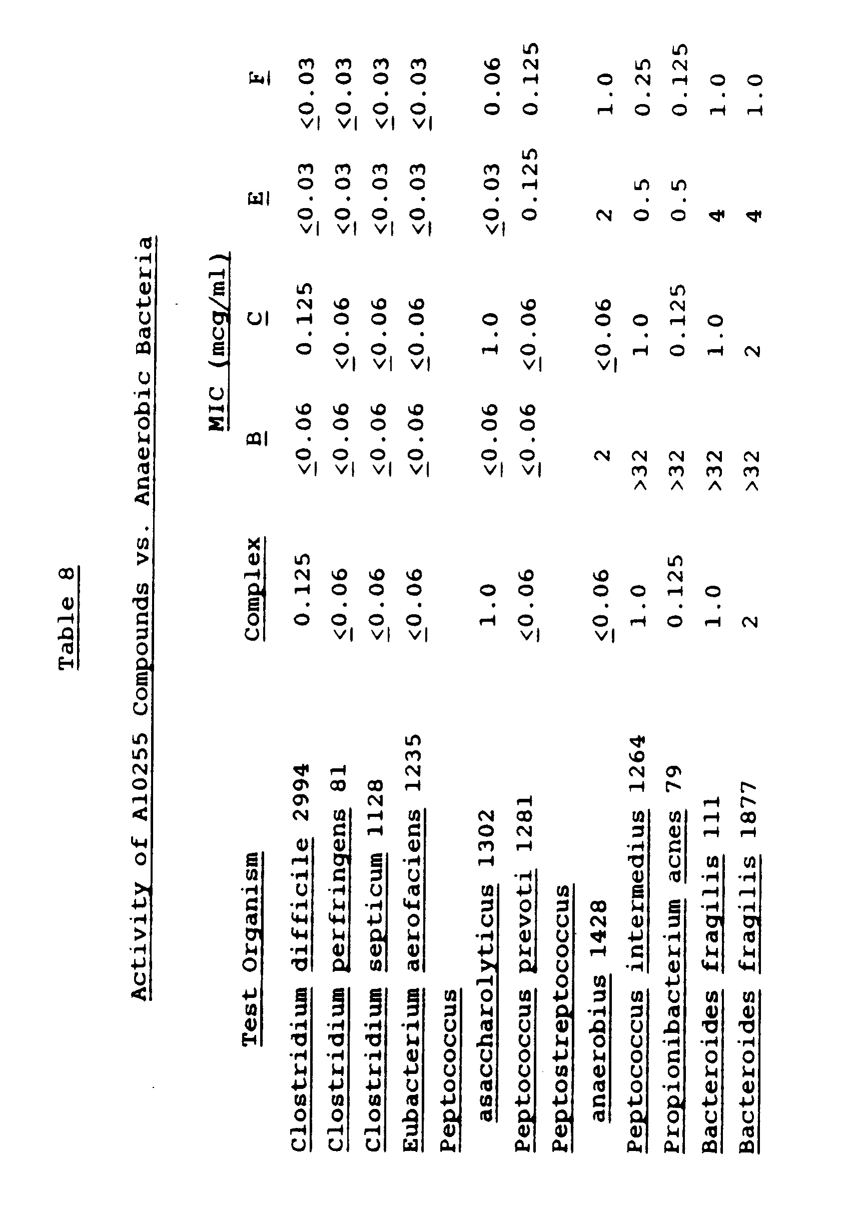

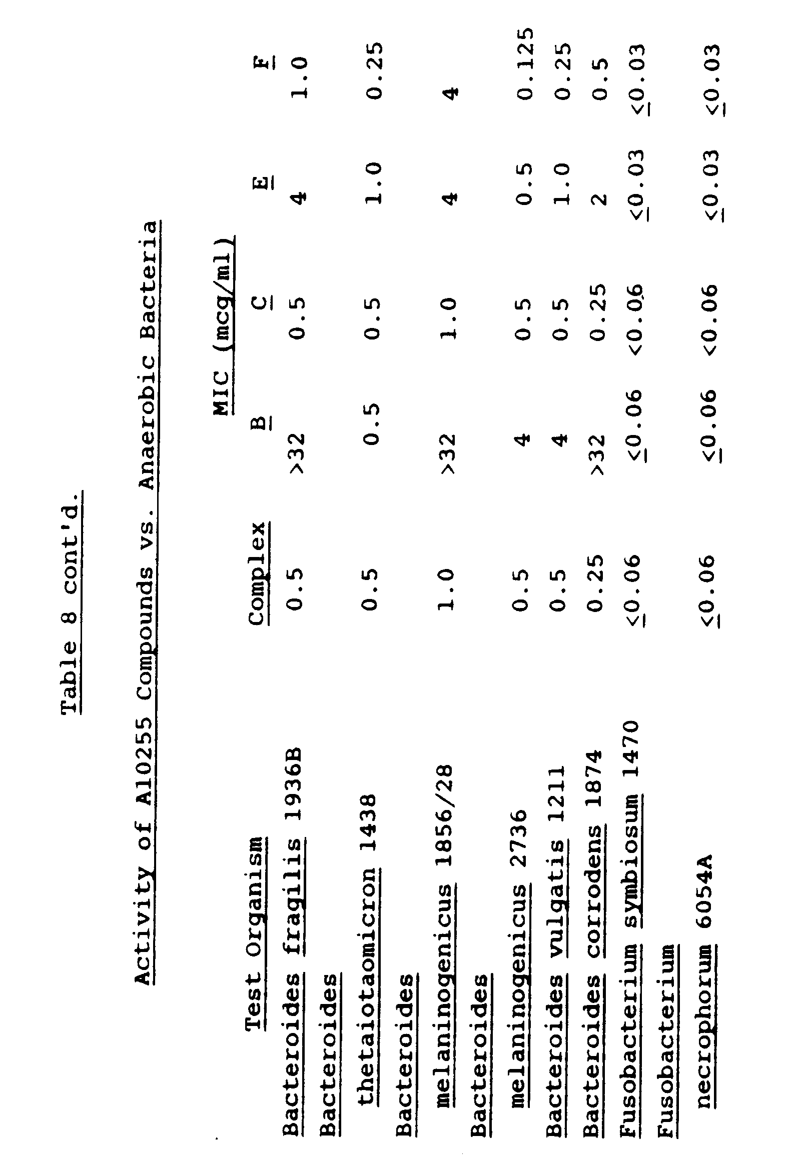

The A10255 complex and factor B demonstrated an LD₅₀ of greater than 300 mg/kg x 1 and greater than 75 mg/kg x 1, respectively. The results were obtained by intraperitoneal injection in mice.

The A10255 antibiotics are growth-promoting agents in economically important warm-blooded animals such as poultry, swine, and cows. In chickens, the A10255 complex improved weight gains and feed efficiency. Table 9 summarizes the results of two tests in which the complex was fed ad libitum to 7-day old chicks for fourteen days in battery testing (ten pens of seven chicks per treatment). The growth-performance data from the chicks treated with the complex was compared to that of an equal number of replicates of a contemporary control treatment.

The growth-enhancing effect in poultry is illustrated by the following test data obtained with chickens.

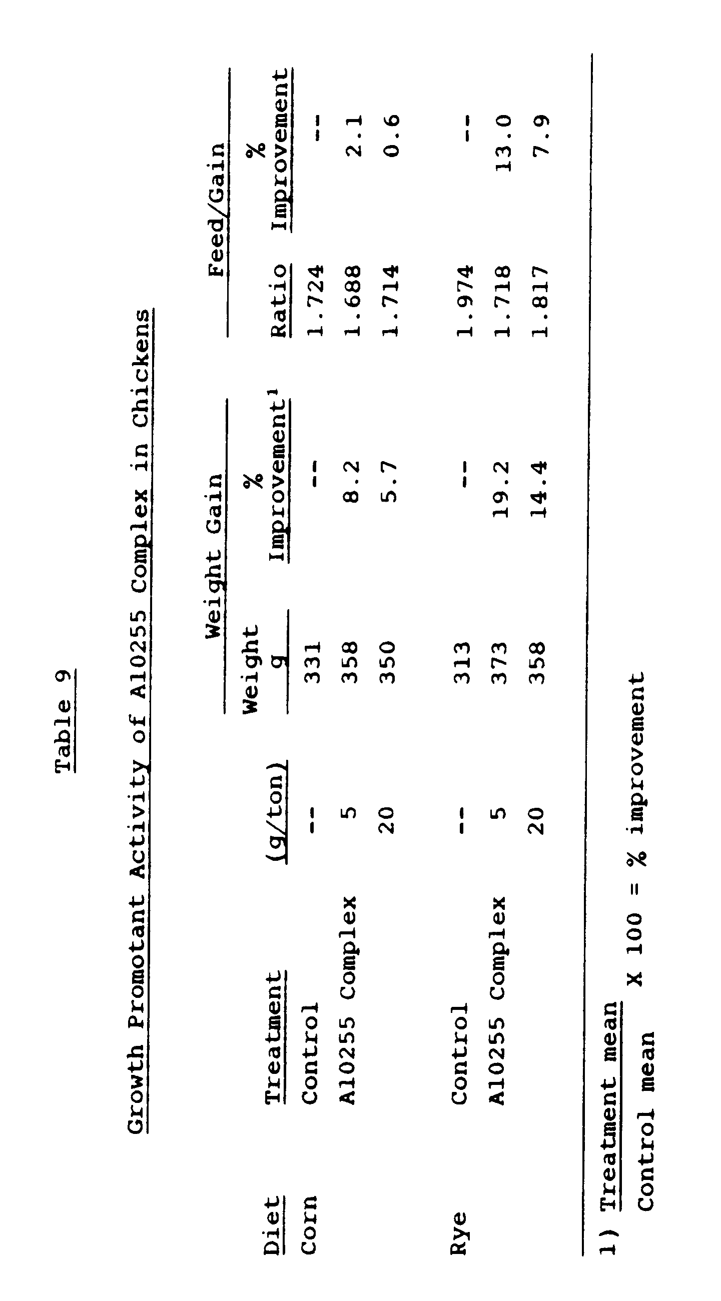

In order to promote the growth of chickens, the A10255 complex or the individual factors thereof should be administered with the chicken's feed at rates of from about 0.5 to about 50 grams of A10255 complex or individual factor per ton of chicken feed. Most beneficial results are observed when the A10255 complex or individual factor is administered at rates of from about 5 to about 20 grams of A10255 complex or factor per ton of chicken feed.

A further aspect of this invention is a feed composition for increasing feed utilization efficiency and promoting growth in poultry. The feed composition comprises chicken feed and effective amount of the A10255 complex, A10255 factor B, A10255 factor C, A10255 factor E, A10255 factor F, A10255 factor G, or A10255 factor H. An "effective amount" of the complex or the individual factors is from about 0.5 to about 50 grams per ton, and preferably from about 5 to about 20 grams per ton, of chicken feed. The chicken feed used in the feen composition can be any standard ration for chickens.

Formulation of the feed compositions is accomplished by mixing procedures well known in the veterinary pharmaceutical art.

A preferred feed composition comprises an effective amount of the A10255 complex and chicken feed.

The A10255 complex exhibited growth promotant and enhanced feed efficiency activity in weanling pigs. Evidence of such activity is set forth below in Table 10 which summarizes the results of nine trials employing the complex. In these trials the pigs had an average beginning weight of 24 lbs and finished to study at an average weight of 53 lbs. The pigs were housed 4 to a pen in an environmentally controlled nursery facility and were fed ad libitum an 18% protein corn-soy diet. All studies spanned 35 days, used two to three experimental units per treatment, and an average of fourteen animals per treatment.

In Table 10, the figures in parenthesis indicate the change in percent from the control group. A negative percentage change in the "Feed/Gain" column indicates an improvement in the Feed/Gain (feed efficiency) of the pigs fed the A10255 complex.

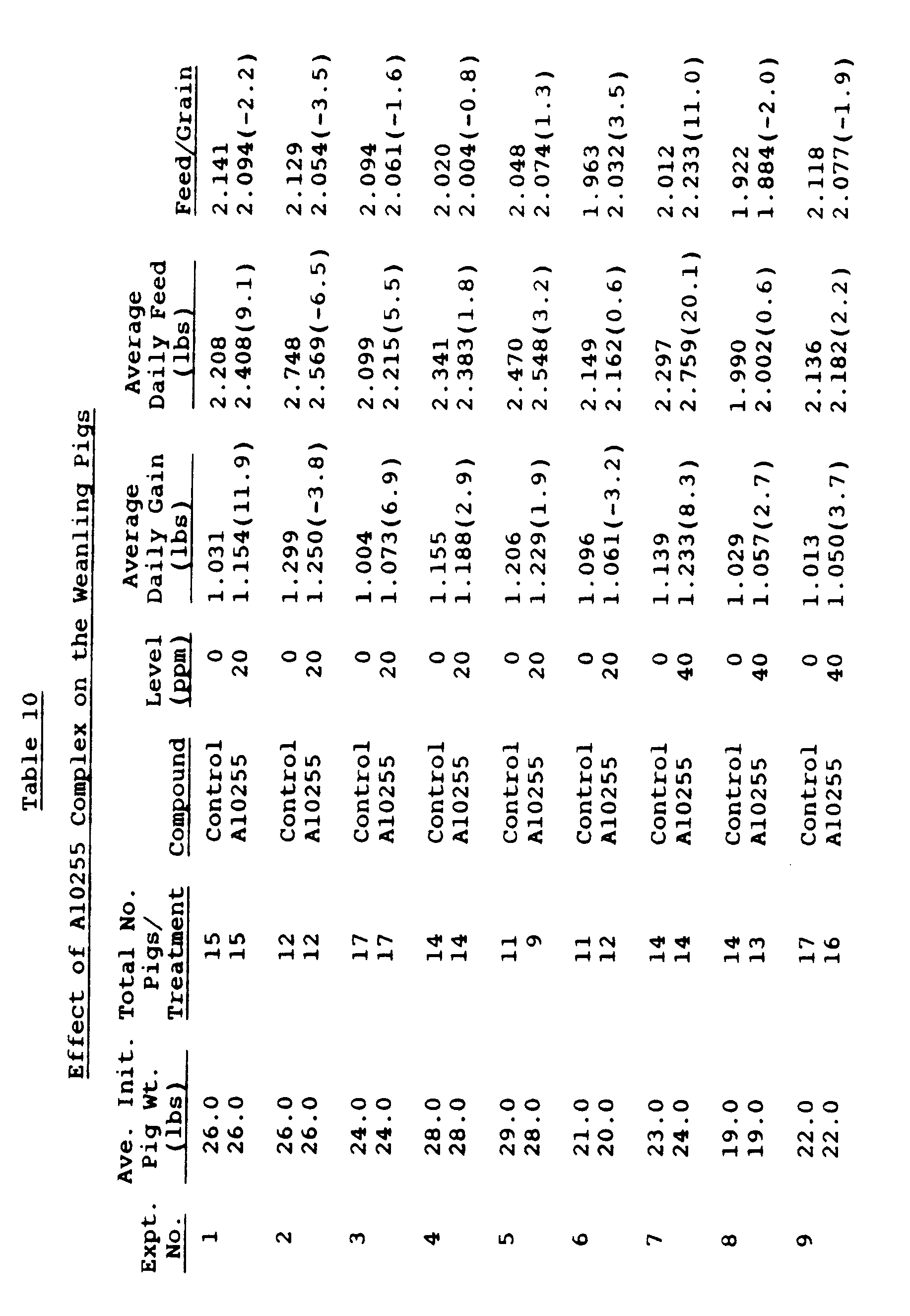

Another aspect of the invention is a feed composition for increasing feed utilization efficiency and promoting growth in weanling pigs. The feed comcomposition comprises swine feed and an effective amount of either the A10255 complex, A10255 factor B, A10255 factor C, A10255 factor E, A10255 factor F, A10255 factor G, or A10255 factor H.

The term "effective amount" in the feed composition for weanling pigs means approximately 20 to approximately 40 parts per million of the A10255 complex or individual factor per unit of whole feed. The feed used for the weanling pig composition is any of the normal feed rations used for weanling pigs. The feed composition may be formulated by any of the methods well known in the veterinary pharmaceutical art.

A preferred feed composition for weanling pigs comprises swine feed and an effective amount of the A10255 complex.

The A10255 complex also improves feed-utilization efficiency in ruminants which have a developed rumen function. The increase in efficiency caused by the A10255 antibiotics can be monitored by observing the production and concentration of propionate compounds in the rumen using the method described by James R. Beck and Joseph A. Yahner, U.S. Patent No. 4,333,923, issued June 8, 1982, herein incorporated by reference. Table 11 shows the ratio of volatile-fatty-acid (VFA) production in A10255 complex-treated flasks to production in control flasks in this test obtained from the method of Beck and Yahner.

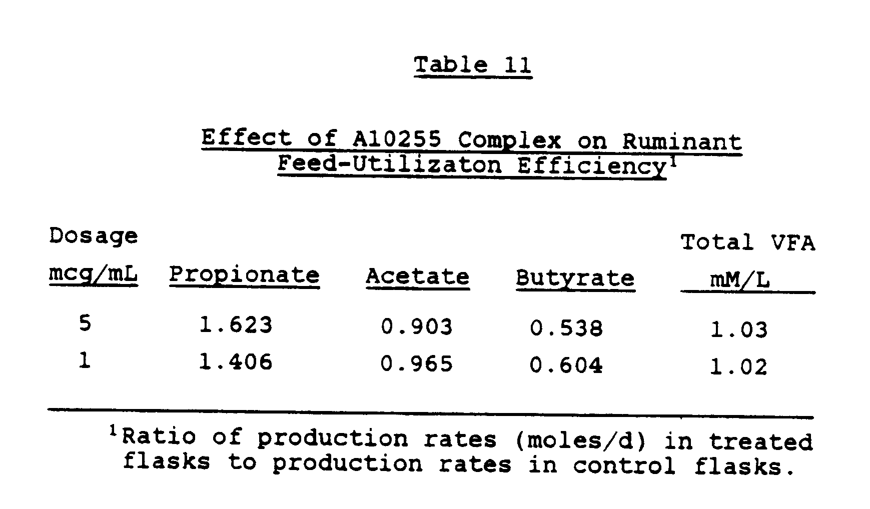

The A10255 compounds are typically effective in increasing propionate and, thereby, the efficiency of feed utilization when administered to ruminants orally.

The following Table 12 summarizes data demonstrating the effects of the A10255 complex on the growth and feed utilization of cattle. The study was conducted for a period of 79 days on three groups of animals.

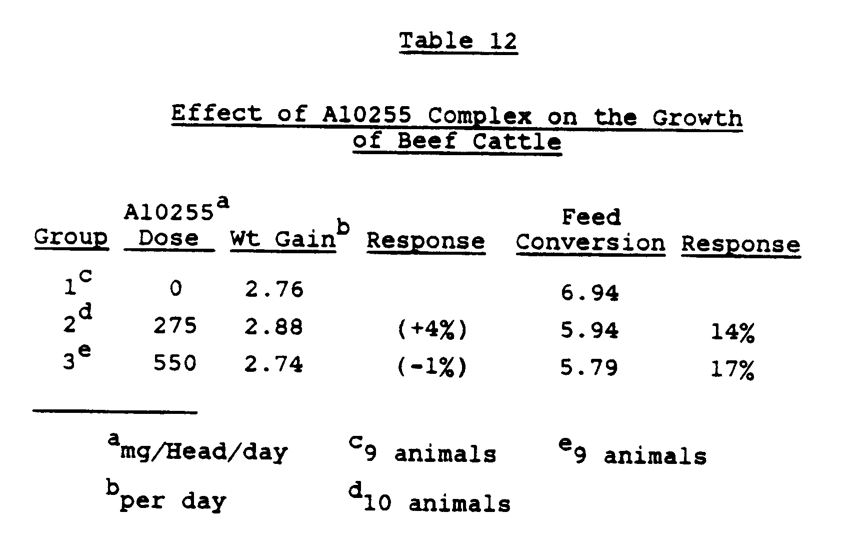

Another aspect of this invention is a method for increasing the feed utilization efficiency of cattle, which comprises administering to cattle an effective amount of A10255 complex, or an effective amount of an A10255 factor. A preferred method comprises administering to cattle an effective amount of the A10255 complex.

According to the method provided herein for promoting growth in cattle, the animals are administered orally an effective amount of the A10255 complex or a factor thereof. The term "effective amount" refers to from about 0.05 mg/kg/day to about 5.0 mg/kg/day. Preferably rates of oral administration are from about 0.1 mg/kg/day to about 3.0 mg/kg/day. The antibiotic complex or factor may be administered orally with the cattle's feed; however it can be administered in other ways, for example, drenches, boluses, or capsules. Mixing the A10255 compound with the cattle's feed is the preferred route of administration. Formulation of these various dosage forms is accomplished by methods well known in the veterinary pharmaceutical arts.

A further aspect of the invention is a feed composition for increasing the feed utilization of cattle. The feed composition comprises cattle feed and an effective amount of the A10255 complex or an A10255 factor. A preferred feed composition for cattle comprises cattle feed and an effective amount of the A10255 complex. The most highly preferred feed composition for cattle comprises the cattle feed and an effective amount of either A10255 factor B or A10255 factor G. The individual A10255 factors B, C, E, F, G and H appear to be roughly minimally bioequivalent.

In the above feed compositions for cattle, the term "effective amount" is as defined above for the method for enhancing feed utilization in cattle. The cattle feed used in conjunction with the feed composition can be any conventional feed ration for cattle.

In yet a further aspect of this invention, the A10255 complex and factors, and especially A10255 factor B, are useful in identifying Streptomyces species possessing a gene conveying thiostrepton resistance. Such a species may have naturally a thiostrepton-resistance gene or may have one after transformation with a plasmid or other cloning vector containing a thiostrepton-resistance gene. Thus, A10255B and the A10255 complex are interchangeable with the antibiotic thiostrepton to detect the thiostrepton resistance marker in recombinant DNA experiments with Streptomyces strains.

Specifically, protoplasts of the species S. fradiae, S. lividans, or S. griseofuscus are transformed with a plasmid (for example, pIJ702 or pHJL401) containing the thiostrepton-resistance conferring gene (C. J. Thompson et al., Nature, 286, p 525-527 (1980)). The protoplasts are inoculated onto a solid medium suitable for cell wall regeneration, and incubated overnight (approximately 16 hours) at 29°C. A10255 is added to a soft agar overlay which is then poured on the surface of the plate containing the regenerating protoplasts. The amount of the A10255 antibiotic(s) used in the overlay must be sufficient to yield a concentration of approximately 50 µg/ml after application to the regeneration medium. The plates are then incubated for from seven to fourteen days at 29°C to allow outgrowth of the subpopulation of cells containing the plasmid-borne thiostrepton-resistance conferring gene. Under these conditions, cells which have not received the plasmid do not grow. The A10255-resistant transformants are then transferred to medium containing 50 µg/ml of A10255 which ensures maintenance of the plasmid carrying the thiostrepton-resistance conferring gene.

The antimicrobial compounds (i.e., A10255 complex and individual factors) of this invention are useful for the therapeutic or prophylactic treatment of infections in warm-blooded animals caused by pathogenic bacteria. The antimicrobial compounds can be administered orally, parenterally (for example, intravenously, intramuscularly or subcutaneously) or as a topical ointment or solution in treating bacterial infections of warm-blooded animals.

A further aspect of this invention is the pharmaceutical compositions of the A10255 complex or the individual factors. These compositions are composed of a therapeutically active amount of the instant antibiotic compounds (i.e., the A10255 complex or factor B, factor C, factor E, factor F, factor G, or factor H, separately) and a suitable vehicle. With regard to compositions for oral administration (for example tablets and capsules), the term "suitable vehicle" means common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose, and starch; fillers and carriers, for example corn starch, gelatin, lactose, sucrose, microcrystalline cellulose, kaolin, mannitol, dicalcium phosphate, sodium chloride, and aliginic acid, disintegrators such as croscarmellose sodium, microcrystalline cellulose, corn starch, sodium starch glycolate, aliginic acid, and mutable wetting agents such as sodium lauryl sulfate; and lubricants such as magnesium stearate and other metallic stearates, stearic acid, silicone fluid, talc, waxes, oils, and colloidal silica. Flavoring agents such as peppermint, oil of wintergreen, cherry flavorings or the like can also be used. It may be desirable to add a coloring agent to make the dosage form more appealing visually or to help identify the product. The tablets may also be coated by methods well known in the art.

The pharmaceutical compositions of the present invention may also be in the form of oral liquid preparations, which may be either a) aqueous or oily suspensions, solutions, emulsions or syrups; or b) a dry powder to be reconstituted with water or another suitable vehicle before use. When used in conjunction with such oral liquid preparations, the term "suitable vehicle" means conventional additives such as suspending agents, for example, sorbitol, syrup, methyl cellulose, glucose/sugar syrup, gelatin, hydroxyethylcellulose, carboxymethyl cellulose, or aluminum stearate gel; or hydrogenated edible oils, for example, almond oil, fractionated coconut oil, oily esters, propylene glycol or ethyl alcohol; and preservatives such as methyl or propyl p-hyroxybenzoates or sorbic acid.

The pharmaceutical composition can also be for intravenous (IV) use. Specifically, a water soluble form of the antibiotic compound can be dissolved in one of the commonly used intravenous fluids and administered by infusion. When used in conjunction with compositions for IV use, the term "suitable vehicle" means such fluids as physiological saline, Ringer's solution or 5% dextrose solution.

For intramuscular preparations a sterile formulation of a suitable salt form of the antibiotic compound (for example, the hydrochloride salt or sodium salt) can be formulated with a "suitable vehicle". Examples of such sterile formulations are a suitable salt form either dissolved in a pharmaceutical diluent (for example, Water-for-Injection, physiological saline, 5% glucose) or suspended in an aqueous base or a pharmaceutically acceptable oil base (for example, an ester of a long chain fatty acid such as ethyl oleate).

Topcal compositions can be formulated with "suitable vehicles" such as hydrophobic or hydrophilic bases. Such bases include ointments, creams or lotions.

Veterinary pharmaceutical compositions of the antibiotic compounds may be administered in the feed or the drinking water of farm animals. Alternatively, the compounds can be formulated as intramammary preparations with "suitable vehicles" such as long- or quick-release bases.

The antibiotic compounds of the instant invention can also be formulated in unit dosage form in sterile vials, sterile plastic pouches containing a part with a septum, or sterile, hermetically sealed ampoules. The anitibiotic compound (or the corresponding pharmaceutically-acceptable salt) may be a dry powder or in crystalline or lyophilized form. the amount of the antibiotic compound per unit dosage may vary from about 250 milligrams to about 10 grams.

A "therapeutically effective amount" of the antibiotic compounds of the present invention is from approximately 3.5 mg to about 50 mg of compound per kilogram of body weight. This amount generally totals from about 1 gram to about 27 grams per day for an adult human.

A further aspect of this invention is a method for treating or controlling infectious diseases caused by gram-positive and gram-negative organisms in warm-blooded animals. This method comprises administering to the animal an therapeutically effective amount of the instant antibiotic compounds. A typical daily dose for an adult human in this method is from about 1 gram to about 12 grams.

In practicing this method, the antibiotic compound can be administered in a single daily dose or in multiple doses per day. The treatment regime may require administration over extended periods of time, for example, for several days or for from two to three weeks. The amount administered per dose or the total amount administered will depend on such factors as the nature and severity of the infection, the age and general health of the patient, and the tolerance to the antibiotic compound of both the patient and the microorganism or microorganisms involved in the infection.

As yet another aspect of the present invention, there is provided an animal premix comprising as an active ingredient, A10255 complex or factor B, C, E, F, G, or H in any combination, or a pharmaceutically-acceptable salt thereof, associated, with a suitable carrier therefor. One skilled in the art will, of course, appreciate which carriers may be selected.

As yet another aspect of the present invention, there is provided a process for producing A10255 complex which comprises
a) cultivating Streptomyces gardneri NRRL 15537, Streptomyces gardneri NRRL 18260, Streptomyces gardneri 15922, or an A10255-producing mutant thereof, in a culture medium containing assimilable sources of carbon, nitrogen, and inorganic salts under submerged aerobic fermentation conditions until the antibiotic A10255 complex is produced;
b) optionally separating the A10255 complex from the culture medium; and
c) optionally isolating one or more of antibiotic A10255 factors B, C, E, F, G, and H from the separated A10255 complex.

The following Examples are provided to further illustrate the invention. It is not intended that the invention be limited in scope by reason of any of the following Examples.

### Experimental Section

### Example 1

The following medium was prepared for use in the agar slant culture of the A10255-producing microorganism:

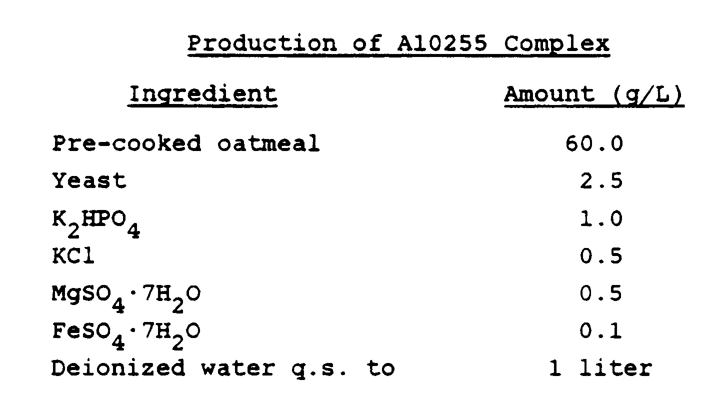

The pH of the resultant medium was adjusted to pH 7.3 with aqueous sodium hydroxide. The medium was then autoclaved, yielding a sterilized medium with a pH of 6.7.

Spores of S. gardneri, NRRL 15537, were inoculated on a nutrient agar slant composed of the above sterilized medium. The inoculated slant was incubated for 7-10 days at a temperature of 30°C. The mature slant culture was then covered with calf serum and scraped with a sterile tool to loosen the spores and mycelia. The resultant suspension was transferred to small tubes and lyophilized for preservation. One lyophilized pellet was used to inoculate sterile vegetative culture medium (50 ml, contained in a 250 ml wide-mouth Erlenmeyer Flask) of the following composition:

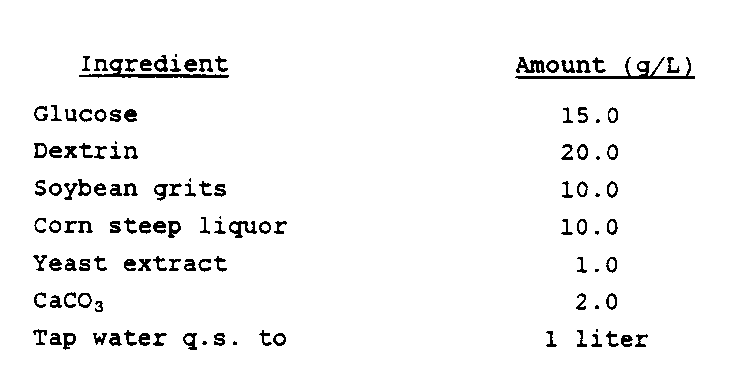

The pH of the medium was adjusted to 6.7 with aqueous sodium hydroxide. The medium was autoclaved, which raised the pH of the medium to between 6.8 and 7.0.

The inoculated vegetative medium was incubated for 48 hours at 30°C on a shaker rotating through an arc two inches in diameter at 250 RPM. The resulting vegetative medium culture was used either to inoculate small fermenters (the inoculum being approximately 1% per volume of fermenter medium), or to inoculate second stage flasks for the production of a larger volume of inoculum.

### Bump Medium

Two wide-mouth Erlenmeyer flasks (2 liter capacity) were charged with a medium (400 ml each) having the following composition:

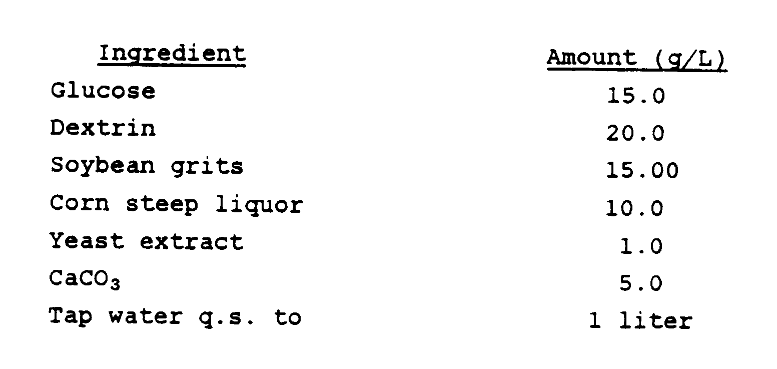

The medium in each Erlenmeyer flask was inoculated with the above vegetative culture. The volume of the inoculum totaled 2.5% of the volume of the medium in the Erlenmeyer flask. The inoculated medium was incubated at 30°C for 24 hours on a rotary shaker at 250 RPM on a 10° angleboard to yield a "bump" culture.

### Large Scale Fermentation

A portion of the above "bump" culture (800 ml) was used to inoculate the following medium (100 liters):

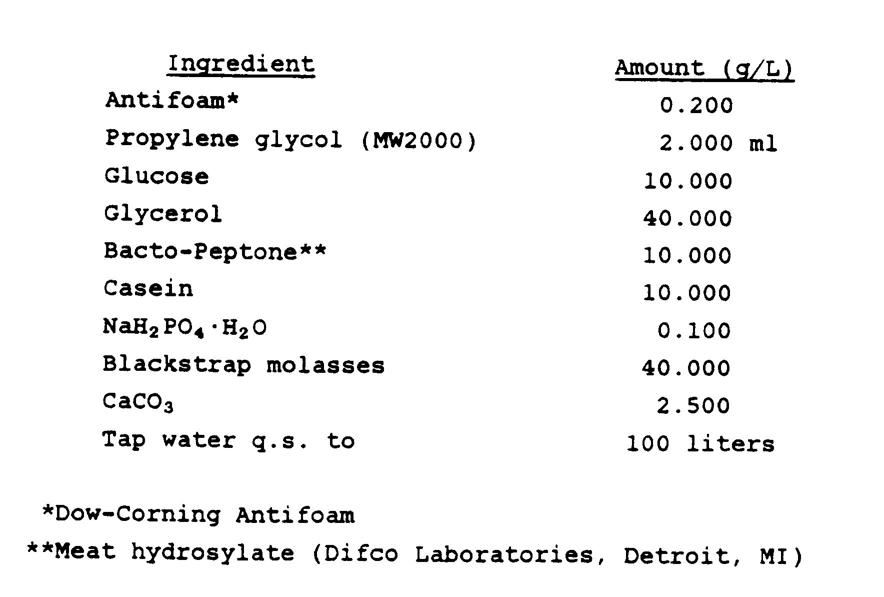

The medium was contained in a 165 liter fermenter. The pH of the medium was adjusted to 6.9 with 5N aqueous sodium hydroxide. The mixture was sterilized for 45 min. at 17-19 psi at 121°C. After the sterilization procedure the medium had pH 7.0. The sterilized medium was aerated with sterile air at the rate of 0.125 v/v/m, stirred with a conventional agitator at 200 RPM, and allowed to ferment for about 6 days at a temperature of 30°C.

### Example 2

### Isolation of the A10255 Antibiotic Complex

Whole fermentation broth (3780 L) was filtered using a filter aid (Hyflo Super-Cel, Johns-Manville Products Corp.). The press containing the mycelia was washed with 300 L of water, then the mycelia was extracted twice with acetone:water (4:1). The first extract was 900 L, the second 800 L. The extracts were combined and concentrated under reduced pressure to 450 L of an aqueous solution. The pH of the solution was adjusted to 3.0 with hydrochloric acid, then extracted three times with ethyl acetate (extract volumes were 165 L, 280 L, and 225 L respectively). The extracts and aqueous phase were analyzed for biological activity by a paper disc assay on agar plates seeded with B. subtilis ATCC 6633. The extracts were combined and concentrated to a volume of 23 L, under reduced pressure. The solids that had precipitated during concentration were collected by filtration and dried in vacuo to give 216 g of A10255 antibiotic complex (assay:90.7 g of activity). The filtrate was further concentrated to 900 ml and then filtered. The second precipitate was dried in vacuo to give 16.2 g of A10255 antibiotic complex (assay: 9.1 g activity).

### Example 3

### Separation of Factors from A10255 Antibiotic Complex

A10255 complex (20 g) was dissolved in chloroform:methanol (9:1, 400 ml total) and filtered. The filtrate was applied to a stainless steel column (8 x 100 cm) packed with 13-24 micron LPS-1 silica gel (4 l, Whatman Chemical Separation Inc., Clifton, New Jersey). The column was part of a Chromatospac Prep-100 unit (Jobin Yvon, 16-18 Rue du Canal 91160, Longjumeau, France). A series of 240 ml fractions were collected from the column, which was eluted at a 60 ml/min flow rate first with a 9:1 chloroform:methanol mixture (7 L), then with a 3:1 chloroform:methanol mixture (15 L). During the chromatography the HPLC detector was set at 280 nm. All fractions were padded on agar plates seeded with Micrococcus luteus ATCC 9341 to detect biological activity. The following fractions were pooled:

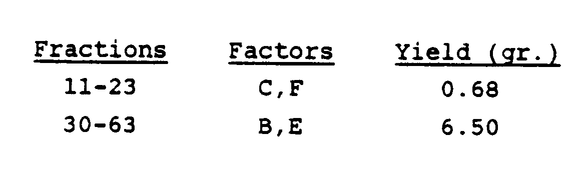

The pool containing factors B and E was concentrated to 400 ml. The resultant precipitate was collected by filtration dried in vacuo to yield 5.86 g of material consisting primarily of factor B with a small amount of factor E. (assay:3.6 g of activity). The supernatant from the precipitation was in turn concentrated. The concentrate was added to diethyl ether to give 0.64 g of a precipitate containing a similar mixture of factors B and E (assay:0.3 g of activity).

### Example 4

### Isolation of Antibiotic Factor A10255B

A 1.5 g portion of the material from Fractions 30-63 in the above Example 3 was dissolved in a mixture of tetrahydrofuran:water (35:65, 150 ml). The sample was applied to a stainless steel column (8 x 100 cm) packed with LP1-C18 reversed phase silica gel (4 l). (The silica gel was prepared by a procedure described in examples 6 and 7 of B. J. Abbott et al., U.S. Patent No. 4,299,763, issued November 10, 1981). The column was eluted at a flow rate of 60 ml/min. collecting 240 ml fractions with a tetrahydrofuran:water:disodium hydrogen phosphate mixture (3:7:0.05M, 12 l, pH 7.8 (phosphoric acid). The HPLC detector was set at 280 nm during the chromatography. Individual fractions were examined by analytical HPLC.

(The analytical HPLC was carried out on a 4.6 x 250 nm stainless steel column on a ultrasphere ODS 5 micron reversed phase material (altex Scientific Inc., Berkeley, CA). The column was eluted at a flow rate of 1 ml/min with a mixture of tetrahydrofuran:-water:sodium hydrogen phosphate (1:2:0.05M, at pH 7.8 due to the addition of phosphoric acid). The detector was set at 254 nm during the chromatography.)

The fractions were pooled as follows:

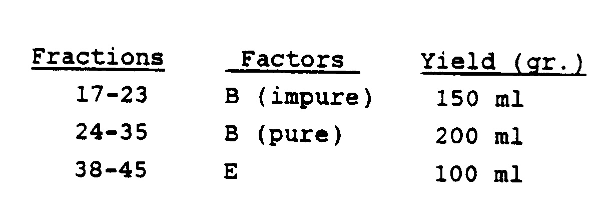

The concentrated pool containing fractions 24-35 was diluted to 300 ml with deionized water and the pH of the resultant mixture was adjusted to pH 3.0 with phosphoric acid. The solution was extracted with a 7:3 mixture of chloroform:methanol. The extract was concentrated to dryness and chromatographed by HPLC. HPLC was carried out on a Michel-Miller high performance low pressure liquid chromatography ("HPLPLC") glass colum (as described in K. Michel et al., U.S. Patent No. 4,131,547, issued December 26, 1978) containing a silica gel support (100-200 micron, Woelm Pharma). The column was eluted with a chloroform:methanol mixture (7:3). The HPLC monitor was set at 254 nm during the chromatography. The fractions containing the major UV chromophore were combined, concentrated in vacuo and lyophilized to give 659 mg Factor B.

### Example 5

### Purification of Antibiotic Factor A10255E

The pool of fractions 38-45 in Example 4 was combined with pools of similar composition from five additional chromatographies of the same manner as the one on the LP1-C₁₈ support in Example 4. The pH of the combined pools (800 ml) was adjusted to pH 3.0 with phosphoric acid. The combined pools were extracted with a 7:3 chloroform:methanol mixture (2X, 800 ml), then with an additional smaller amount (400 ml) of the solvent mixture. The extracts were combined, concentrated to dryness, and dissovled in 50 ml of a CHCl₃:MeOH mixture (7:3, 500 ml). The solution was applied to a 5.1 x 42 cm Michel-Miller HPLPLC glass column containing a silica gel support (500 ml, 100-200 micron, Woelm). The column was eluted with a mixture of 7:3 (v:v) chloroform:methanol and the major UV chromophore (254 nm) was collected and taken to dryness to yield 349 mg of pure factor E.

### Example 6

### Purification of Antibiotic Factors C and F

The dried preparation (0.68 g) obtained from the pool of fractions 11-23 in Example 3 was combined with preparations obtained in a similar manner to total 3.03 g of solid. This material was dissolved in a mixture of tetrahydrofuran:water (6:4) and the solution chromatographed on reversed phase silica gel (4 l) in a stainless steel column (8 x 100 cm) eluted at a flow rate of 60 ml/min with a mixture of 3:7 tetrahydrofuran:-water (39 l) monitored at 280 nm. The 480 ml fractions collected were padded on agar plates seeded with Micrococcus luteus ATCC 9341 to detect biological activity. Biologically active fractions were further analyzed by the analytical-scale HPLC system described in Example 4. The fractions were pooled as follows:

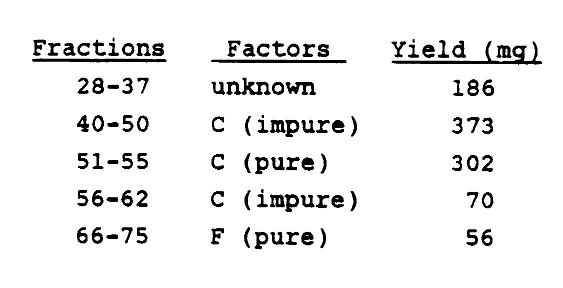

Each pool was concentrated and the resultant precipitate was collected on a filter and dried in vacuo.

### Example 7

The agar slant medium used in Example 1 was used to culture the A10255-producing microorganism NRRL 18260.

Accordingly, one lyophylized pellet from the above procedure was used to inoculate sterile vegetative culture medium (50 ml, contained in a 250 ml wide-mouth Erlenmeyer flask) of the following composition:

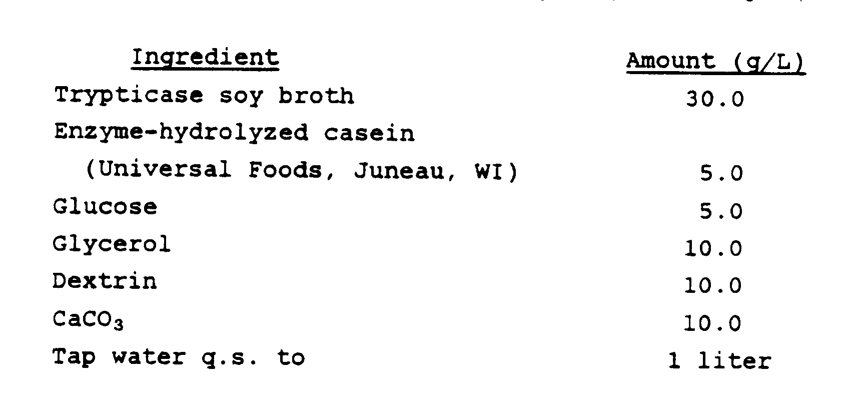

The pH of the medium was adjusted to 7.0 with aqueous sodium hydroxide before autoclaving.

The inoculated vegetative medium was incubated for 72 h at 30°C on a shaker rotating through an arc two inches in diameter at 250 RPM. The resulting vegetative medium culture was used either to inoculate small fermenters (the inoculum being approximately 1% per volume of fermenter medium), or to inoculate second stage flasks for the production of a larger volume of inoculum.

### Bump Medium

Two wide-mouth Erlenmeyer flasks (2 liter capacity) were charged with a medium (400 ml each) identical to the vegetative medium described above.

The medium in each Erlenmeyer flask was inoculated with the above vegetative culture. The volume of the inoculum totaled 2.5% of the volume of the medium in the Erlenmeyer flask. The inoculated medium was incubated at 30°C for 48 hours on a rotary shaker at 250 RPM on a 10° angle from horizontal to yield a "bump" culture.

### Large Scale Fermentation

A portion of the above "bump" culture (800 ml) was used to inoculate the following medium (100 liter):

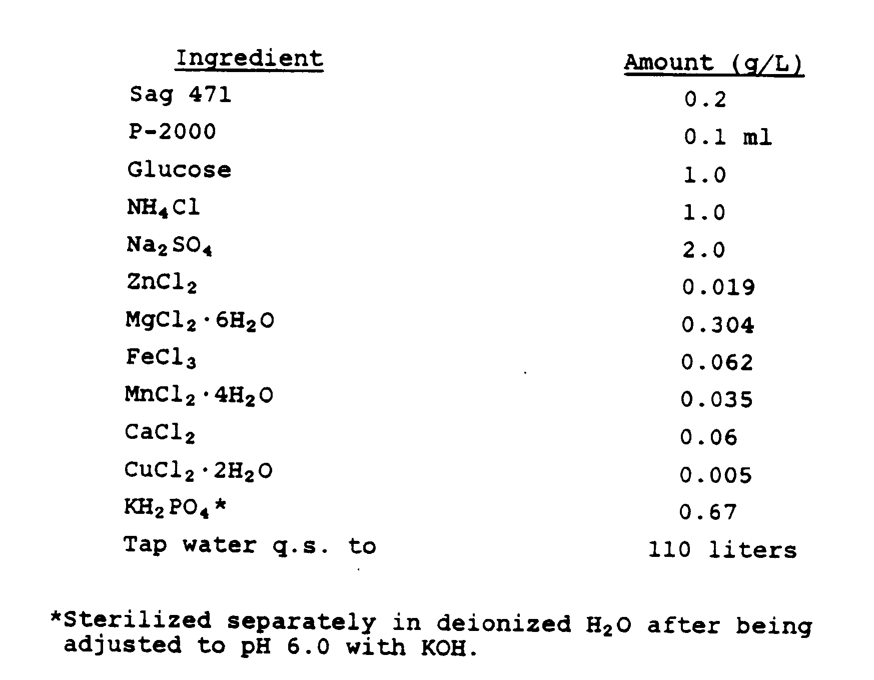

The medium was contained in a 165 liter fermenter. The mixture was steam sterilized by the application of 20 Fₒ heating units (where 1 Fₒ equals exactly one minute at 121°C). After the sterilization procedure the medium had pH 8.3. The pH after KH₂PO₄ addition was 7.0. The sterilized medium was aerated with sterile air and stirred with a conventional agitator to maintain the dissolved oxygen level at 45% of air saturation at 5 psi above atmospheric pressure, and allowed to ferment for about 7 days at a temperature of 30°C.

During the Large Scale Fermentation as outlined above, it was found to be efficacious to "feed" the fermentation mixture with glucose at the rate of 5.7 g/l/day. Casein hydrolysate was also fed after 17 hours at 4.5 g/l/day.

### Example 8

### Isolation of Antibiotic Factor A10255G

A 1.0 g portion of the antibiotic complex from Example 2 was dissolved into 2 l of H₂O:CN₃CN (4:1) at pH 6.0. This solution was applied to a Waters Prep 500 using a C18 cartridge column. The column was eluted using a shallow linear gradient of 4 l of (A) H₂O:CH₃CN:THF:HOAc (70:30:5:0.5) to 4 l of (B) H₂O:CH₃CN:THF:HOAc (65:35:5:0.5) at a flow rate of 250 ml per minute, collecting one fraction per minute. This was followed by 2 l of solvent (B). The column eluate was monitored at 280 nm. Seven additional runs were made using identical conditions. Pools containing a mixture of factors C and G were combined, the pH adjusted to 6.0 using 5N NaOH, diluted with an equal volume of water (to a total volume of 26 l) and chromatographed in two separate runs, using 13 l each time. These runs were also made on a C18 cartridge column on a Waters Prep 500. A linear gradient from 4 l of solvent (A) 0.05N NH₄OAc (pH 5.5):CH₃CN (75:25) to 4 l of solvent (B) 60:40 was used for the chromatography, followed by 2 l of solvent (B). The flow rate used was 250 ml per minute and one fraction was collected each minute. Fractions containing mainly factor G were combined, diluted using an equal volume of water, and rechromatographed on a C18 cartridge column using a Prep 500. The following linear gradient was used: (A) 4 l of H₂O:CH₃CN:THF:HOAc (70:30:5:0.5) to (B) 4 l of (65:35:5:0.5), at the same flow rate described above. Fractions containing factor G only were combined and concentrated in vacuo to an aqueous solution. The precipitated factor G was then separated by centrifugation, washed with water, suspended in water and lyophilized to give 686 mg of factor G.

### Example 9

### Isolation of Antibiotic Factor A10255H

A 1.0 g portion of the antibiotic complex from Example 2 was dissolved into 2 l of H₂O:CH₃CN (4:1) at pH 5.0 and filtered through glass wool on a filter funnel. The solution was applied to a C18 cartridge column on a Waters Prep 500. The column was eluted using a linear gradient from 4 l of (A) H₂O:CH₃CN:THF:HOAc (70:30:5:0.5) to 4 l of (B) 65:35:5:0.5 at a flow rate of 250 ml per minute, collecting one fraction every minute. This was followed by 2 l of solvent (B).

The column eluate was monitored at 280 nm. Thirteen additional runs were made using identical conditions. All pools containing factor H were combined (17 l), concentrated and dried in vacuo to give 690 mg of crude factor H. This material was combined with 329 mg of a similar preparation, dissolved into 3 l of H₂O:CH₃CN:THF (75:20:5) at pH 6.0 and applied to a C18 cartridge column as described above. A linear gradient from 4 l of solvent (A) 0.05 M NH₄OH:CH₃CN (75:25) to 4 l of solvent (B) 0.05 M NH₄OH:CH₃CN (60:40) was used for the chromatography. This was followed by 2 l of solvent (B). Flow rate and fraction volume were the same as described above. Fractions containing factor H were combined (750 ml), added to an equal volume of water and applied again to a C18 cartridge column as described above. The column was developed using a linear gradient from 4 l of (A) H₂O:CH₃CN:THF:HOAc (75:25:5:0.5) to 4 l of (B) H₂O:CH₃CN:THF:HOAc (60:40:5:0.5), collecting one 250 ml fraction every minute. The factor H was recovered by filtration, washed with water and dried in vacuo to give 285 mg of factor H.

### Example 10

### Feed Composition for Promoting Growth in Cattle

The A10255 complex or its individual factors can be administered orally to cattle by mixing an effective amount of the complex or factor with the feed. For example, one aspect of the invention is a standard feed ration of the composition:

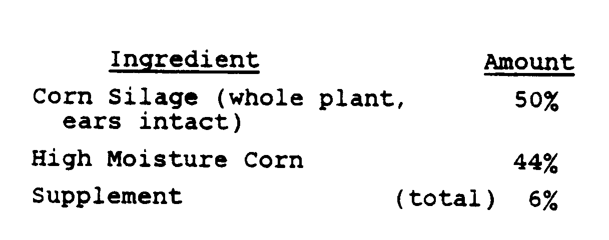

wherein the Supplement is composed of:

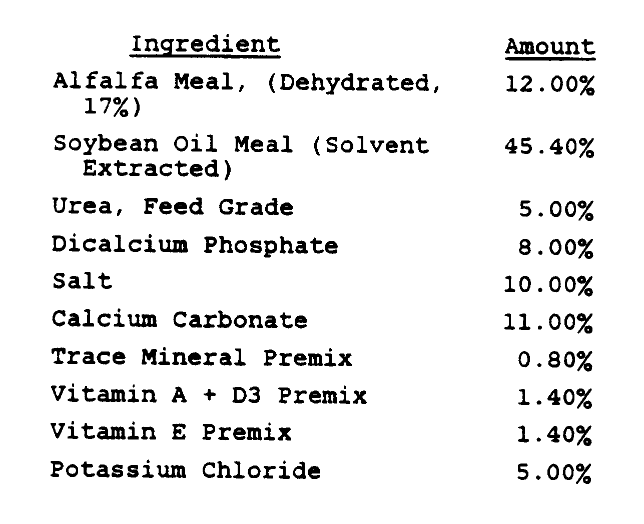

Prior to administration, the standard ration was mixed with an A10255 complex-containing premix. The premix was composed of the following ingredients:

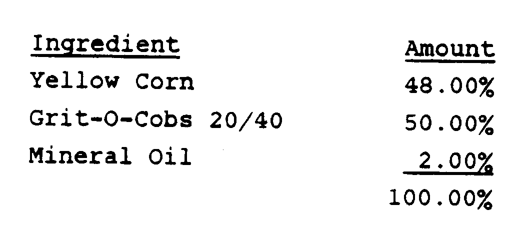

The premix was first mixed with the A10255 complex at an application rate of, for example, either 275 mg/head/day or 550 mg/head/day (which for this particular experiment, translated to 28.6 and 61.7 g/ton, respectively). The A10255 complex-containing premix is then top-dressed (and subsequently mixed) with the above standard feed ration at an application rate of 0.35 lb/head/day.

### Example 11

The following medium was prepared for use in the agar slant culture of the A10255.2 producing microorganism:

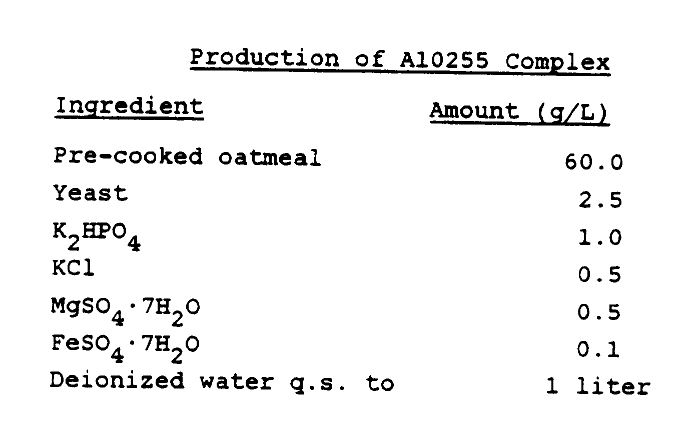

The pH of the resultant medium was adjusted to pH 7.3 with aqueous sodium hydroxide. The medium was then autoclaved, yielding a sterilized medium with a pH of 6.7.

Spores of S. gardneri, NRRL 15922, were inoculated on a nutrient agar slant composed of the above sterilized medium. The inoculated slant was incubated for 7-10 days at a temperature of 30°C. The mature slant culture was then covered with calf serum and scraped with a sterile tool to loosen the spores and mycelia. The resultant suspension was transferred to small tubes and lyophilized for preservation. One lyophilized pellet was used to inoculate sterile vegetative culture medium (50 ml, contained in a 250 ml wide-mouth Erlenmeyer flask) of the following composition:

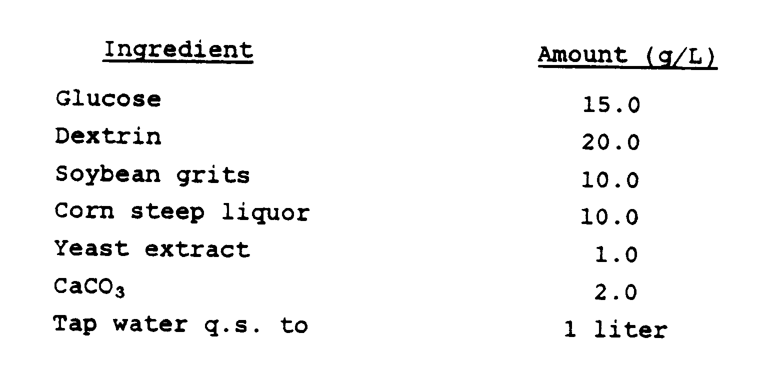

The pH of the medium was adjusted to 6.7 with aqueous sodium hydroxide. The medium was autoclaved, which raised the pH of the medium to between 6.8 and 7.0.

The inoculated vegetative medium was incubated for 48 hours at 30°C on a shaker rotating through an arc two inches in diameter at 250 RPM. The resulting vegetative medium culture was used either to inoculate small fermenters (the inoculum being approximately 1% per volume of fermenter medium), or to inoculate second stage flasks for the production of a larger volume of inoculum.

### Bump Medium

Two wide-mouth Erlenmeyer flasks (2 liter capacity) were charged with a medium (400 ml each) having the following composition:

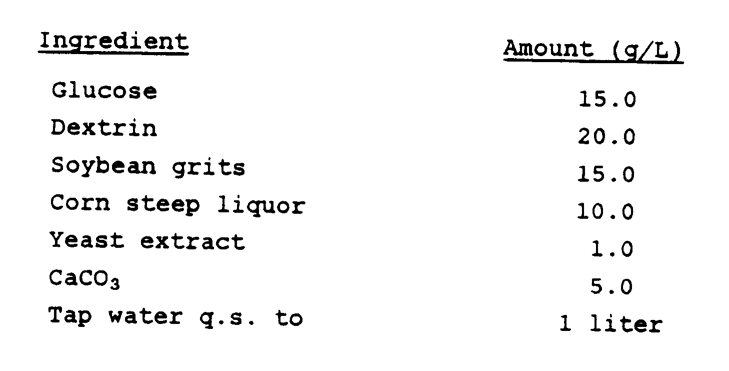

The medium in each Erlenmeyer flask was inoculated with 2.5% of its volume of the above vegetative culture. The inoculated medium was incubated at 30°C for 23 hours on a rotary shaker at 250 RPM to yield a "bump" culture.

### Large Scale Fermentation

A portion of the above "bump" culture (800 ml) was used to inoculate the following medium (115 liters):

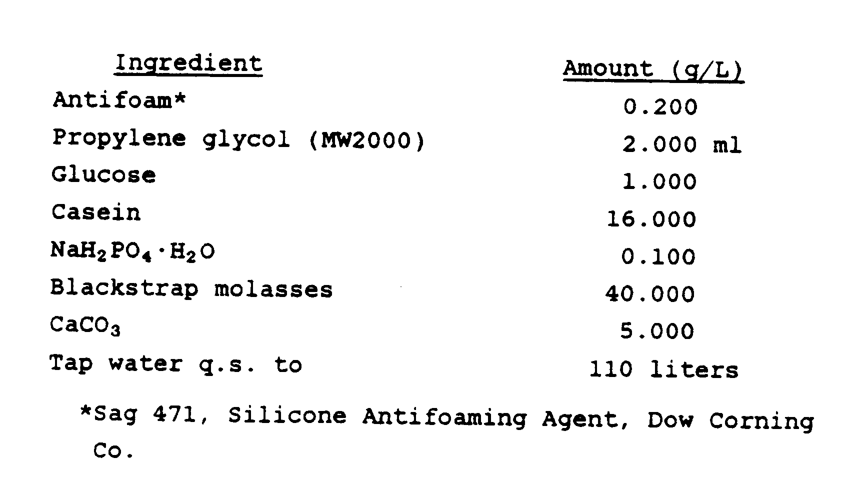

The medium was contained in a 165 liter fermenter. The pH of the medium was adjusted to 6.9 with 5N aqueous sodium hydroxide. The mixture was sterilized for 45 min. at 17-19 psi at 121°C. After the sterilization procedure the medium had pH 6.8. The sterilized medium was aerated with sterile air at the rate of 0.5 v/v/m, stirred with conventional agitators at an initial rate of 300 RPM, and allowed to ferment for about 7 days at a temperature of 30°C. During the fermentation period, the pH was maintained at 7.0, dissolved oxygen level was maintained at 45% of air saturation and glucose was fed to the medium at a constant rate of 3.5-4.0 g/l/day. After 24 hours, Hy Case Amino (acid hydrosylate of casein, Humko Sheffield Chemical Co., Hyndhurst, NJ was also fed into the medium at a constant rate of 3 g/l/day.

The crude A10255 complex was isolated in a procedure similar to that of Example 13 below.

### Example 12

### Large Volume Fermentation of the A10255.2 Straion

### Vegetative Inoculum

As a starting point for large volume fermentations of the A10255.2 strain (i.e., larger volumes than Example 1 above), vegetative inoculum is prepared in two stages. Both stages use the following medium:

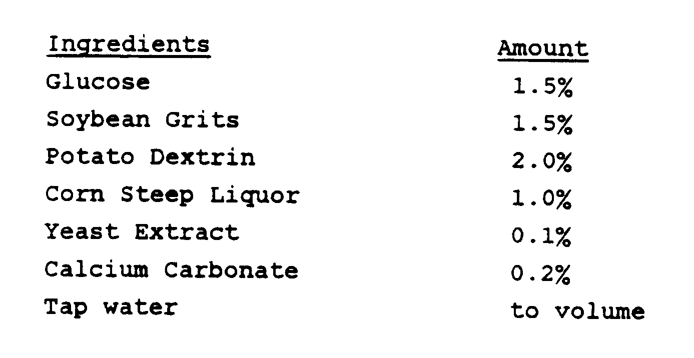

The first stage of incubation was carried out in a 250 ml flask containing 80 ml of the sterilized medium. The flask was inoculated with a lyophilized pellet of the A10255.2 culture. The medium was incubated at 30°C for 48 hours on a rotary shaker at 250 rpm.

A portion (10 ml) of the first stage inoculum was used to inoculate a sterilized second stage incubation medium, composed of 400 ml of the above medium contained in a two liter wide-mouth Erlenmeyer flask. This second stage medium was incubated at 30°C for 24 hours on a rotary shaker at 250 rpm. The incubated medium was used below in the tank fermentation.

### Tank Fermentation

The medium from the above Second Stage Vegetative Inoculum was used to inoculate the following tank medium contained in a 150 liter fermenter:

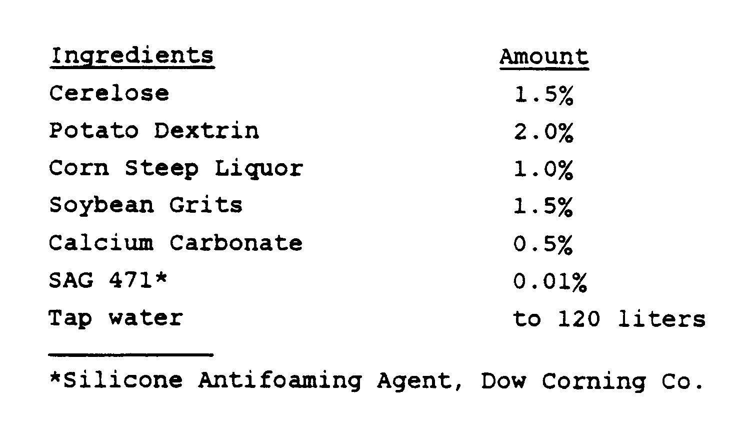

This medium was first sterilized by autoclaving. After sterilization, the pH of the medium was adjusted to 6.5 by the addition of 5N sodium hydroxide solution. The medium was then inoculated and fermented at 30°C for about 24 hours. During fermentation the medium was aerated with sterile air at an initial rate of 1 cubic foot per minute (cfm) and agitated by a conventional stirrer at an initial rate of 350 rpm.

### Large Scale Fermentation

A portion of the above tank medium (40 liters) was used to inoculate the following large-scale medium:

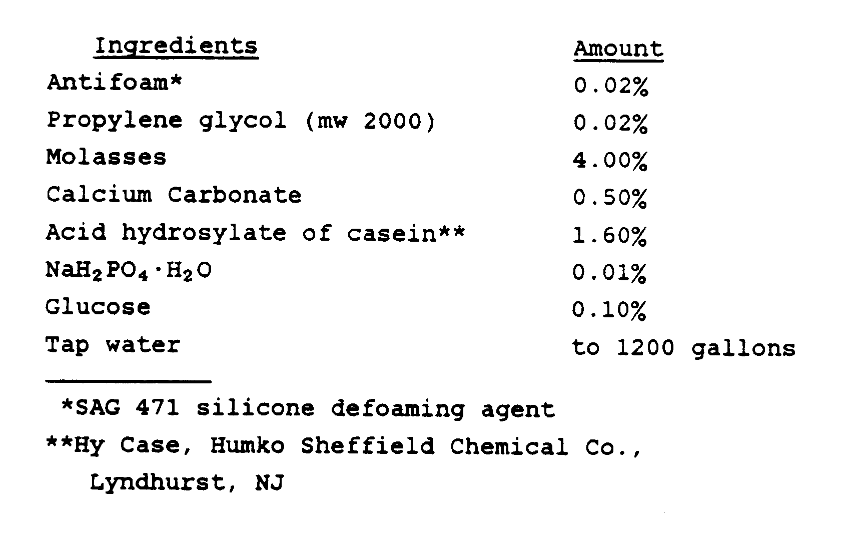

The medium was contained in a 1600 gallon fermenter. The pH of the medium was adjusted to 7.0 by the addition of 5N sodium hydroxide solution. The medium was sterilized for thirty minutes at 121°C at 17 psi. The sterilized medium was aerated with sterile air at an initial rate of 20 cubic feet per minute, stirred with a conventional agitator at an initial speed of 100 rpm, and allowed to ferment for about 6 days at 30°C. During the fermentation period, the pH of the medium was maintained at about 7.0, the dissolved oxygen content of the medium was maintained at about 45% of air saturation, and glucose was fed to the medium at a constant rate of 4 g/l/day. After 24 hours, Hy Case was also fed to the medium at the rate of 3 g/l/day.

### Example 13

### Isolation of the A10255 Antibiotic Complex

Whole fermentation broth (5000 l) of the A10255.2 organism was harvested and filtered using filter aid (Hyflo Super-Cel, Johns Manville Products Corp.). The filtrate and water wash (1200 l of water) of the biomass were discarded.

The biomass was then extracted batchwise with a mixture of 1:4 water:acetone (2000 l). The extraction was repeated and the extracts were combined (total: 4000 liters). The combined extracts contained the bulk of the A10255 complex.

The combined extracts were concentrated under vacuum to an aqueous suspension. Upon standing, a fine solid precipitated from the suspension. The solid was the crude A10255 complex. The concentrated extracts were then centrifuged and the supernatant discarded.

The solids obtained from the centrifugation were dried in vacuo to give 1.5 Kg of a brown powder containing 423 microgram/milligram of A10255 antibiotic acitivity.

## Claims (Claims for the following Contracting State(s): AT, BE, CH, DE, FR, GB, IT, LI, LU, NL, SE)

1. The antibiotic which can be produced by cultivating Streptomyces gardneri NRRL 15537, Streptomyces gardneri NRRL 18260, and Streptomyces gardneri NRRL 15922 in a culture medium containing assimilable sources of carbohydrate, nitrogen, and inorganic salts under submerged aerobic fermentation conditions.

2. Antibiotic A10255 factor B, which is a non-crystalline white to light-yellow powder having the following characteristics:
(a) An approximate averaged elemental analysis of 49.25 percent carbon; 3.94 percent hydrogen, 15.65 percent nitrogen, 21.63 percent oxygen, and 6.73 percent sulfur.
(b) An infrared absorption spectrum in a potassium bromide pellet with significant absorption maxima at the following frequencies (in cm⁻¹): 3373, 2969, 2932, 2875, 1661, 1598, 1520, 1494, 1395, 1250, 1114, 1084, 996, 932, and 900;
(c) Titratable groups measured in 66% aqueous dimethylformamide with pKa values at approximately 4.9, 11.2, and 12.8;
(d) A molecular weight of about 1244 daltons as determined by fast atom bombardment mass spectroscopy;
(e) Is soluble in dimethylsulfoxide, dimethylformamide, pyridine, chloroform/methanol mixtures, and an approximately 4:1 (v:v) mixture of tetrahydrofuran:-water;
(f) Contains approximately 629 millimoles/milligram of threonine and approximately 5,268 millimole of ammonia equivalent per milligram, as determined by hydrolysis with 6N hydrochloric acid;
(g) An ultraviolet absorption spectrum in acidic, basic and neutral methanol exhibiting an absorption maximum at 245 nm (ε = 66,000);
(h) A proton nuclear magnetic resonance spectrum in perdeuterated dimethylsulfoxide at 500 MHz and had the following signals: 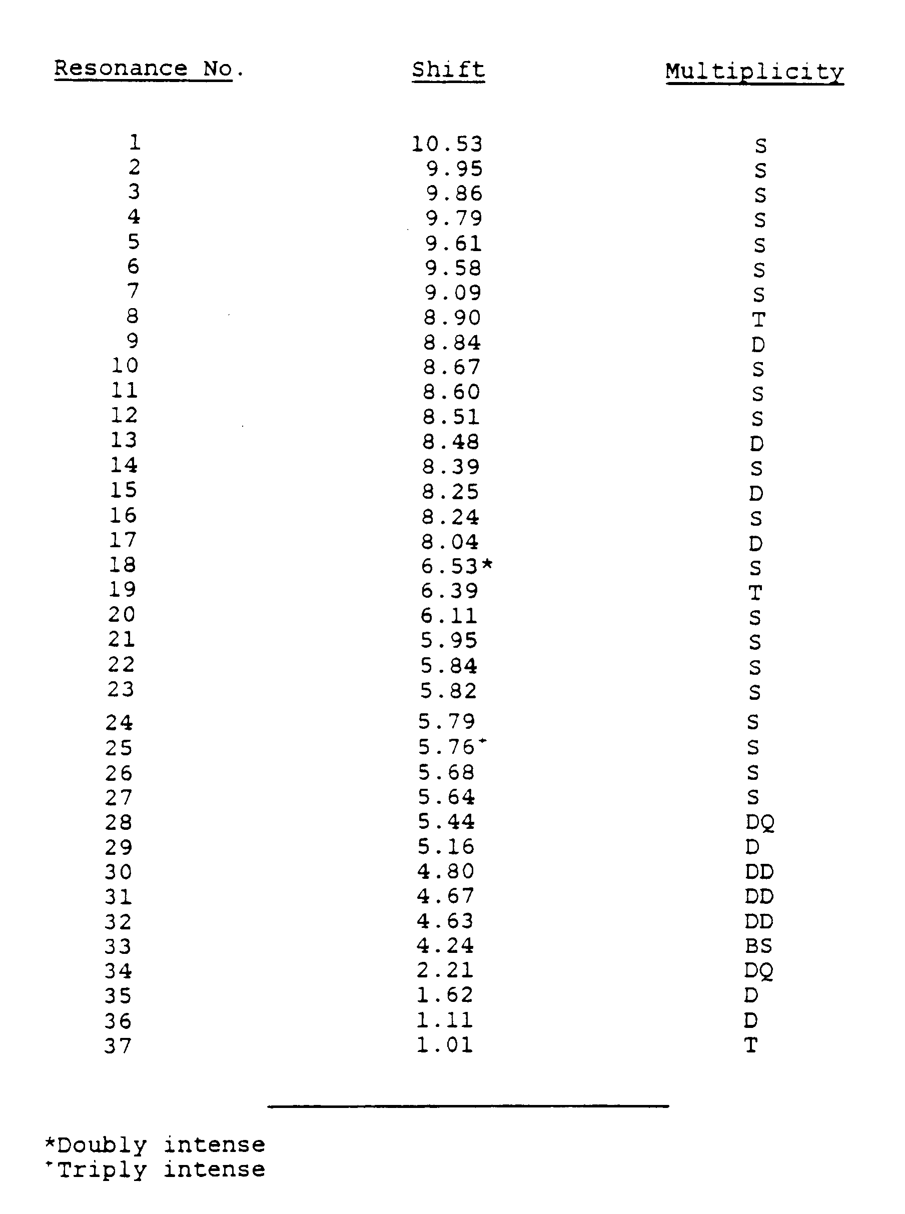
(i) A ¹³C nuclear magnetic resonance spectrum in perdeuterated dimethylsulfoxide at 125 MHz with the following signals: 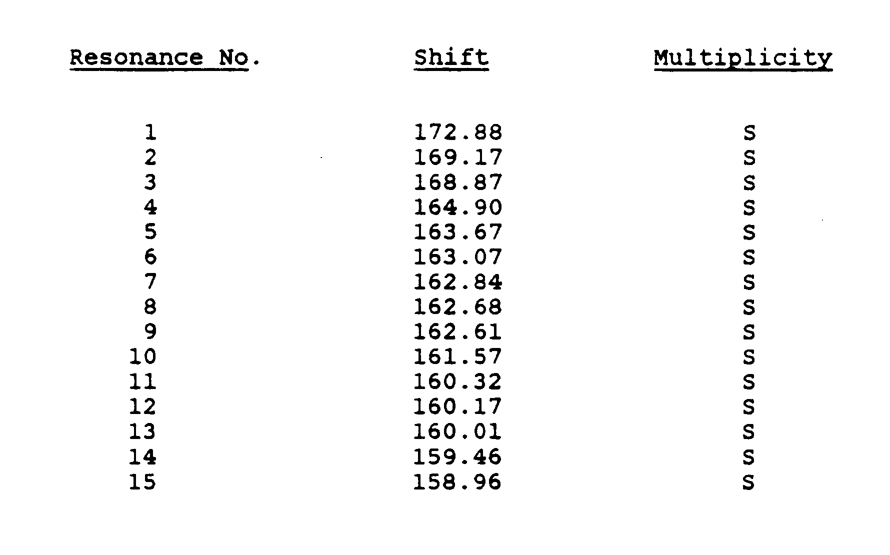 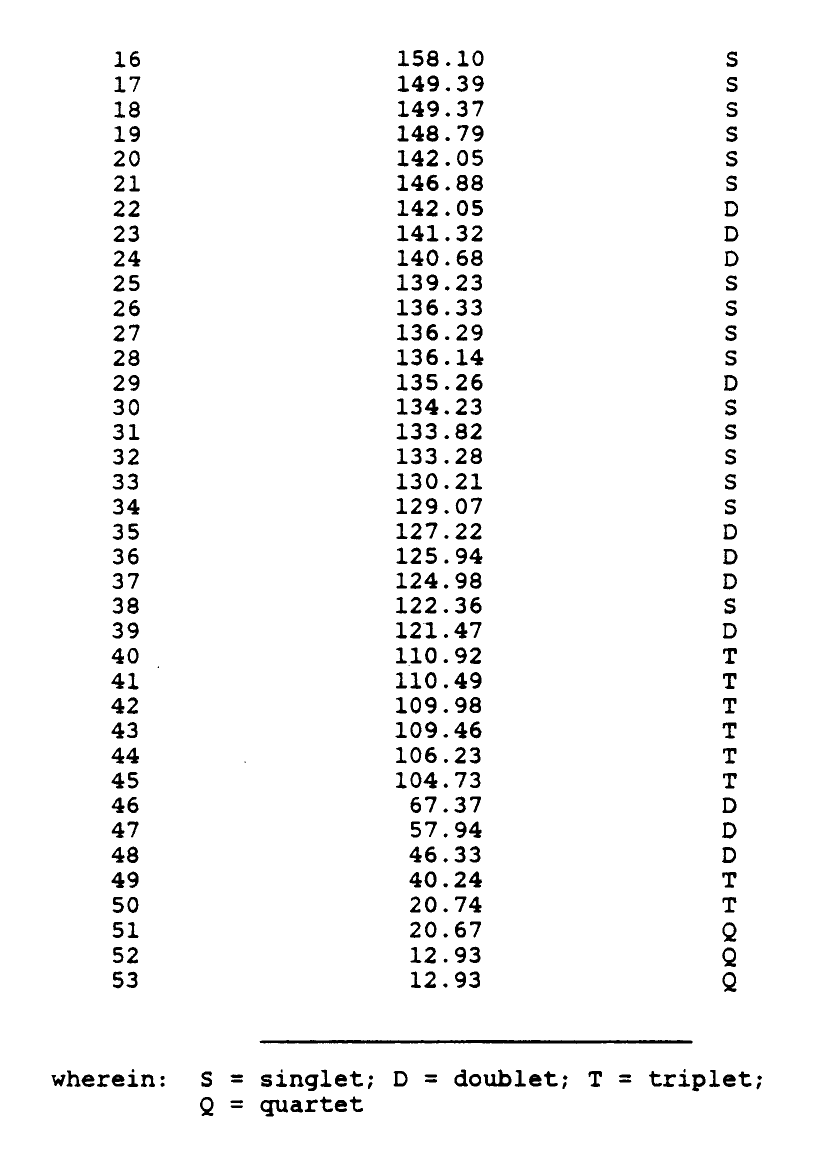 or a pharmaceutically-acceptable salt thereof;

3. Antibiotic A10255 factor C, which is a non-crystalline white to light-yellow powder possessing the following physical characteristics:
(a) An approximately, averaged elemental analysis of 49.18 percent carbon, 3.86 percent hydrogen, 17.89 percent nitrogen, 18.28 percent oxygen and 6.46 percent sulfur;
(b) A proton nuclear magnetic resonance spectrum in perdeutero dimethylsulfoxide at 360 MHz with signals at the following δ values: 10.51, 10.08, 9.84, 9.57, 9.10, 8.88, 8.03, 7.94, 7.53, 5.15 (exchangeables) 8.67, 8.59, 8.51, 8.49, 8.38, 8.25, 8.24, 6.57, 6.52, 6.38, 6.11, 5.91, 5.78, 5.74, 5.70, 5.65, 5.63, 5.44, 5.15, 4.79, 4.67, 4.63, 4.23, 2.21, 1.62, 1.11, 1.01;
(c) An infrared absorption spectrum in potassium bromide with absorption maxima at 3375, 2973, 2932, 2876, 1661, 1597, 1494, 1427, 1345, 1305, 1249, 1111, 1083, 981, 933, and 894 cm⁻¹;
(d) An ultraviolet absorption spectrum with an absorption maximum at 245 nm (ε = 63,000) in neutral, acidic, and basic methanol;
(e) A molecular weight of approximately 1174 daltons, as determined by fast atom bombardment mass spectral analysis;
(f) Titratable groups measured in 66% aqueous dimethylformamide with pKa values of approximately 2.9 and 12.
(g) Contains approximately 758 millimoles/milligram of threonine and approximately 7,429 millimoles/milligram of ammonia equivalents, as determined by standard hydrolysis procedures with 6N hydrochloric acid;
(h) Is soluble in dimethylsulfoxide, dimethylformamide, pyridine, 1:1 (v:v) chloroform:methanol, and 4:1 (v:v) of tetrahydrofuran:water;
or a pharmaceutically acceptable salt thereof;

4. Antibiotic A190255 factor E, which is a non-crystalline white to light-yellow powder having the following characteristics:
(a) An approximate averaged elemental analysis of 48.03 percent carbon; 3.91 percent hydrogen, 15.76 percent nitrogen, 17.09 percent oxygen and 5.63 percent sulfur;
(b) An infrared absorption spectrum in a potassium bromide pellet with significant absorption maxima at the following frequencies (in cm⁻¹): 3367, 3361, 2966, 1664, 1501, 1389, 1254, 1102, and 889;
(c) Titratable groups measured in 66% aqueous dimethylformamide with pKa values at approximately 4.85, 11.1, and 13.2;
(d) A molecular weight of about 1258 daltons as determined by fast atom bombardment mass spectroscopy;
(e) Is soluble in dimethylsulfoxide, dimethylformamide, pyridine, and an approximately 4:1 (v:v) mixture of tetrahydrofuran:water;
(f) Contains approximately 716 millimoles/milligram of threonine and approximately 8,580 millimoles of ammonia equivalent per milligram, as determined by hydrolysis with 6N hydrochloric acid;
(g) An ultraviolet absorption spectrum in acidic, basic and neutral methanol exhibiting an absorption maximum at 245 nm (ε = 77,000);
(h) A proton nuclear magnetic resonance spectrum in perdeuterated dimethylsulfoxide at 270 MHz exhibiting signals at δ 10.54, 10.00, 9.94, 9.81, 9.60, 9.56, 9.45, 8.89, 8.84, 8.66, 8.59, 8.50, 8.47, 8.39, 8.25, 8.22, 8.10, 6.53, 6.50, 6.24, 5.95, 5.86, 5.84, 5.77, 5.64, 5.55, 5.52, 5.44, 5.10, 4.80, 4.66, 4.64, 4.22, 2.78, 1.60, 1.11 and 1.00;
or a pharmaceutically-acceptable salt thereof;

5. Antibiotic A10255 factor F, which is a non-crystalline white to light-yellow powder posessing the following physical characteristics:
(a) An approximately, averaged elemental analysis of 49.65 percent carbon, 4.23 percent hydrogen, 17.11 percent nitrogen, 22.08 percent oxygen, and 7.78 percent sulfur;
(b) A proton nuclear magnetic resonance spectrum in perdeuterated dimethylsulfoxide at 360 MHz with signal at δ 10.51, 10.17, 9.88, 9.77, 9.54, 9.10, 8.90, 8.88, 8.66, 8.59, 8.51, 8.49, 8.38, 8.25, 8.24, 8.06, 7.94, 7.53, 6.56, 6.51, 6.27, 6.23, 6.12, 6.12, 5.96, 5.77, 5.76, 5.71, 5.71, 5.64, 5.62, 5.47, 5.14, 4.77, 4.65, 4.62, 4.20, 2.77, 2.48, 2.48, 1.58, 1.08, 0.98, and 0.98;
(c) An infrared absorption spectrum in potassium bromide with absorption maxima at 3369, 2943, 2907, 2846, 1663, 1588, 1519, 1493, 1425, 1337, 1288, 1251, 1151, 1110, 1083, 995, 927, 890, 807, 776, and 751 cm⁻¹;
(d) An ultraviolet absorption spectrum with an absorption maximum at 245 nm (ε = 71,500) in neutral, acidic, and basic methanol;
(e) A molecular weight of approximately 1188 daltons, as determined by fast atom bombardment mass spectroscopy;
(f) A titratable group measured in 66% aqueous dimethylformamide at a pKa of 12.5.
(g) Contains approximately 735 millimoles/milligram of threonine and approximately 7,226 millimoles/milligram of ammonia equivalents, as determined by standard hydrolysis procedures with 6N hydrochloric acid;
(h) Is soluble in dimethylsulfoxide, dimethylformamide, pyridine, 1:1 (v:v) chloroform:methanol, and 4:1 (v:v) of tetrahydrofuran:water;
or a pharmaceutically acceptable salt thereof;

6. Antibiotic A190255 factor G, which is a non-crystalline white to light-yellow powder which is soluble in dimethylsulfoxide, dimethylformamide, pyridine, chloroform/methanol mixtures, and 4:1 (v:v) tetrahydrofuran:water, having the following physical characteristics:
a) an ultraviolet spectrum in neutral ethanol which shows a λₘₐₓ = 247 nm (ε = 72,200); acidic solution, λₘₐₓ = 247 nm (ε = 73,400); and basic solution, λₘₐₓ 211 nm (ε = 27,200);
b) a ¹H nuclear magnetic resonance spectrum at 500 MHz in perdeuterated dimethylsulfoxide having the following signals: 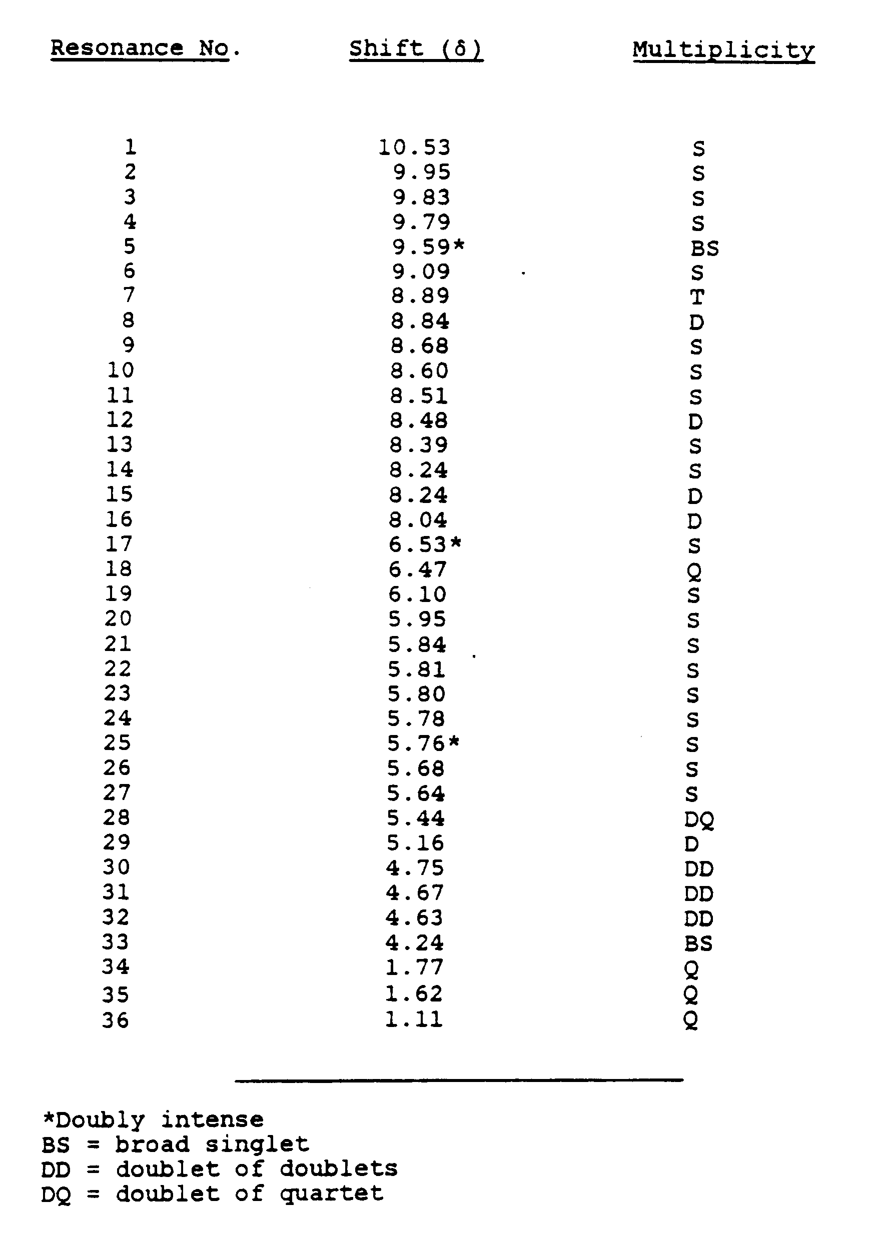
c) a ¹³C nuclear magnetic resonance spectrum at 500 MHz in perdeuterated dimethylsulfoxide having the following adsorption maxima: 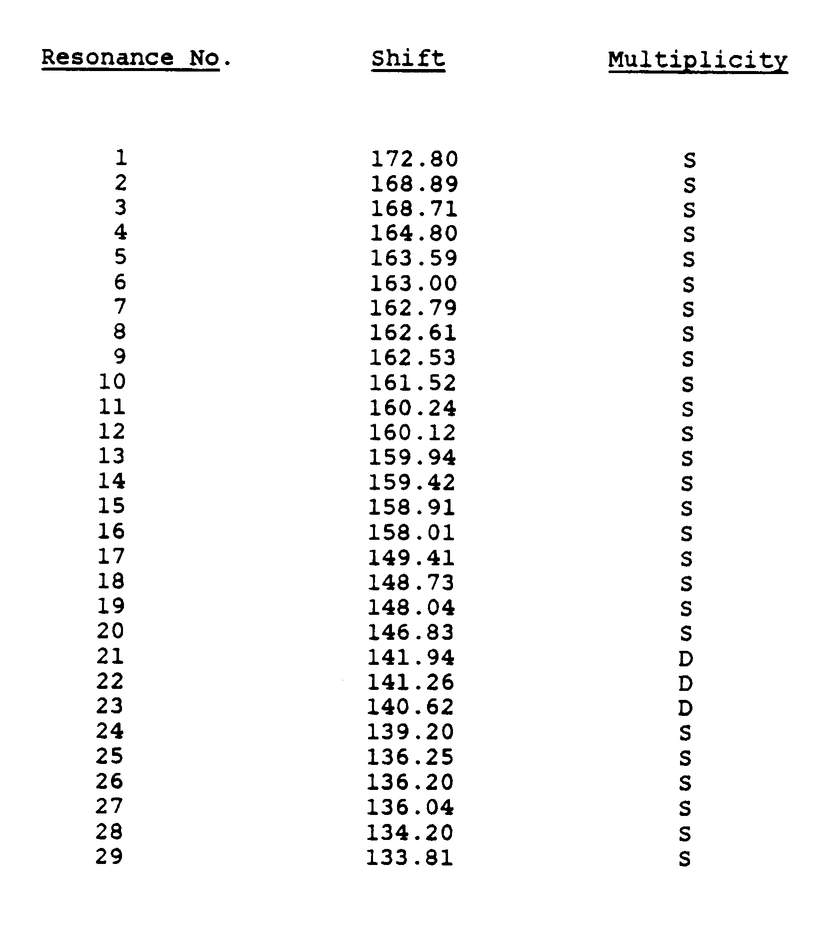 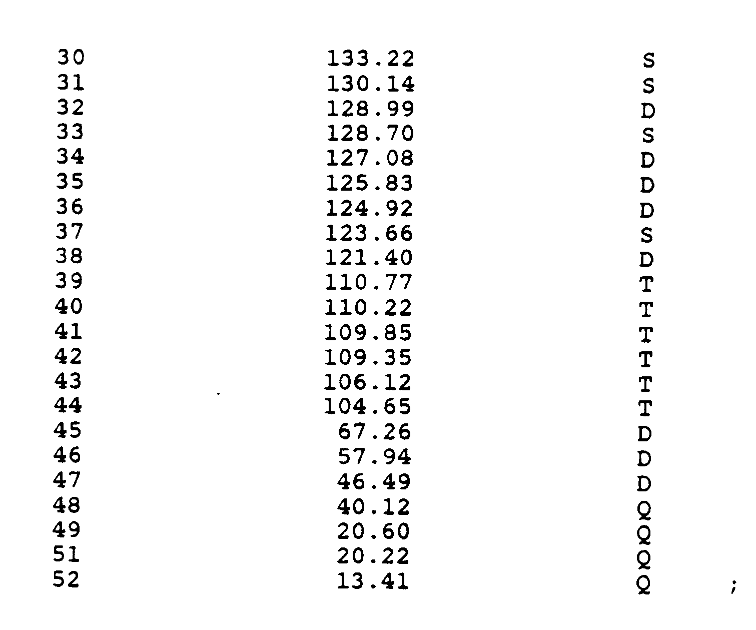 and
d) an elemental analysis which indicates the following percentage composition: 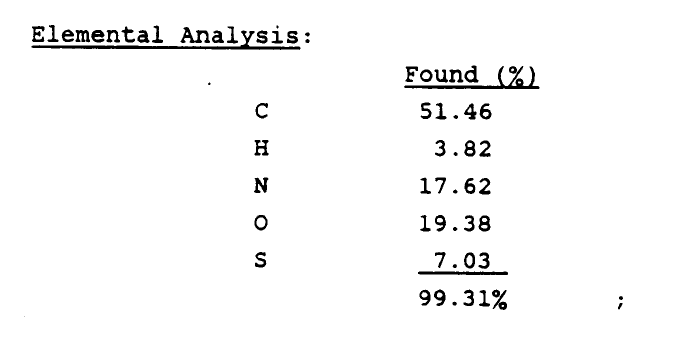 and
e) an infrared spectrum as shown in figure 5.

7. Antibiotic A10255 factor H, which is a non-crystalline white to light-yellow powder which is soluble in dimethylfulfoxide, dimethylformamide pyridine, 1:1 (v:v) methylene chloride/methanol, and 4:1 (v:v) tetrahydrofuran:water, having the following physical characteristics:
a) an ultraviolet spectrum in neutral ethanol which shows a λₘₐₓ = 244 nm (ε = 74,000); acidic solution, λₘₐₓ = 245 nm (ε = 74,500); and basic solution, λₘₐₓ = 211 nm (ε = 23,800); and
b) a molecular weight of 1162.32 daltons.
c) a ¹³C nuclear magnetic resonance spectrum at 125 MHz in perdeuterated dimethylsulfoxide having the following signals: 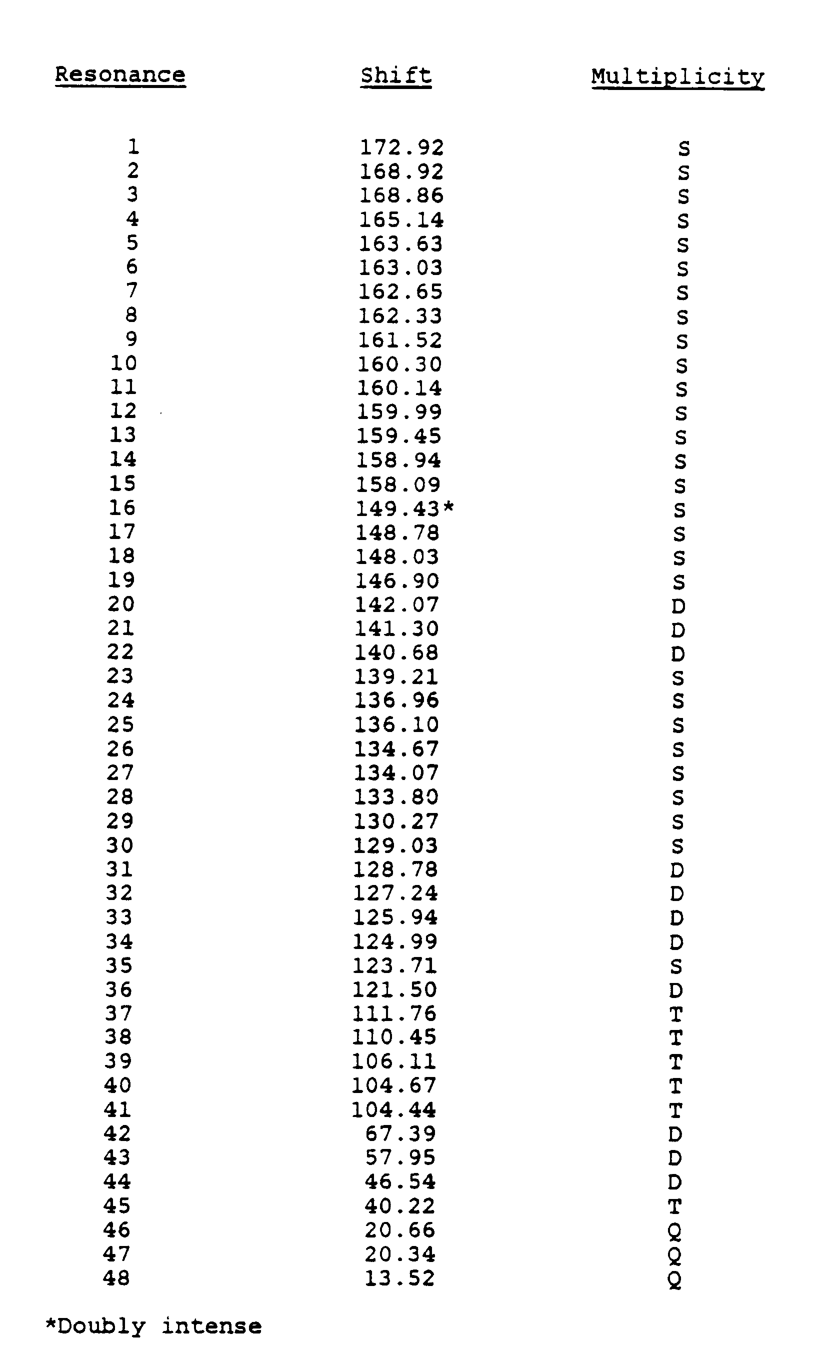
d) a ¹H nuclear magnetic resonance spectrum in 500 MHz in perdeuterated dimethylsulfoxide having the following signals: 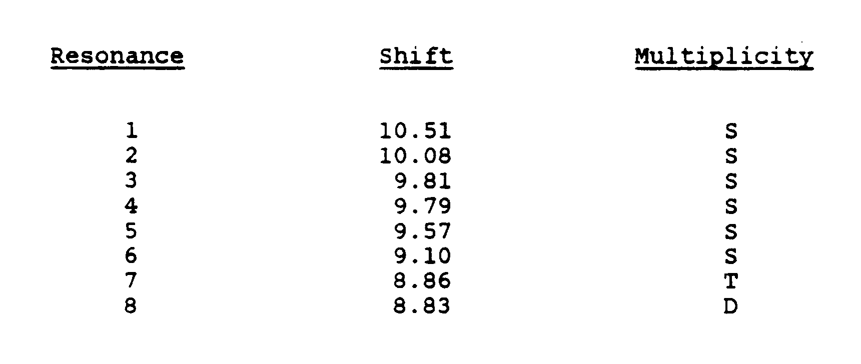 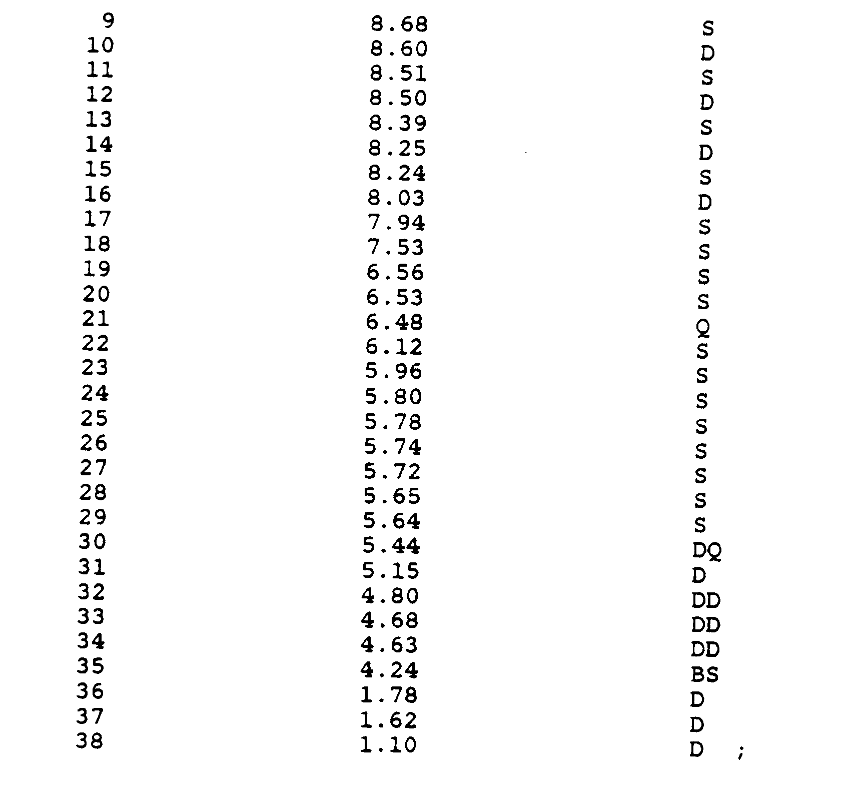 and
e) an infrared spectrum as shown in figure 6.

8. A process for producing A10255 complex, or factor B, C, E, F, G, or H, or any combination thereof, which comprises
a) cultivating Streptomyces gardneri NRRL 15537, Streptomyces gardneri NRRL 18260, Streptomyces gardneri NRRL 15922, or an A10255-producing mutant thereof, in a culture medium containing assimilable sources of carbon, nitrogen, and inorganic salts under submerged aerombic fermentation conditions until the antibiotic A10255 complex is produced;
b) optionally separating the A10255 complex from the culture medium; and
c) optionally isolating one or more of antibiotic A10255 factors B, C, E, F, G and H from the separated A10255 complex.

9. A process according to claim 8 utilizing the cultivation of Streptomyces gardneri NRRL 15537 or an A10255-producing mutant thereof.

10. A process according to claim 8 utilizing the cultivation of Streptomyces gardneri NRRL 15922 or an A10255-producing mutant thereof.

11. A process according to claim 8 utilizing the cultivation of Streptomyces gardneri NRRL 18260 or an A10255-producing mutant thereof.

12. A feed composition for increasing feed utilization efficienty, which comprises animal feed and the A10255 complex, an A10255 factor selected from the group of A10255 factors B, C, E, F, G and H, or a combination of one or more of said factors.

13. An animal premix comprising as an active ingredient A10255 complex, or factor B, C, E, F, G or H, in any combination, or a physiologically-acceptable salt thereof, associated with a suitable inert carrier therefor.

14. A biologically pure culture of the microorganism Streptomyces gardneri NRRL 15537, NRRL 15922, or NRRL 18260, or a mutant thereof, which produces the antibiotic A10255 complex.

15. A pharmaceutical composition comprising as an active ingredient A10255 complex, an A10255 factor chosen from the group consisting of A10255 factor B, A10255 factor C, A10255 factor E, A10255 factor F, A10255 factor G, and A10255 factor H, or a mixture of one or more of said factors, or a pharmaceutically-acceptable salt thereof, associated with one or more pharmaceutically-acceptable carriers or excipients therefor.

16. A method for increasing the feed utilization efficiency of a domesticated animal, which comprises administering to a domesticated animal A10255 complex, an A10255 factor selected from the group consisting of A10255 factor B, A10255 factor C, A10255 factor E, A10255 factor F, A10255 factor G, and A10255 factor H, or a combination of one or more of said factors.

17. A method for determining thiostrepton resistance in Streptomyces species, which comprises applying the A10255 complex, A10255 factor B, A10255 factor C, A10255 factor E, A10255 factor F, A10255 factor G, or A10255 factor H, to the culture media of a Streptomyces species.

18. A pharmaceutically-acceptable salt of the A10255 complex.

## Claims (Claims for the following Contracting State(s): GR, ES)

1. A process for producing A10255 complex, or factor B, C, E, F, G, or H, or any combination thereof, which comprises
a) cultivating Streptomyces gardneri NRRL 15537, Streptomyces gardneri NRRL 18260, Streptomyces gardneri NRRL 15922, or an A10255-producing mutant thereof, in a culture medium containing assimilable sources of carbon, nitrogen, and inorganic salts under submerged aerobic fermentation conditions until the antibiotic A10255 complex is produced;
b) optionally separating the A10255 complex from the culture medium; and
c) optionally isolating one or more of antibiotic A10255 factors B, C, E, F, G and H from the separated A10255 complex.

2. A process according to claim 1 utilizing the cultivation of Streptomyces gardneri NRRL 15537 or an A10255-producing mutant thereof.

3. A process according to claim 1 utilizing the cultivation of Streptomyces gardneri NRRL 15922 or an A10255-producing mutant thereof.

4. A process according to claim 1 utilizing the cultivation of Streptomyces gardneri NRRL 18260 or an A10255-producing mutant thereof.

5. A process according to any one of claims 1 to 4, wherein A10255 factor B is isolated.

6. A process according to any one of claims 1 to 4, wherein A10255 factor G is isolated.

7. A feed composition for increasing feed utilization efficienty, which comprises animal feed and the A10255 complex, an A10255 factor selected from the group of A10255 factors B, C, E, F, G and H, or a combination of one or more of said factors.

8. A method for increasing the feed utilization efficiency of a domesticated animal, which comprises administering to a domesticated animal A10255 complex, an A10255 factor selected from the group consisting of A10255 factor B, A10255 factor C, A10255 factor E, A10255 factor F, A10255 factor G, and A10255 factor H, or a combination of one or more of said factors.

9. A method for determining thiostrepton resistance in Streptomyces species, which comprises applying the A10255 complex, A10255 factor B, A10255 factor C, A10255 factor E, A10255 factor F, A10255 factor G, or A10255 factor H, to the culture media of a Streptomyces species.

## Patentansprüche (Patentansprüche für folgende(n) Vertragsstaat(en): AT, BE, CH, DE, FR, GB, IT, LI, LU, NL, SE)

1. Antibiotikum, des hergestellt werden kann, indem Streptomyces gardneri NRRL 15537, Streptomyces gardneri NRRL 18260 und Streptomyces gardneri NRRL 15922 in einem Kulturmedium, das assimilierbare Quellen für Kohlenhydrate, Stickstoff und anorganische Salze enthält, unter aeroben Submersfermentationsbedingungen kultiviert werden.

2. Faktor B von Antibiotikum A10255, der ein nicht-kristallines weißes bis hellgelbes Pulver ist, mit den folgenden Eigenschaften:
(a) eine ungefähre durchschnittliche Elementaranalyse von 49,25% Kohlenstoff, 3,94% Wasserstoff, 15,65% Stickstoff, 21,63% Sauerstoff und 6,73% Schwefel;
(b) ein Infrarotabsorptionsspektrum in einem Kaliumbromidpellet mit wesentlichen Absorptionsmaxima bei den folgenden Frequenzen (in cm⁻¹): 3373, 2969, 2932, 2875, 1661, 1598, 1520, 1494, 1395, 1250, 1114, 1084, 996, 932 und 900;
(c) titrierbare Gruppen, gemessen in 66% wäßrigem Dimethylformamid mit pKa-Werten von ungefähr 4,9, 11,2 und 12,8;
(d) ein Molekulargewicht von etwa 1244 Dalton bestimmt mit Fast Atom Bombardment-Massenspektroskopie;
(e) ist löslich in Dimethylsulfoxid, Dimethylformamid, Pyridin, Chloroform/Methanol-Mischungen und einer ungefähr 4:1 (V:V) Mischung von Tetrahydrofuran:Wasser;
(f) enthält ungefähr 629 mmol/mg Threonin und ungefähr 5268 mmol Ammoniakäquivalente pro mg, bestimmt durch Hydrolyse mit 6N Chlorwasserstoffsäure;
(g) ein Ultraviolettabsorptionsspektrum in saurem, basischem und neutralem Methanol mit einem Absorptionsmaximum bei 245 nm (ε = 66000);
(h) ein kernmagnetisches Protonenresonanzspektrum in perdeuteriertem Dimethylsulfoxid bei 500 MHz mit den folgenden Signalen:
| Resonanz-Nr. | Verschiebung | Multiplizität |
|---|---|---|
| 1 | 10,53 | S |
| 2 | 9,95 | S |
| 3 | 9,86 | S |
| 4 | 9,79 | S |
| 5 | 9,61 | S |
| 6 | 9,58 | S |
| 7 | 9,09 | S |
| 8 | 8,90 | T |
| 9 | 8,84 | D |
| 10 | 8,67 | S |
| 11 | 8,60 | S |
| 12 | 8,51 | S |
| 13 | 8,48 | D |
| 14 | 8,39 | S |
| 15 | 8,25 | D |
| 16 | 8,24 | S |
| 17 | 8,04 | D |
| 18 | 6,53* | S |
| 19 | 6,39 | T |
| 20 | 6,11 | S |
| 21 | 5,95 | S |
| 22 | 5,84 | S |
| 23 | 5,82 | S |
| 24 | 5,79 | S |
| 25 | 5,76⁺ | S |
| 26 | 5,68 | S |
| 27 | 5,64 | S |
| 28 | 5,44 | DQ |
| 29 | 5,16 | D |
| 30 | 4,80 | DD |
| 31 | 4,67 | DD |
| 32 | 4,63 | DD |
| 33 | 4,24 | BS |
| 34 | 2,21 | DQ |
| 35 | 1,62 | D |
| 36 | 1,11 | D |
| 37 | 1,01 | T |
| | | |
|---|---|---|
| * doppelt intensiv | | |
| ⁺ dreifach intensiv | | |
(i) ein kernmagnetisches ¹³C-Resonanzspektrum in perdeuteriertem Dimethylsulfoxid bei 125 MHz mit den folgenden Signalen: 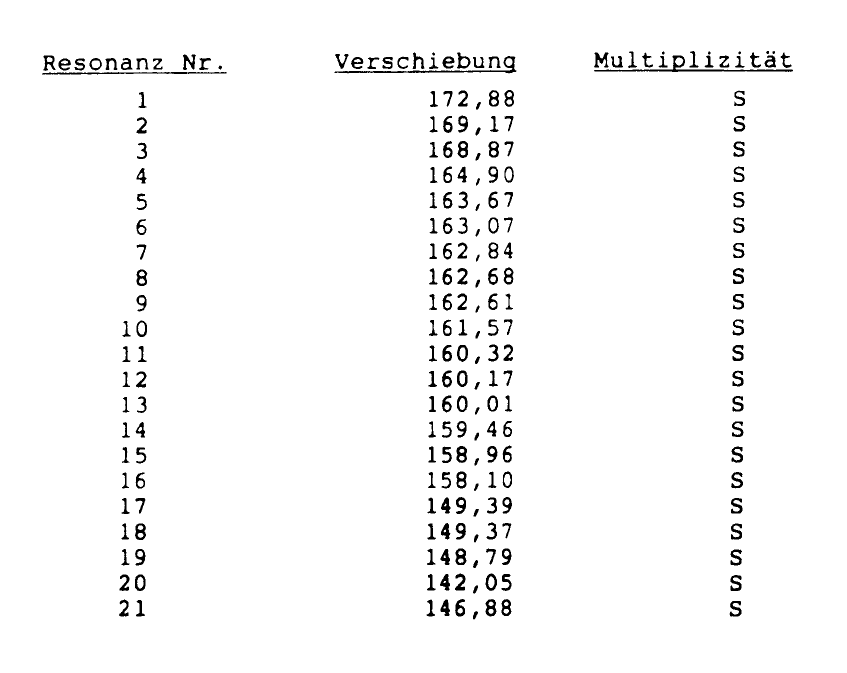 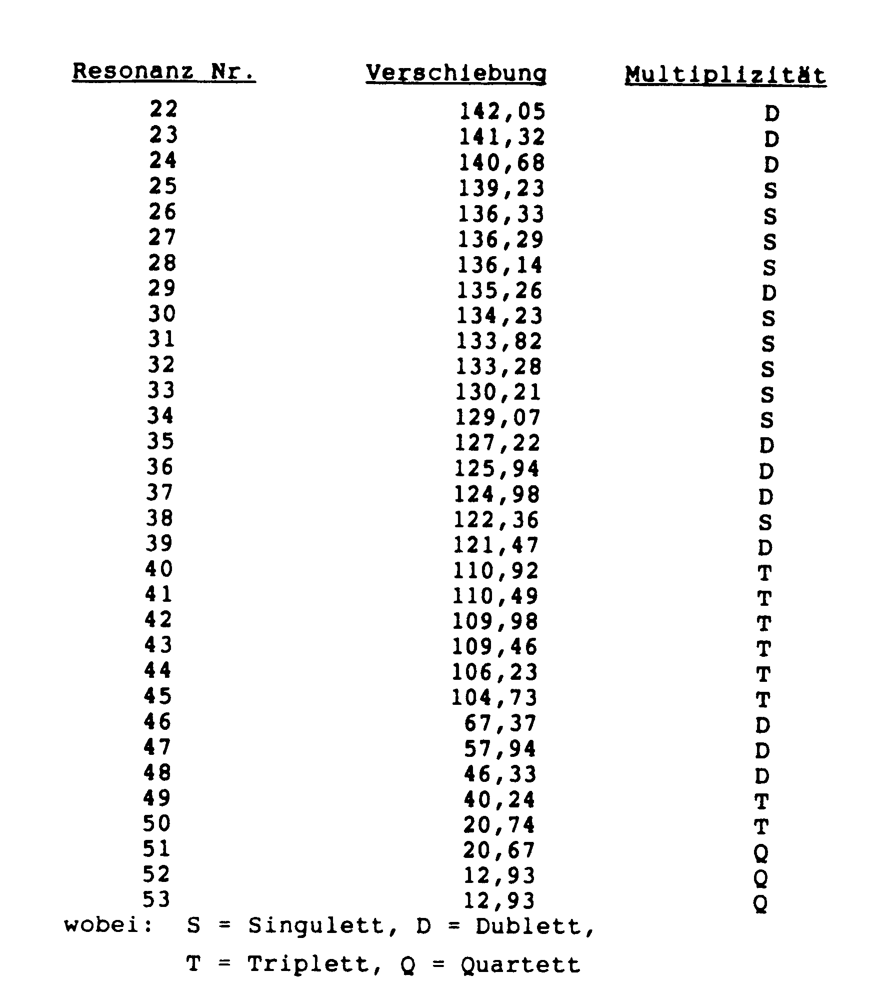 oder ein pharmazeutisch annehmbares Salz davon.

3. Faktor C von Antibiotikum A10255, der ein nicht-kristallines weißes bis hellgelbes Pulver ist, mit den folgenden physikalischen Eigenschaften:
(a) eine ungefähre durchschnittliche Elementaranalyse von 49,18% Kohlenstoff, 3,86% Wasserstoff, 17,89% Stickstoff, 18,28% Sauerstoff und 6,46% Schwefel;
(b) ein kernmagnetisches Protonenresonanzspektrum in perdeuteriertem Dimethylsulfoxid bei 360 MHz mit Signalen bei den folgenden δ-Werten: 10,51, 10,08, 9,84, 9,57, 9,10, 8,88, 8,03, 7,94, 7,53, 5,15 (austauschbar), 8,67, 8,59, 8,51, 8,49, 8,38, 8,25, 8,24, 6,57, 6,52, 6,38, 6,11, 5,91, 5,78, 5,74, 5,70, 5,65, 5,63, 5,44, 5,15, 4,79, 4,67, 4,63, 4,23, 2,21, 1,62, 1,11 und 1,01,
(c) ein Infrarotabsorptionsspektrum in Koliumbromid mit Absorptionsmaxima bei 3375, 2973, 2932, 2876, 1661, 1597, 1494, 1427, 1345, 1305, 1249, 1111, 1083, 981, 933 und 894 cm⁻¹;
(d) ein Ultraviolettabsorptionsspektrum mit einem Absorptionsmaximum bei 245 nm (ε = 63000) in neutralem, saurem und basischem Methanol;
(e) Molekulargewicht von ungefähr 1174 Dalton, bestimmt mit Fast Atom Bombardment-Massenspektralanalyse;
(f) titrierbare Gruppen, gemessen in 66% wäßrigem Dimethylformamid mit pKa-Werten von ungefähr 2,9 und 12;
(g) enthält ungefähr 758 mmol/mg Threonin und ungefähr 7429 mmol/mg Ammoniakäquivalente, bestimmt durch Standardhydrolyseverfahren mit 6N Chlorwasserstoffsäure;
(h) ist löslich in Dimethylsulfoxid, Dimethylformamid, Pyridin, 1:1 (V:V) Chloroform:Methanol und 4:1 (V:V) Tetrahydrofuran:
Wasser;
oder ein pharmazeutisch annehmbares Salz davon.

4. Faktor E von Antibiotikum A10255, der ein nicht-kristallines weißes bis hellgelbes Pulver ist, mit den folgenden Eigenschaften:
(a) ungefähre durchschnittliche Elementaranalyse von 48,03% Kohlenstoff, 3,91% Wasserstoff, 15,76% Stickstoff, 17,09% Sauerstoff und 5,63% Schwefel;
(b) Infrarotabsorptionsspektrum in einem Kaliumbromidpellet mit wesentlichen Absorptionsmaxima bei den folgenden Frequenzen (in cm⁻¹): 3367, 3361, 2966, 1664, 1501, 1389, 1254, 1102 und 889;
(c) titrierbare Gruppen gemessen in 66% wäßrigem Dimethylformamid mit pKa-Werten von ungefähr 4,85, 11,1 und 13,2;
(d) Molekulargewicht von etwa 1258 Dalton bestimmt mit Fast Atom Bobardment-Massenspektroskopie;
(e) ist löslich in Dimthylsulfoxid, Dimethylformamid, Pyridin und einer ungefähr 4:1 (V:V) Mischung von Tetrahydrofuran:Wasser;
(f) enthält ungefähr 716 mmol/mg Threonin und ungefähr 8580 mmol Ammoniakäquivalente pro mg, bestimmt durch Hydrolyse mit 6N Chlorwasserstoffsäure;
(g) Ultraviolettabsorptionsspektrum in saurem, basischem und neutralem Methanol zeigt ein Absorptionsmaximum bei 245 nm (ε = 77000);
(h) kernmagnetisches Protonenresonanzspektrum in perdeuteriertem Dimethylsulfoxid bei 270 MHz weist Signale auf bei δ 10,54, 10,00, 9,94, 9,81, 9,60, 9,56, 9,45, 8,89, 8,84, 8,66, 8,59, 8,50, 8,47, 8,39, 8,25, 8,22, 8,10, 6,53, 6,50, 6,24, 5,95, 5,86, 5,84, 5,77, 5,64, 5,55, 5,52, 5,44, 5,10, 4,80, 4,66, 4,64, 4,22, 2,78, 1,60, 1,11 und 1,00;
oder ein pharmazeutisch annehmbares Salz davon.

5. Faktor F von Antibiotikum A10255, der ein nicht-kristallines weißes bis hellgelbes Pulver ist, das die folgenden physikalischen Eigenschaften besitzt:
(a) ungefähre durchschnittliche Elementaranalyse von 49,65% Kohlenstoff, 4,23% Wasserstoff, 17,11% Stickstoff, 22,08% Sauerstoff und 7,78% Schwefel;
(b) kernmagnetisches Protonenresonanzspektrum in perdeuteriertem Dimethylsulfoxid bei 360 MHz mit Signalen bei δ 10,51, 10,17, 9,88, 9,77, 9,54, 9,10, 8,90, 8,88, 8,66, 8,59, 8,51, 8,49, 8,38, 8,25, 8,24, 8,06, 7,94, 7,53, 6,56, 6,51, 6,27, 6,23, 6,12, 6,12, 5,96, 5,77, 5,76, 5,71, 5,71, 5,64, 5,62, 5,47, 5,14, 4,77, 4,65, 4,62, 4,20, 2,77, 2,48, 2,48, 1,58, 1,08, 0,98 und 0,98;
(c) Infrarotabsorptionsspektrum in Kaliumbromid mit Absorptionsmaxima bei 3369, 2943, 2907, 2846, 1663, 1588, 1519, 1493, 1425, 1337, 1288, 1251, 1151, 1110, 1083, 995, 927, 890, 807, 776 und 751 cm⁻¹;
(d) ein Ultraviolettabsorptionsspektrum mit einem Absorptionsmaximum bei 245 nm (ε = 71500) in neutralem, saurem und basischem Methanol;
(e) ein Molekulargewicht von ungefähr 1188 Dalton, bestimmt mit Fast Atom Bombardment Massenspektroskopie;
(f) eine titrierbare Gruppe gemessen in 66% wäßrigem Dimethylformamid mit einem pKa von 12,5;
(g) enthält ungefähr 735 mmol/mg Threonin und ungefähr 7226 mmol/mg Ammoniakäquivalente, bestimmt durch Standardhydrolyseverfahren mit 6N Chlorwasserstoffsäure;
(h) ist löslich in Dimethylsulfoxid, Dimethylformamid, Pyridin, 1:1 (V:V) Chloroform:Methanol und 4:1 (U:V) Tetrahydrofuran:
Wasser;
oder ein pharmazeutisch annehmbares Salz davon.

6. Faktor G von Antibiotikum A10255, der ein nicht-kristallines weißes bis hellgelbes Pulver ist, das in Dimethylsulfoxid, Dimethylformamid, Pyridin, Chloroform/Methanol-Mischungen und 4:1 (V:V) Tetrahydrofuran:Wasser löslich ist, mit den folgenden physikalischen Eigenschaften:
(a) ein Ultraviolettspektrum in neutralem Ethanol, das ein λmax = 247 nm (ε = 72200) zeigt, in saurer Lösung λmax = 247 nm (ε = 73400) und in basischer Lösung λmax = 211 nm (ε = 27200);
(b) ein kernmagnetisches ¹H-Resonanzspektrum bei 500 MHz in perdeuteriertem Dimethylsulfoxid mit den folgenden Signalen:
| Resonanz-Nr. | Verschiebung (δ) | Multiplizität |
|---|---|---|
| 1 | 10,53 | S |
| 2 | 9,95 | S |
| 3 | 9,83 | S |
| 4 | 9,79 | S |
| 5 | 9,59* | BS |
| 6 | 9,09 | S |
| 7 | 8,89 | T |
| 8 | 8,84 | D |
| 9 | 8,68 | S |
| 10 | 8,60 | S |
| 11 | 8,51 | S |
| 12 | 8,48 | D |
| 13 | 8,39 | S |
| 14 | 8,24 | S |
| 15 | 8,24 | D |
| 16 | 8,04 | D |
| 17 | 6,53* | S |
| 18 | 6,47 | Q |
| 19 | 6,10 | S |
| 20 | 5,95 | S |
| 21 | 5,84 | S |
| 22 | 5,81 | S |
| 23 | 5,80 | S |
| 24 | 5,78 | S |
| 25 | 5,76* | S |
| 26 | 5,68 | S |
| 27 | 5,64 | S |
| 28 | 5,44 | DQ |
| 29 | 5,16 | D |
| 30 | 4,75 | DD |
| 31 | 4,67 | DD |
| 32 | 4,63 | DD |
| 33 | 4,24 | BS |
| 34 | 1,77 | Q |
| 35 | 1,62 | Q |
| 36 | 1,11 | Q |
| | | |
|---|---|---|
| * doppelt intensiv | | |
| BS = breites Singulett DD = Dublett von Dubletts DQ = Dublett von Quartett | | |
(c) kernmagnetisches ¹³C-Resonanzspektrum bei 500 MHz in perdeuteriertem Dimethylsulfoxid mit den folgenden Adsorptionsmaxima: 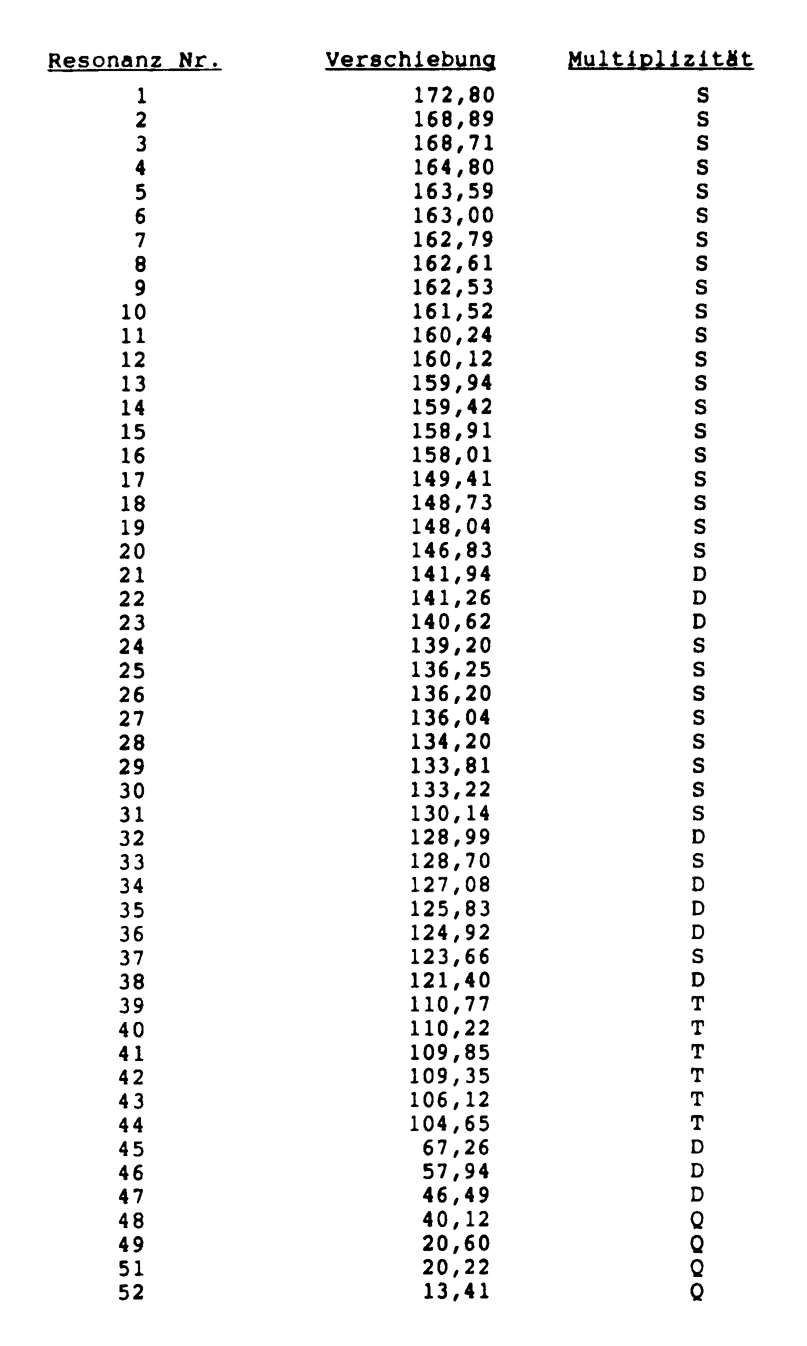 und
(d) eine Elementaranalyse, die die folgende prozentuale Zuammensetzung anzeigt:
| Elementaranalyse | Gefunden (%) |
|---|---|
| C | 51,46 |
| H | 3,82 |
| N | 17,62 |
| O | 19,38 |
| S | 7,03 |
| | 99,31% |
und
(e) ein Infrarotspektrum wie in Figur 5 gezeigt.

7. Faktor H von Antibiotikum A10255, der ein nicht-kristallines weißes bis hellgelbes Pulver ist, das in Dimethylsulfoxid, Dimethylformamid, Pyridin, 1:1 (V:V) Methylenchlorid:Methanol und 4:1 (V:V) Tetrahydrofuran:Wasser löslich ist, mit den folgenden physikalischen Eigenschaften:
(a) ein Ultraviolettspektrum in neutralem Ethanol, das ein λmax = 244 nm (ε = 74000) zeigt; in saurer Lösung λmax = 245 nm (ε = 74500) und in basischer Lösung λmax = 211 nm (ε = 23800); und
(b) ein Molekulargewicht von 1162,32 Dalton;
(c) ein kernmagnetisches ¹³C-Resonanzspektrum bei 125 MHz in perdeuteriertem Dimethylsulfoxid mit den folgenden Signalen: 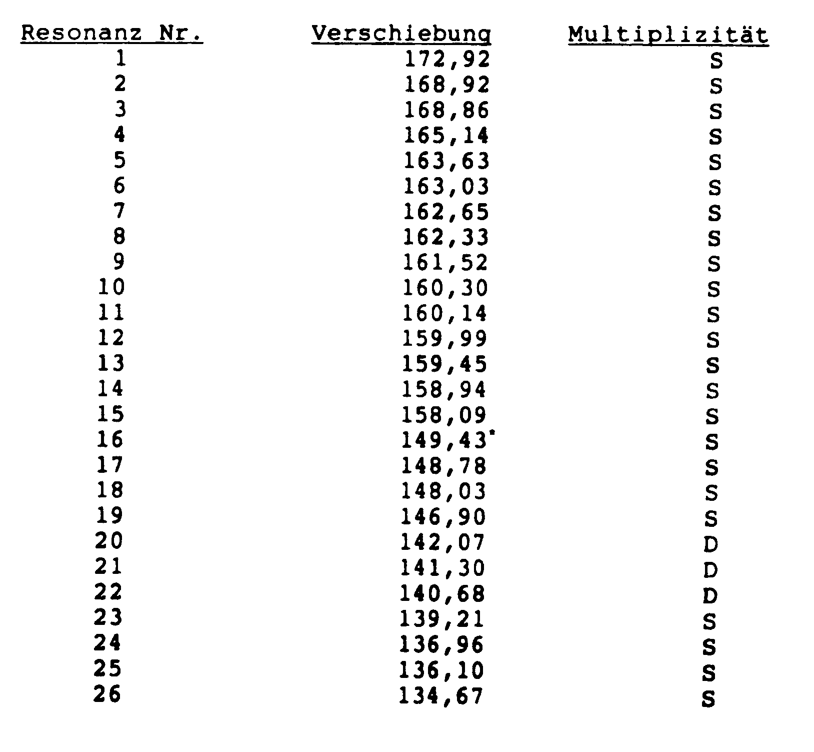 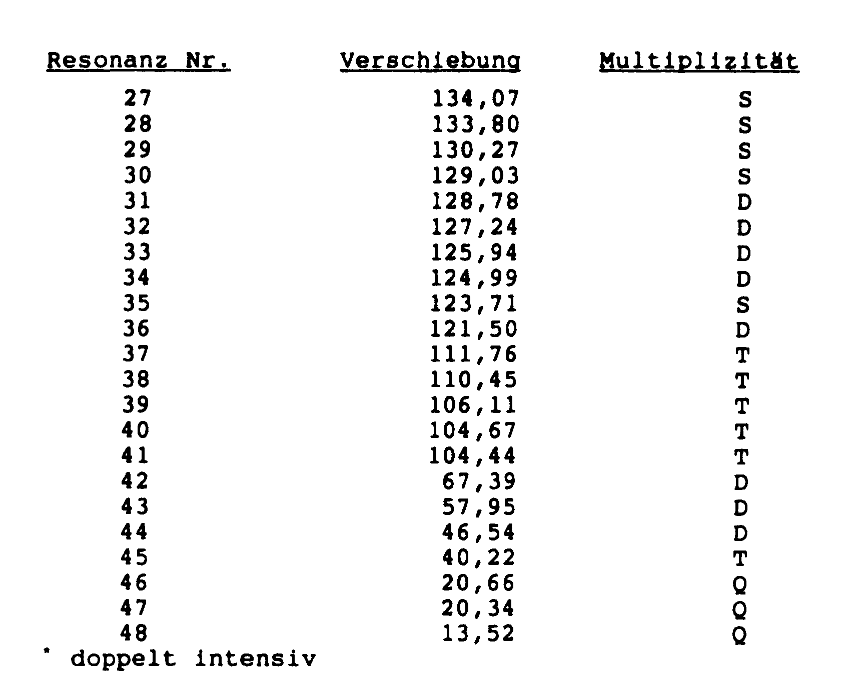 (d) kernmagnetisches ¹H-Resonanzspektrum bei 500 MHz in perdeuteriertem Dimethylsulfoxid mit den folgenden Signalen:
| Resonanz Nr. | Verschiebung | Multiplizität |
|---|---|---|
| 1 | 10,51 | S |
| 2 | 10,08 | S |
| 3 | 9,81 | S |
| 4 | 9,79 | S |
| 5 | 9,57 | S |
| 6 | 9,10 | S |
| 7 | 8,86 | T |
| 8 | 8,83 | D |
| 9 | 8,68 | S |
| 10 | 8,60 | D |
| 11 | 8,51 | S |
| 12 | 8,50 | D |
| 13 | 8,39 | S |
| 14 | 8,25 | D |
| 15 | 8,24 | S |
| 16 | 8,03 | D |
| 17 | 7,94 | S |
| 18 | 7,53 | S |
| 19 | 6,56 | S |
| 20 | 6,53 | S |
| 21 | 6,48 | Q |
| 22 | 6,12 | S |
| 23 | 5,96 | S |
| 24 | 5,80 | S |
| 25 | 5,78 | S |
| 26 | 5,74 | S |
| 27 | 5,72 | S |
| 28 | 5,65 | S |
| 29 | 5,64 | S |
| 30 | 5,44 | DQ |
| 31 | 5,15 | D |
| 32 | 4,80 | DD |
| 33 | 4,68 | DD |
| 34 | 4,63 | DD |
| 35 | 4,24 | BS |
| 36 | 1,78 | D |
| 37 | 1,62 | D |
| 38 | 1,10 | D |
und
e) ein Infrarotspektrum, wie in Figur 6 gezeigt.

8. Verfahren zur Herstellung von A10255-Komplex oder Faktor B, C, E, F, G oder H oder irgendeiner Kombination davon, das umfaßt, daß man
a) Streptomyces gardneri NRRL 15537, Streptomyces gardneri NRRL 18260, Streptomyces gardneri NRRL 15922 oder eine A10255-produzierende Mutante davon in einem Kulturmedium, das assimilierbare Quellen für Kohlenstoff, Stickstoff und anorganische Salze enthält, unter aeroben Submersfermentationsbedingungen züchtet, bis der antibiotische A10255-Komplex erzeugt wird;
b) gegebenenfalls den A10255-Komplex von dem Kulturmedium abtrennt und
c) gegebenenfalls einen oder mehrere antibiotische A10255-Faktoren B, C, E, F, G und H von dem abgetrennten A10255-Komplex isoliert.

9. Verfahren nach Anspruch 8 unter Anwendung der Züchtung von Streptomyces gardneri NRRL 15537 oder einer A10255-produzierenden Mutante davon.

10. Verfahren nach Anspruch 8 unter Verwendung der Züchtung von Streptomyces gardneri NRRL 15922 oder einer A10255-produzierenden Mutante davon.

11. Verfahren nach Anspruch 8 unter Verwendung der Züchtung von Streptomyces gardneri NRRL 18260 oder einer A10255-produzierenden Mutante davon.

12. Futterzusammensetzung zur Erhöhung der Futterverwertungseffizienz, die ein Tierfutter und den A10255-Komplex, einen A10255-Faktor ausgewählt aus der Gruppe der A10255-Faktoren B, C, E, F, G und H oder eine Kombination eines oder mehrerer der Faktoren umfaßt.

13. Tierfuttervormischung, umfassend als aktiven Inhaltsstoff den A10255-Komplex oder Faktor B, C, E, F, G oder H in irgendeiner Kombination oder ein physiologisch annehmbares Salz davon zusammen mit einem geeigneten inerten Träger.

14. Biologisch reine Kultur des Mikroorganismus Streptomyces gardneri NRRL 15537, NRRL 15922 oder NRRL 18260 oder eine Mutante davon, die den antibiotischen A10255-Komplex produziert.

15. Pharmazeutische Zusammensetzung, umfassend als aktiven Inhaltsstoff den A10255-Komplex, einen A10255-Faktor ausgewählt aus der Gruppe bestehend aus A10255-Faktor B, A10255-Faktor C, A10255-Faktor E, A10255-Faktor F, A10255-Faktor G und A10255-Faktor H oder eine Mischung von einem oder mehreren dieser Faktoren oder ein pharmazeutisch annehmbares Salz davon zusammen mit einem oder mehreren pharmazeutisch annehmbaren Trägern oder Hilfsstoffen dafür.

16. Verfahren zur Erhöhung der Futterverwertungseffizienz eines Nutztieres, umfassend, daß man dem Nutztier einen A10255-Komplex, einen A10255-Faktor ausgewählt aus der Gruppe bestehend aus A10255-Faktor B, A10255-Faktor C, A10255-Faktor E, A10255-Faktor F, A10255-Faktor G und A10255-Faktor H oder eine Kombination eines oder mehrerer dieser Faktoren verabreicht.

17. Verfahren zur Bestimmung der Thiostreptonresistenz einer Streptomyces-Art, umfassend, daß man den A10255-Komplex, A10255-Faktor B, A10255-Faktor C, A10255-Faktor E, A10255-Faktor F, A10255-Faktor G oder A10255-Faktor H in das Kulturmedium einer Streptomyces-Art bringt.

18. Pharmazeutisch annehmbares Salz des A10255-Komplexes.

## Patentansprüche (Patentansprüche für folgende(n) Vertragsstaat(en): GR, ES)

1. Verfahren zur Herstellung von A10255-Komplex oder Faktor B, C, E, F, G oder H oder irgendeiner Kombination davon, das umfaßt, daß man
a) Streptomyces gardneri NRRL 15537, Streptomyces gardneri NRRL 18260, Streptomyces gardneri NRRL 15922 oder eine A10255-produzierende Mutante davon in einem Kulturmedium, das assimilierbare Quellen fur Kohlenstoff, Stickstoff und anorganische Salze enthält, unter aeroben Submersfermentationsbedingungen züchtet, bis der antibiotische A10255-Komplex erzeugt wird;
b) gegebenenfalls den A10255-Komplex von dem Kulturmedium abtrennt und
c) gegebenenfalls einen oder mehrere antibiotische A10255-Faktoren B, C, E, F, G und H von dem abgetrennten A10255-Komplex isoliert.

2. Verfahren nach Anspruch 1 unter Anwendung der Züchtung von Streptomyces gardneri NRRL 15537 oder einer A10255-produzierenden Mutante davon.

3. Verfahren nach Anspruch 1 unter Verwendung der Züchtung von Streptomyces gardneri NRRL 15922 oder einer A10255-produzierenden Mutante davon.

4. Verfahren nach Anspruch 1 unter Verwendung der Züchtung von Streptomyces gardneri NRRL 18260 oder einer A10255-produzierenden Mutante davon.

5. Verfahren nach einem der Ansprüche 1 bis 4, worin A10255-Faktor B isoliert wird.

6. Verfahren nach einem der Ansprüche 1 bis 4, worin A10255-Faktor G isoliert wird.

7. Futterzusammensetzung zur Erhöhung der Futterverwertungseffizienz, die ein Tierfutter und den A10255-Komplex, einen A10255-Faktor ausgewählt aus der Gruppe der A10255-Faktoren B, C, E, F, G und H oder eine Kombination eines oder mehrerer der Faktoren umfaßt.

8. Verfahren zur Erhöhung der Futterverwertungseffizienz eines Nutztieres, umfassend, daß man dem Nutztier einen A10255-Komplex, einen A10255-Faktor ausgewählt aus der Gruppe bestehend aus A10255-Faktor B, A10255-Faktor C, A10255-Faktor E, A10255-Faktor F, A10255-Faktor G und A10255-Faktor H oder eine Kombination eines oder mehrerer dieser Faktoren verabreicht.

9. Verfahren zur Bestimmung der Thiostreptonresistenz einer Streptomyces-Art, umfassend, daß man den A10255-Komplex, A10255-Faktor B, A10255-Faktor C, A10255-Faktor E, A10255-Faktor F, A10255-Faktor G oder A10255-Faktor H in das Kulturmedium einer Streptomyces-Art bringt.

## Revendications (Revendications pour l'(les) Etat(s) contractant(s) suivant(s): AT, BE, CH, DE, FR, GB, IT, LI, LU, NL, SE)

1. Antibiotique que l'on peut préparer en cultivant **Streptomyces gardneri** NRRL 15537, **Streptomyces gardneri** NRRL 18260 et **Streptomyces gardneri** NRRL 15922 dans un milieu de culture contenant des sources assimilables de glucides, d'azote et de sels inorganiques dans des conditions de fermentation aérobie immergée.

2. Antibiotique A10255 facteur B, à savoir une poudre blanche à jaune clair non cristalline, possédant les caractéristiques ci-après :
(a) une analyse élémentaire approximative moyenne: carbone, 49,25%; hydrogène, 3,94%; azote, 15,65%; oxygène, 21,63%; et soufre, 6,73%;
(b) un spectre d'absorption infrarouge dans une pastille de bromure de potassium, avec des maxima d'absorption significatifs aux fréquences ci-après (en cm⁻¹) : 3373, 2969, 2932, 2875, 1661, 1598, 1520, 1494, 1395, 1250, 1114, 1084, 996, 932 et 900;
(c) des groupes titrables mesurés dans du diméthylformamide aqueux à 66% avec des valeurs pKa d'approximativement 4,9; 11,2 et 12,8;
(d) un poids moléculaire d'environ 1244 daltons, comme déterminé par spectrométrie de masse par bombardement d'atomes rapides;
(e) est soluble dans le diméthylsulfoxyde, le diméthylformamide, la pyridine, des mélanges de chloroforme/méthanol et un mélange d'approximativement 4:1 (v:v) tétrahydrofuranne:eau;
(f) contient approximativement 629 millimoles/milligramme de thréonine et approximativement 5.268 millimoles d'équivalent d'ammoniac par milligramme, comme déterminé par hydrolyse avec de l'acide chlorhydrique 6N;
(g) un spectre d'absorption ultraviolet dans du méthanol acide, basique et neutre manifestant un maximum d'absorption à 245 nm (ε = 66.000).
(h) un spectre de résonance magnétique nucléaire protonique dans du diméthylsulfoxyde perdeutérique à 500 MHz avec les valeurs ci-après : 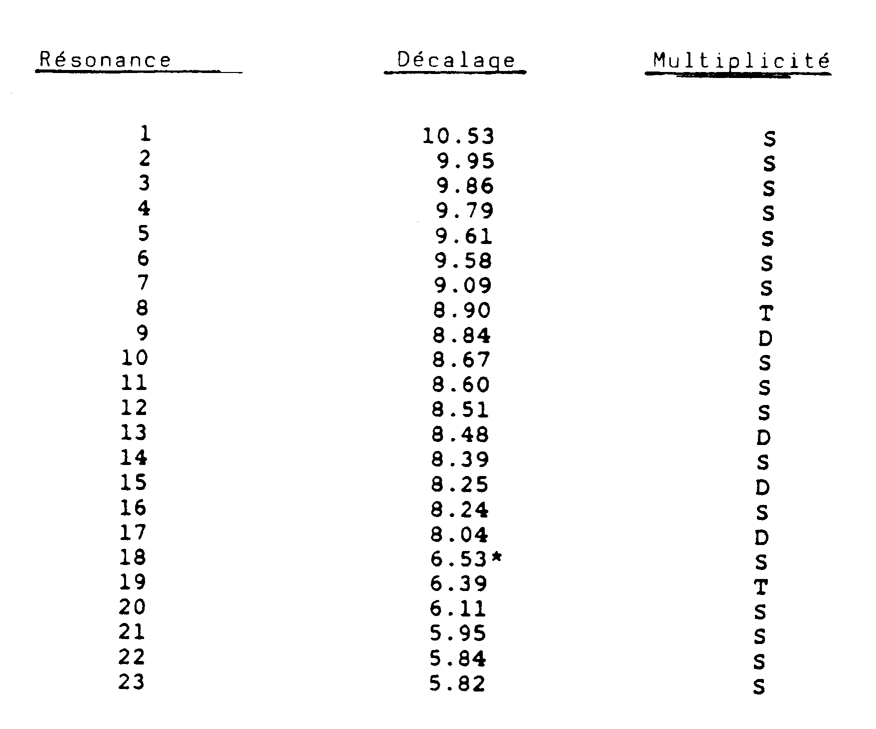 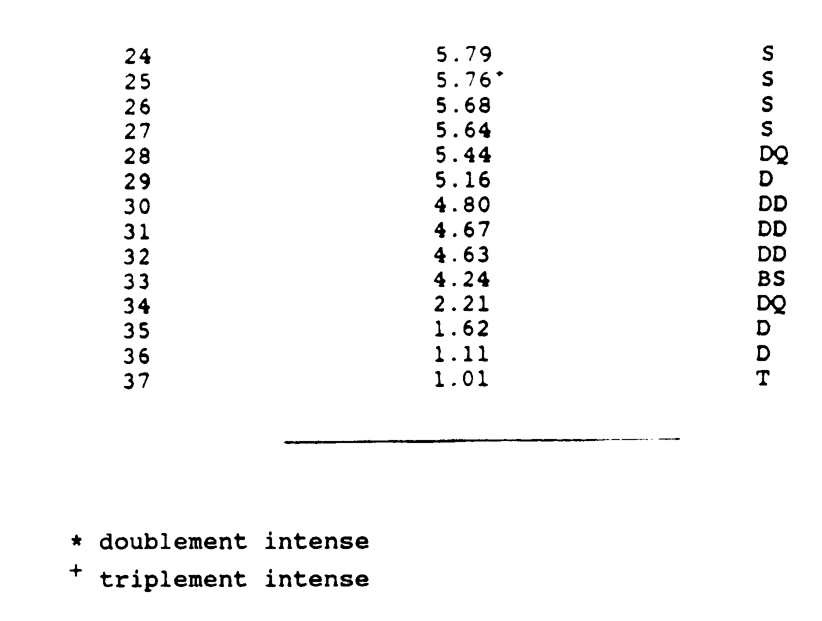
(i) un spectre de résonance magnétique nucléaire ¹³C dans du diméthylsulfoxyde perdeutérique à 125 MHz avec les valeurs suivantes : 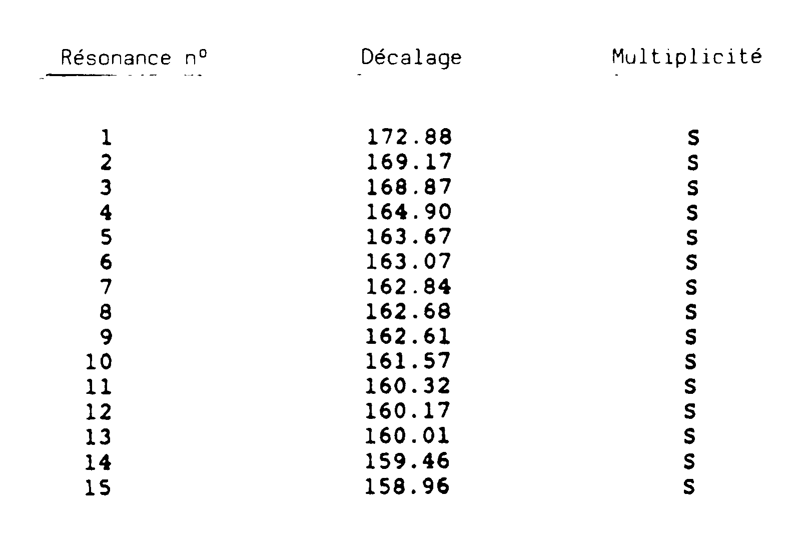 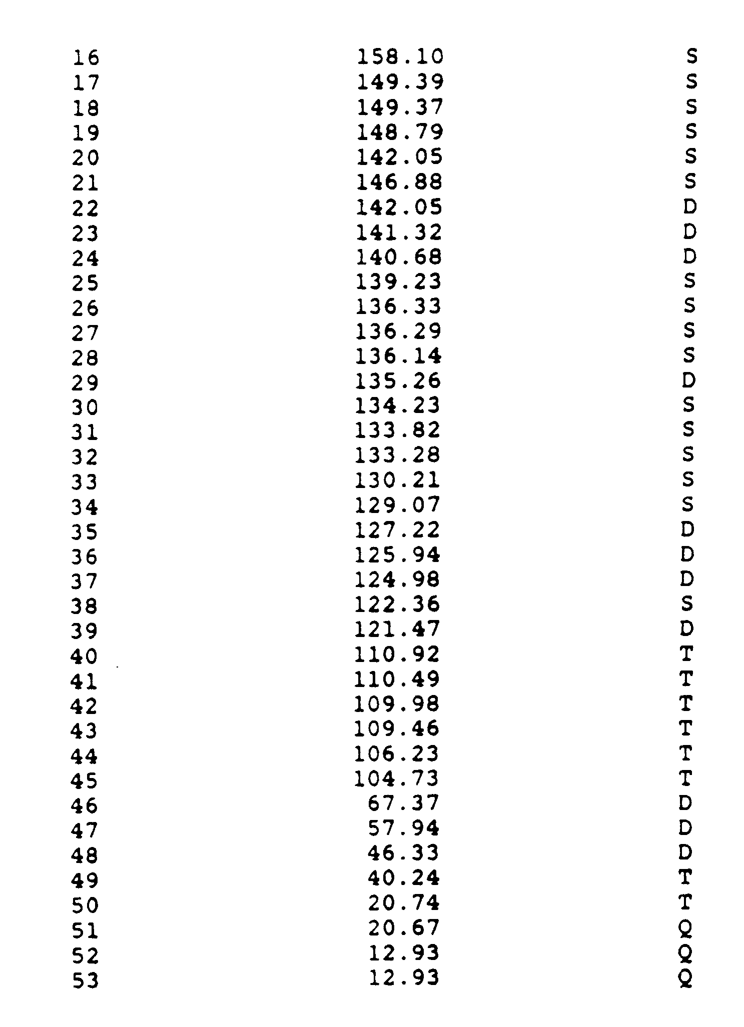 où S = singulet; D = doublet; T = triplet; Q = quartet ou un de ses sels pharmaceutiquement acceptables.

3. Antibiotique A10255 facteur C, à savoir une poudre blanche à jaune clair non cristalline, possédant les caractéristiques physiques ci-après :
(a) une analyse élémentaire approximative moyenne: carbone, 49,18%; hydrogène, 3,86%; azote, 17,89%; oxygène, 18,28%; et soufre, 6,46%;
(b) un spectre de résonance magnétique nucléaire protonique dans du diméthylsulfoxyde perdeutérique à 360 MHz avec les indications aux valeurs δ ci-après : 10,51; 10,08; 9,84; 9,57; 9,10; 8,88; 8,03; 7,94; 7,53; 5,15 (échangeables); 8,67; 8,59; 8,51; 8,49; 8,38; 8,25; 8,24; 6,57; 6,52; 6,38; 6,11; 5,91; 5,78; 5,74; 5,70, 5,65; 5,63; 5,44; 5,15; 4,79; 4,67; 4,63; 4,23; 2,21; 1,62; 1,11 et 1,01.
(c) un spectre d'absorption infrarouge dans du bromure de potassium avec des maxima d'absorption à 3375, 2973, 2932, 2876, 1661, 1597, 1494, 1427, 1345, 1305, 1249, 1111, 1083, 981, 933 et 894 cm⁻¹;
(d) un spectre d'absorption ultraviolet avec un maximum d'absorption à 245 nm (ε = 63.000) dans du méthanol neutre, acide et basique;
(e) un poids moléculaire d'approximativement 1174 daltons, comme déterminé par analyse spectrale de masse par bombardement d'atomes rapides;
(f) des groupes titrables mesurés dans du diméthylformamide aqueux à 66% avec des valeurs pKa d'approximativement 2,9 et 12;
(g) contient approximativement 758 millimoles/milligramme de thréonine et approximativement 7.429 millimoles/milligramme d'équivalents d'ammoniac, comme déterminé par des procédés d'hydrolyse classiques avec de l'acide chlorhydrique 6N;
(h) est soluble dans le diméthylsulfoxyde, le diméthylformamide, la pyridine, dans 1:1 (volume:volume) chloroforme:méthanol et dans 4:1 (volume:volume) tétrahydrofuranne:eau;
ou un de ses sels pharmaceutiquement acceptables.

4. Antibiotique A10255 facteur E, à savoir une poudre blanche à jaune clair non cristalline, possédant les caractéristiques ci-après :
(a) une analyse élémentaire approximative moyenne: carbone, 48,03%; hydrogène, 3,91%; azote, 15,76%; oxygène, 17,09%; et soufre, 5,63%;
(b) un spectre d'absorption infrarouge dans une pastille de bromure de potassium, avec des maxima d'absorption significatifs aux fréquences ci-après (en cm⁻¹) : 3367, 3361, 2966, 1664, 1501, 1389, 1254, 1102 et 889;
(c) des groupes titrables mesurés dans du diméthylformamide aqueux à 66% avec des valeurs pKa d'approximativement 4,85; 11,1 et 13,2;
(d) un poids moléculaire d'environ 1258 daltons, comme déterminé par spectrométrie de masse par bombardement d'atomes rapides;
(e) est soluble dans le diméthylsulfoxyde, le diméthylformamide, la pyridine et dans un mélange d'approximativement 4:1 (v:v) tétrahydrofuranne:eau;
(f) contient approximativement 716 millimoles/milligramme de thréonine et approximativement 8.580 millimoles d'équivalent d'ammoniac par milligramme, comme déterminé par hydrolyse avec de l'acide chlorhydrique 6N;
(g) un spectre d'absorption ultraviolet dans du méthanol acide, basique et neutre manifestant un maximum d'absorption à 245 nm (ε = 77.000);
(h) un spectre de résonance magnétique nucléaire protonique dans du diméthylsulfoxyde perdeutérique à 270 MHz manifestant des valeurs δ 10,54; 10,00; 9,94; 9,81; 9,60; 9,56; 9,45; 8,89; 8,84; 8,66; 8,59; 8,50; 8,47; 8,39; 8,25; 8,22; 8,10; 6,53; 6,50; 6,24; 5,95; 5,86; 5,84; 5,77; 5,64; 5,55; 5,52; 5,44; 5,10; 4,80; 4,66; 4,64; 4,22; 2,78; 1,60; 1,11 et 1,00;
ou un de ses sels pharmaceutiquement acceptables.

5. Antibiotique A10255 facteur F, à savoir une poudre blanche à jaune clair non cristalline, possédant les caractéristiques physiques ci-après :
(a) une analyse élémentaire approximative moyenne: carbone, 49,65%; hydrogène, 4,23%; azote, 17,11%; oxygène, 22,08%; et soufre, 7,78%;
(b) un spectre de résonance magnétique nucléaire protonique dans du diméthylsulfoxyde perdeutérique à 360 MHz avec des valeurs à δ : 10,51; 10,17; 9,88; 9,77; 9,54; 9,10; 8,90; 8,88; 8,66; 8,59; 8,51; 8,49; 8,38; 8,25; 8,24; 8,06; 7,94; 7,53; 6,56; 6,51; 6,27; 6,23; 6,12; 6,12; 5,96; 5,77; 5,76; 5,71; 5,71; 5,64; 5,62; 5,47; 5,14; 4,77; 4,65; 4,62; 4,20; 2,77; 2,48; 2,48; 1,58; 1,08; 0,98 et 0,98.
(c) un spectre d'absorption infrarouge dans du bromure de potassium avec des maxima d'absorption à 3369, 2943, 2907, 2846, 1663, 1588, 1519, 1493, 1425, 1337, 1288, 1251, 1151, 1110, 1083, 995, 927, 890, 807, 776 et 751 cm⁻¹.
(d) un spectre d'absorption ultraviolet avec un maximum d'absorption à 245 nm (ε = 71.500) dans du méthanol neutre, acide et basique;
(e) un poids moléculaire d'approximativement 1188 daltons, comme déterminé par spectroscopie de masse par bombardement d'atomes rapides;
(f) un groupe titrable mesuré dans du diméthylformamide aqueux à 66% à une valeur pKa de 12,5;
(g) contient approximativement 735 millimoles/milligramme de thréonine et approximativement 7.226 millimoles/milligramme d'équivalents d'ammoniac, comme déterminé par des procédés d'hydrolyse classiques avec de l'acide chlorhydrique 6N;
(h) est soluble dans le diméthylsulfoxyde, le diméthylformamide, la pyridine, dans 1:1 (volume:volume) chloroforme:méthanol et dans 4:1 (volume:volume) tétrahydrofuranne:eau;
ou un de ses sels pharmaceutiquement acceptables.

6. Antibiotique A10255 facteur G, à savoir une poudre blanche à jaune clair non cristalline qui est soluble dans le diméthylsulfoxyde, le diméthylformamide, la pyridine, dans des mélanges chloroforme:méthanol et dans 4:1 (volume:volume) tétrahydrofuranne:eau, possédant les caractéristiques physiques ci-après :
a) un spectre ultraviolet dans de l'éthanol neutre qui présente une valeur λmax = 247 nm (ε = 72.200); dans une solution acide, une valeur λmax = 247 nm (ε = 73.400); et dans une solution basique, une valeur λmax = 211 nm (ε = 27.200);
b) un spectre de résonance magnétique nucléaire ¹H à 500 MHz dans du diméthylsulfoxyde perdeutérique avec les valeurs suivantes :
| Résonance n° | Décalage (δ) | Multiplicité |
|---|---|---|
| 1 | 10.53 | S |
| 2 | 9.95 | S |
| 3 | 9.83 | S |
| 4 | 9.79 | S |
| 5 | 9.59* | BS |
| 6 | 9.09 | S |
| 7 | 8.89 | T |
| 8 | 8.84 | D |
| 9 | 8.68 | S |
| 10 | 8.60 | S |
| 11 | 8.51 | S |
| 12 | 8.48 | D |
| 13 | 8.39 | S |
| 14 | 8.24 | S |
| 15 | 8.24 | D |
| 16 | 8.04 | D |
| 17 | 6.53* | S |
| 18 | 6.47 | Q |
| 19 | 6.10 | S |
| 20 | 5.95 | S |
| 21 | 5.84 | S |
| 22 | 5.81 | S |
| 23 | 5.80 | S |
| 24 | 5.78 | S |
| 25 | 5.76* | S |
| 26 | 5.68 | S |
| 27 | 5.64 | S |
| 28 | 5.44 | DQ |
| 29 | 5.16 | D |
| 30 | 4.75 | DD |
| 31 | 4.67 | DD |
| 32 | 4.63 | DD |
| 33 | 4.24 | BS |
| 34 | 1.77 | Q |
| 35 | 1.62 | Q |
| 36 | 1.11 | Q |
| | | |
|---|---|---|
| * doublement intense | | |
| BS = singulet large DD = doublet de doublets DQ = doublet de quartets | | |
c) un spectre de résonance magnétique nucléaire ¹³C à 500 MHz dans du diméthylsulfoxyde perdeutérique avec les maxima d'absorption ci-après : 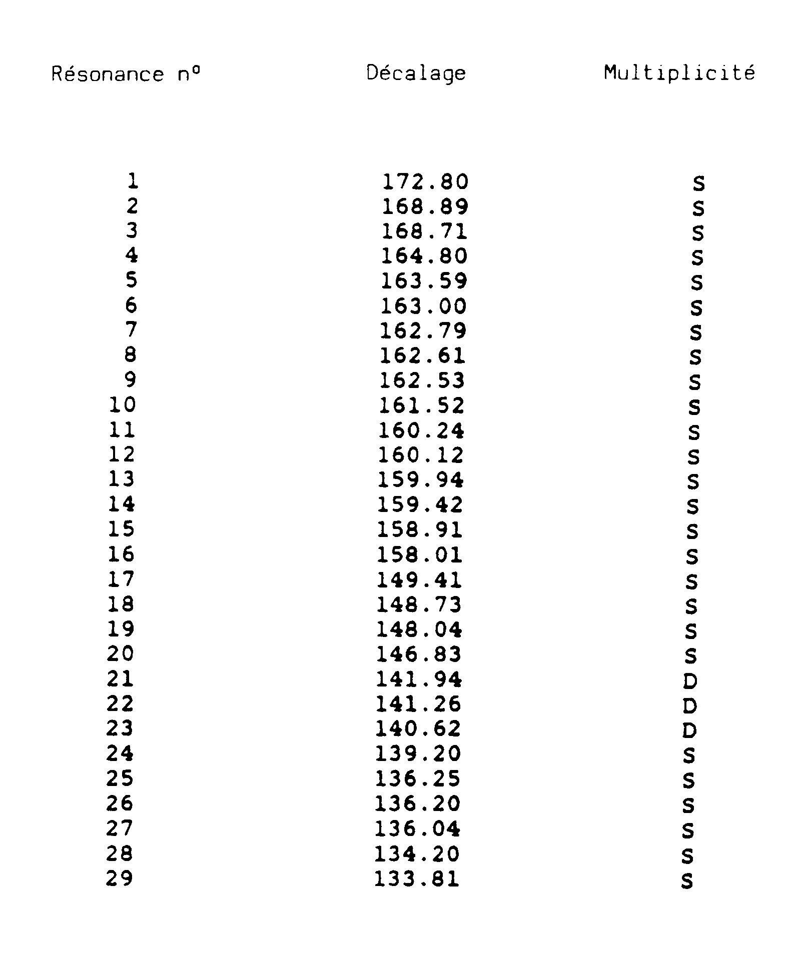 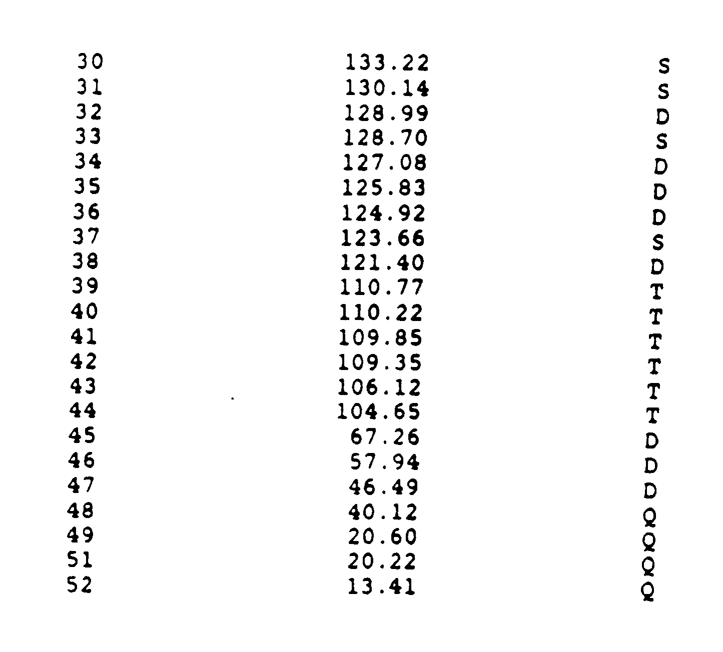 ; et
d) une analyse élémentaire qui indique la composition en pourcentage ci-après :
| Analyse élémentaire : | Trouvé (%) |
|---|---|
| C | 51,46 |
| H | 3,82 |
| N | 17,62 |
| O | 19,38 |
| S | 7,03 |
| | 99,31% |
; et
e) un spectre infrarouge tel que représenté en figure 5.

7. Antibiotique A10255 facteur H, à savoir une poudre blanche à jaune clair non cristalline qui est soluble dans le diméthylsulfoxyde, le diméthylformamide, la pyridine, dans 1:1 (v:v) chlorure de méthylène:méthanol et dans 4:1 (v:v) tétrahydrofuranne:eau, possédant les caractéristiques physiques ci-après :
a) un spectre ultraviolet dans de l'éthanol neutre qui indique une valeur λmax = 244 nm (ε = 74.000); dans une solution acide, une valeur λmax = 245 nm (ε = 74.500); et dans une solution basique, une valeur λmax= 211 nm (ε = 23.800);
b) un poids moléculaire de 1.162,32 daltons;
c) un spectre de résonance magnétique nucléaire ¹³C à 125 MHz dans du diméthylsulfoxyde perdeutérique avec les valeurs suivantes : 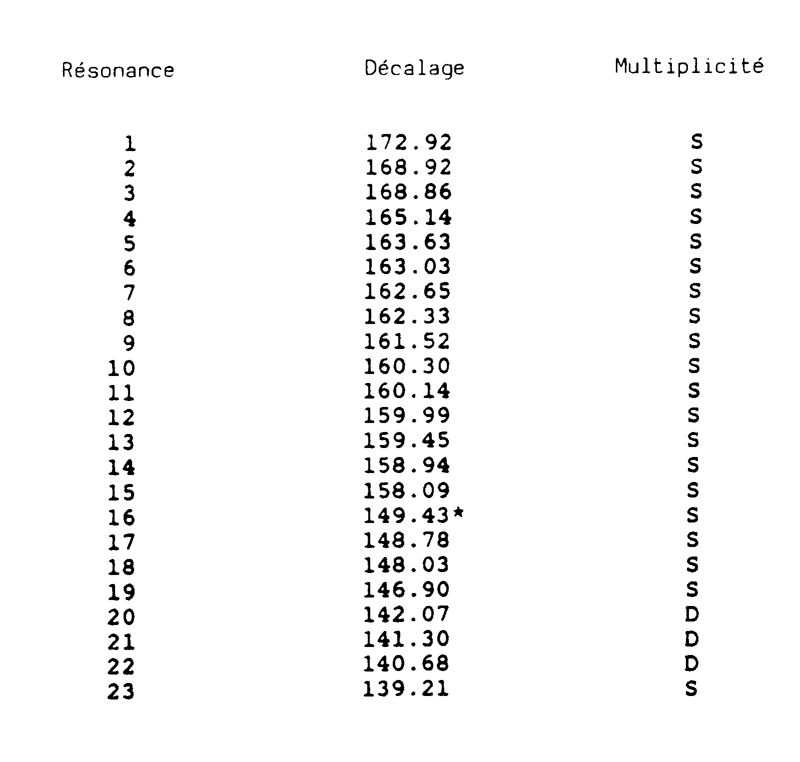 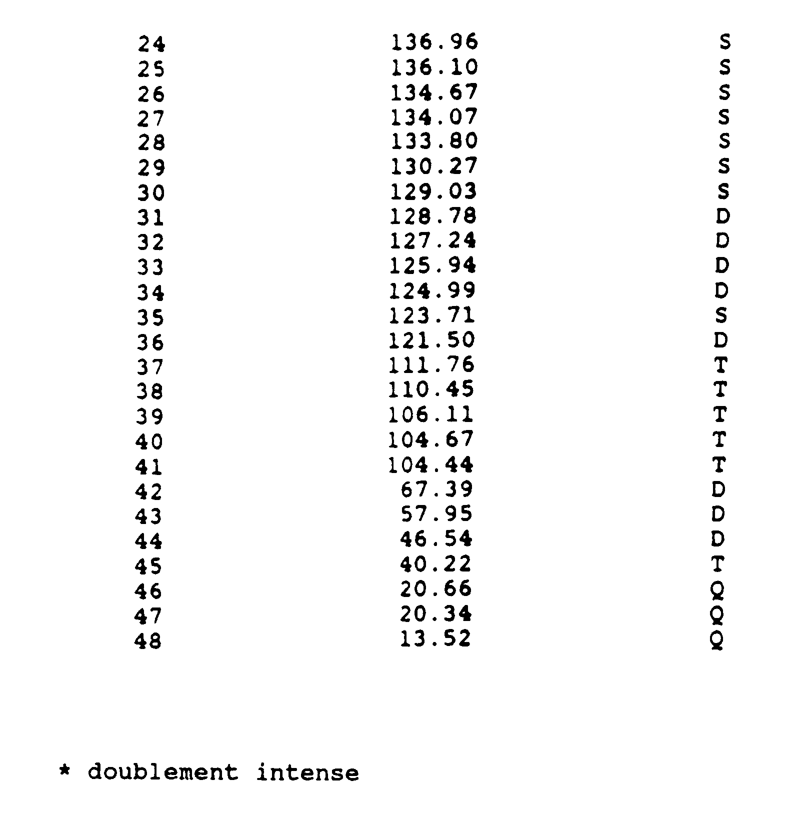
d) un spectre de résonance magnétique nucléaire ¹H à 500 MHz dans du diméthylsulfoxyde perdeutérique avec les valeurs ci-après :
| Résonance | Décalage | Multiplicité |
|---|---|---|
| 1 | 10.51 | S |
| 2 | 10.08 | S |
| 3 | 9.81 | S |
| 4 | 9.79 | S |
| 5 | 9.57 | S |
| 6 | 9.10 | S |
| 7 | 8.86 | T |
| 8 | 8.83 | D |
| 9 | 8.68 | S |
| 10 | 8.60 | D |
| 11 | 8.51 | S |
| 12 | 8.50 | D |
| 13 | 8.39 | S |
| 14 | 8.25 | D |
| 15 | 8.24 | S |
| 16 | 8.03 | D |
| 17 | 7.94 | S |
| 18 | 7.53 | S |
| 19 | 6.56 | S |
| 20 | 6.53 | S |
| 21 | 6.48 | Q |
| 22 | 6.12 | S |
| 23 | 5.96 | S |
| 24 | 5.80 | S |
| 25 | 5.78 | S |
| 26 | 5.74 | S |
| 27 | 5.72 | S |
| 28 | 5.65 | S |
| 29 | 5.64 | S |
| 30 | 5.44 | DQ |
| 31 | 5.15 | D |
| 32 | 4.80 | DD |
| 33 | 4.68 | DD |
| 34 | 4.63 | DD |
| 35 | 4.24 | BS |
| 36 | 1.78 | D |
| 37 | 1.62 | D |
| 38 | 1.10 | D |
; et
e) un spectre infrarouge tel que représenté en figure 6.

8. Procédé pour préparer le complexe A10255 ou le facteur B, C, E, F, G ou H, ou encore n'importe quelle combinaison de ces derniers, qui consiste à:
a) cultiver **Streptomyces gardneri** NRRL 15537, **Streptomyces gardneri** NRRL 18260, **Streptomyces gardneri** NRRL 15922 ou un de leurs mutants producteurs de A10255, dans un milieu de culture contenant des sources assimilables de carbone, d'azote et de sels inorganiques dans des conditions de fermentation aérobie immergée jusqu'à ce que l'on obtienne le complexe antibiotique A10255;
b) éventuellement séparer le complexe A10255 du milieu de culture; et
c) éventuellement isoler un ou plusieurs des facteurs antibiotiques B, C, E, F, G et H de A10255 du complexe A10255 séparé.

9. Procédé selon la revendication 8, qui utilise la mise en culture de **Streptomyces gardneri** NRRL 15537 ou un de ses mutants producteurs de A10255.

10. Procédé selon la revendication 8, qui utilise la mise en culture de **Streptomyces gardneri** NRRL 15922 ou un de ses mutants producteurs de A10255.

11. Procédé selon la revendication 8, qui utilise la mise en culture de **Streptomyces gardneri** NRRL 18260 ou un de ses mutants producteurs de A10255.

12. Composition alimentaire pour augmenter le rendement alimentaire, qui comprend des aliments pour animaux et le complexe A10255, un facteur A10255 choisi parmi le groupe des facteurs B, C, E, F, G et H de A10255 ou encore une combinaison d'un ou de plusieurs desdits facteurs.

13. Prémélange pour animaux comprenant, comme ingrédient actif, le complexe A10255 ou le facteur B, C, E, F, G ou H, dans n'importe quelle combinaison, ou encore un de leurs sels physiologiquement acceptables, en association avec un support inerte approprié pour l'ingrédient actif.

14. Culture biologiquement pure du microorganisme **Streptomyces gardneri** NRRL 15537, NRRL 15922 ou NRRL 18260, ou un de leurs mutants, qui procure le complexe antibiotique A10255.

15. Composition pharmaceutique comprenant, comme ingrédient actif, le complexe A10255, un facteur de A10255 choisi parmi le groupe comprenant A10255 facteur B, A10255 facteur C, A10255 facteur E, A10255 facteur F, A10255 facteur G et A10255 facteur H, ou un mélange d'un ou de plusieurs desdits facteurs, ou encore un de leurs sels pharmaceutiquement acceptables, en association avec un ou plusieurs supports ou excipients pharmaceutiquement acceptables pour l'ingrédient actif.

16. Procédé pour augmenter le rendement alimentaire d'un animal d'élevage, qui consiste à administrer à un animal d'élevage, le complexe A10255, un facteur de A10255 choisi parmi le groupe comprenant A10255 facteur B, A10255 facteur C, A10255 facteur E, A10255 facteur F, A10255 facteur G et A10255 facteur H, ou une combinaison d'un ou de plusieurs desdits facteurs.

17. Procédé pour déterminer la résistance à la thiostreptone dans l'espèce **Streptomyces,** qui consiste à appliquer le complexe A10255, A10255 facteur B, A10255 facteur C, A10255 facteur E, A10255 facteur F, A10255 facteur G ou A10255 facteur H, aux milieux de culture d'une espèce **Streptomyces.**

18. Sel pharmaceutiquement acceptable du complexe A10255.

## Revendications (Revendications pour l'(les) Etat(s) contractant(s) suivant(s): GR, ES)

1. Procédé pour préparer le complexe A10255 ou le facteur B, C, E, F, G ou H, ou encore n'importe quelle combinaison de ces derniers, qui consiste à:
a) cultiver **Streptomyces gardneri** NRRL 15537, **Streptomyces gardneri** NRRL 18260, **Streptomyces gardneri** NRRL 15922 ou un de leurs mutants producteurs de A10255, dans un milieu de culture contenant des sources assimilables de carbone, d'azote et de sels inorganiques dans des conditions de fermentation aérobie immergée jusqu'à ce que l'on obtienne le complexe antibiotique A10255;
b) éventuellement séparer le complexe A10255 du milieu de culture; et
c) éventuellement isoler un ou plusieurs des facteurs antibiotiques B, C, E, F, G et H de A10255 du complexe A10255 séparé.

2. Procédé selon la revendication 1, qui utilise la mise en culture de **Streptomyces gardneri** NRRL 15537 ou un de ses mutants producteurs de A10255.

3. Procédé selon la revendication 1, qui utilise la mise en culture de **Streptomyces gardneri** NRRL 15922 ou un de ses mutants producteurs de A10255.

4. Procédé selon la revendication 1, qui utilise la mise en culture de **Streptomyces gardneri** NRRL 18260 ou un de ses mutants producteurs de A10255.

5. Procédé selon l'une quelconque des revendications l à 4, dans lequel on isole le facteur B de A10255.

6. Procédé selon l'une quelconque des revendications 1 à 4, dans lequel on isole le facteur G de A10255.

7. Composition alimentaire pour augmenter le rendement alimentaire, que comprend des aliments pour animaux et le complexe A10255, un facteur A10255 choisi parmi le groupe des facteurs B, C, E, F, G et H de A10255 ou encore une combinaison d'un ou de plusieurs desdits facteurs.

8. Procédé pour augmenter le rendement alimentaire d'un animal d'élevage, qui consiste à administrer à un animal d'élevage, le complexe A10255, un facteur de A10255 choisi parmi le groupe comprenant A10255 facteur B, A10255 facteur C, A10255 facteur E, A10255 facteur F, A10255 facteur G et A10255 facteur H, ou une combinaison d'un ou de plusieurs desdits facteurs.

9. Procédé pour déterminer la résistance à la thiostreptone dans l'espèce **Streptomyces,** qui consiste à appliquer le complexe A10255, A10255 facteur B, A10255 facteur C, A10255 facteur E, A10255 facteur F, A10255 facteur G ou A10255 facteur H, aux milieux de culture d'une espèce **Streptomyces.**
